# EUROPEAN PATENT APPLICATION

(11) **EP 3 943 083 A1**
(43) Date of publication of application: **26.01.2022**
(21) Application number: 20778787.0
(22) Date of filing: 20.03.2020
(51) Int. Cl.: A61K 31/4025, A61K 39/395, A61P 35/00

(54) **SMALL-MOLECULE INHIBITOR OF PD-1/PD-L1, PHARMACEUTICAL COMPOSITION THEREOF WITH PD-L1 ANTIBODY, AND APPLICATION OF SAME**

(30) Priority: 22.03.2019 CN 201910222624
(71) Applicant: Guangzhou Maxinovel Pharmaceuticals Co., Ltd., Guangzhou Hitech Park Guangzhou Guangdong 510000 (CN)
(72) Inventor: WANG, Yuguang, Shanghai 201210 (CN); WANG, Feilan, Shanghai 201210 (CN); ZHANG, Nong, Shanghai 201210 (CN)
(74) Representative: Secerna LLP
(86) International application number: PCT/CN2020/080362
(87) International publication number: WO 2020/192570

(57) **Abstract**

A small molecule inhibitor of PD-1/PD-L1, a pharmaceutical composition thereof with a PD-L1 antibody, and an application of the same. The small molecule inhibitor of PD-1/PD-L1 is aromatic ethylene or aromatic ethylene derivative represented by general formula (I) and can be used to treat cancer.

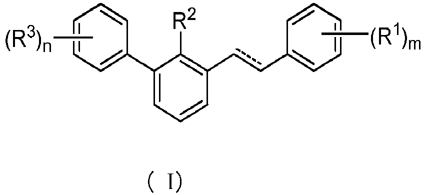

## Description

The application claims priority of Chinese Patent Application CN201910222624.0 filed on March 22, 2019, the contents of which are incorporated herein by reference in their entirety.

### Technical Field

The present disclosure relates to a small-molecule PD-1/PD-L1 inhibitor, a pharmaceutical composition thereof with PD-L1 antibody, and application of the same.

### Backgroud

Human programmed death ligand-1 (PD-L1), also known as B7-H1, is a member of the B7 family and widely distributed in peripheral tissues and hematopoietic cells. The full-length cDNA of PD-L1q is 870 bp, encoding a type I transmembrane protein with 290 amino acids. PD-L1 is mainly expressed on the surface of hematopoietic cells such as CD4 T cells, CD8 T cells, B cells, monocytes, dendritic cells (DCs), macrophages and some non-hematopoietic cells, such as endothelial cells, islet cells, mast cells, etc. PD-L1 is highly expressed in a variety of tumors, such as lung cancer, gastric cancer, multiple bone marrow, melanoma and breast cancer. Programmed death-1 (PD-1) is the main receptor of PD-L1, which is mainly distributed in immune-related cells such as T cells, B cells and NK cells, and plays an important role in the immune response process of autoimmune diseases, tumors, infections, organ transplantation, allergy and immune privilege.

PD-L1 inhibits T cell activation or induces mature T cell apoptosis by interacting with its receptor programmed death-1, which inhibits immune response. During tumor progression, cancer cells induce apoptosis of T cells by upregulating PD-L1 expression to avoid immune system clearance. PD-L1 targeted antibody drugs can break tumor immune tolerance by specifically blocking the interaction between PD-1 and PD-L1, restore the killing function of tumor specific T cells to tumor cells, and thus achieving tumor clearance. At present, four PD-L1 antibody drugs are on the market, they are Atezolizumab (Roche, commercial name: Tecentriq), its indications include melanoma, urothelial carcinoma (bladder cancer), and metastatic non-small cell lung cancer (stage IV); Durvalumab (Astra Zeneca, commercial name: Imfinzi), its indications include advanced or metastatic urothelial carcinoma (bladder cancer); Avelumab (Pfizer and Merck, commercial name: Bavencio), its indications include rare skin cancer Merkel cell carcinoma (MCC); Cemiplimab (Regeneron, commercial name: Libtayo), its indications include metastatic or locally advanced cutaneous squamous cell carcinoma. In the same time, there are many PD-L1 antibody drugs in development and clinical studies have been started for PD-L1 antibody drugs of Alphamab, Cstone, Hengrui, Mabspace Biosciences, BeiGene, Kelun-Biotech, Zhaoke, etc.

With the good efficacy of immunotherapy in many cancers and clinical trials, many cancer patients benefit from PD-1/PD-L1. However, studies have found that PD-1/PD-L1 macromolecular antibodies do not have good therapeutic effect on all cancers. The clinical trial data show that the efficacy of PD-1/PD-L1 monoclonal antibody drugs in the treatment of tumors is only between 10 and 30%.

Recently, Gao Fu's team of Institute of Microbiology, Chinese Academy of Sciences has successfully resolved the complex structure of Durvalumab antibody and human PD-L1 molecule through structural immunology platform, and clarified the mechanism of PD-L1 as targeted tumor treatment antibody (Protein & Cell, 2018, 9 (1), 135 - 139). It is found that both heavy and light chains of Durvalumab are involved in binding to PD-L1, and the folding of the spatial structure caused by binding prevents the binding of PD-L1 to PD-1. By analyzing the crystal complex of Atezolizumab, the first PD-L1 antibody approved by FDA (Oncotarget, 2017, 8, 90215 - 90224), it can be found that the binding of Atezolizumab and PD-L1 involves a large number of hydrogen bonds, hydrophobic interactions and π-π or cation-π interactions. In addition, mutation studies show that PD-L1 has two hot residues (E58, R113). In conclusion, Atezolizumab competes with PD-1for the same surface binding site of PD-L1.

Not long ago, Tad A. Holak, from the University of Jaguaron, Poland, published an article (J. Med. Chem. 2017, 60, 5857-5867), revealing the mechanism of small molecule inhibitors acting on PD-1/PD-L1 pathway, which is inducing PD-L1 dimerization when ligand small molecules bind to PD-L1 protein. PD-L1 of tumor cells cannot bind to PD-1 once dimerized. When PD-L1 of tumor cells cannot bind to PD-1 of T cells, tumor cells will be killed by activated T cells.

Due to the advantages of small molecule drugs in mass production and quality control, small-molecule PD-1/PD-L1 inhibitors are also being actively developed. WO2018006795 discloses a new small-molecule PD-1/PD-L1 inhibitor, which shows anti-tumor effect in mouse tumor model.

There is no public report about the combination of PD-L1 antibody and small-molecule PD-1/PD-L1 inhibitors. If this combination strategy can improve the therapeutic effect of tumor, it will have a profound impact on the immunotherapy of tumor.

### Content of the present invention

The technical problem to be solved in the present disclosure is for providing a small-molecule PD-1/PD-L1 inhibitor, a pharmaceutical composition thereof with PD-L1 antibody, and application of the same.

The present disclosure provides an aromatic vinyl or aromatic ethyl derivate represented by general formula (I), a pharmaceutically acceptable salt, a deuterated compound, a metabolite, a metabolic precursor or a prodrug thereof. 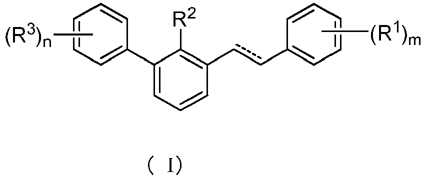 wherein,
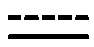 is a single bond or a double bond;
each of R¹ is identical or different, and is independently deuterium, halogen, substituted or unsubstituted hydroxyl, substituted or unsubstituted amino, substituted or unsubstituted alkyl, or substituted or unsubstituted alkoxy;
or two adjacent R¹ together with the two carbon atoms to which they are attached form a 5- to 7- membered carbocyclic ring or heterocarbocyclic ring; in the heterocarbocyclic ring, the heteroatom(s) is(are) oxygen and/or nitrogen, and the number of the heteroatom(s) is 1 to 4;
R² is substituted or unsubstituted alkyl or halogen;
each of R³ is identical or different, and is independently deuterium, halogen, substituted or unsubstituted alkylthio, substituted or unsubstituted hydroxyl, substituted or unsubstituted amino, substituted or unsubstituted alkyl, substituted or unsubstituted alkoxy, 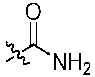 or 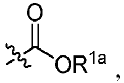 wherein R^{1a} is C₁-C₄ alkyl, or two adjacent R³ together with the two carbon atoms to which they are attached form a 5- to 7- membered carbocyclic ring or heterocarbocyclic ring; in the heterocarbocyclic ring, the heteroatom(s) is(are) oxygen and/or nitrogen, and the number of the heteroatom(s) is 1 to 4;
in each of R¹ or R³, the substituent(s) in the substituted alkyl, the substituted alkoxy or the substituted alkylthio is(are) one or more selected from halogen, C₁₋₄ alkyl, hydroxyl, 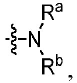 C₁₋₄ alkoxy, C₁₋₄ carboxyl, C₁₋₄ ester group and C₁₋₄ amide group;
when there are more substituents than one, the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C4 alkyl, hydroxyl, 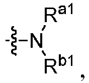 C₁-C₄ alkoxy, C₁-C₄ carboxyl, C₁-C₄ ester group and C₁-C4 amide group; R^{a1} and R^{b1} are independently hydrogen or C₁-C₄ alkyl;
in each of R¹ or R³, the substituent(s) in the substituted hydroxyl or the substituted amino is(are) one or more selected from C₁₋₄ alkyl, C₁₋₄ alkoxy, C₁₋₄ carboxyl, C₁₋₄ ester group and C₁₋₄ amide group;
m is 1, 2, 3, 4 or 5, preferably 1, 2 or 3;
n is 0, 1, 2, 3, 4 or 5, preferably 0, 1, 2 or 3;
when 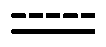 is a double bond and m is 2, then two R¹ are located on ortho and meta positions of the phenyl, respectively, such as 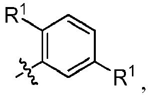 and the two R¹ are identical or different;
when 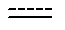 is a double bond and m is 3, then two of R¹ are adjacent, and the two adjacent R¹ together with the two carbon atoms to which they are attached form a 5- to 7- membered heterocarbocyclic ring;
the aromatic vinyl or aromatic ethyl derivative represented by general formula (I) excludes the compound as followed: 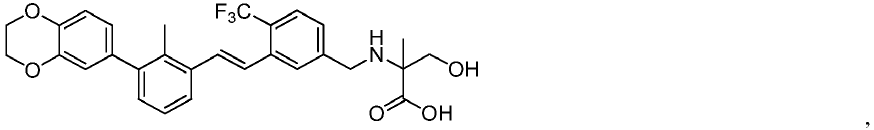 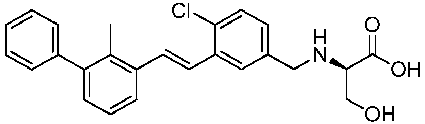 or

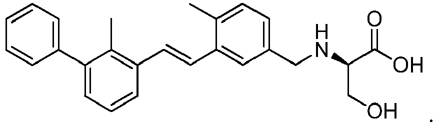

In one preferred embodiment of the present disclosure, each of R¹ is independently halogen, or, substituted or unsubstituted alkyl; or two adjacent R¹ together with the two carbon atoms to which they are attached form a 5- to 7- membered heterocarbocyclic ring.

In one preferred embodiment of the present disclosure, each of R¹ is independently halogen, substituted or unsubstituted alkyl, or, substituted or unsubstituted alkoxy.

In one preferred embodiment of the present disclosure, R² is alkyl or halogen.

In one preferred embodiment of the present disclosure, each of R³ is independently halogen, alkylthio or alkoxy; or two adjacent R³ together with the two carbon atoms to which they are attached form a 5- to 7- membered heterocarbocyclic ring.

In one preferred embodiment of the present disclosure, in each of R¹, the substituent(s) in the substituted alkyl is(are) one or more selected from halogen and 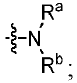 R^{a} and R^{b} are independently hydrogen, or, substituted or unsubstituted alkyl; in R^{a} or R^{b}, the substituent(s) in the substituted alkyl is(are) one or more selected from hydroxyl or C₁-C₄ carboxyl.

In one preferred embodiment of the present disclosure, in each of R¹, the substituent(s) in the substituted alkyl, the substituted alkoxy, the substituted hydroxyl, or, the substituted amino is(are) substituted by one or more than one halogen, preferably substituted by one or more than one F.

In one preferred embodiment of the present disclosure, when two R³ are adjacent, and the two adjacent R³ together with the two carbon atoms to which they are attached form a 5- to 7- membered carbocyclic ring or heterocarbocyclic ring, then the carbocyclic ring or heterocarbocyclic ring can be further substituted by one or more than one C₁₋₄ alkyl (such as

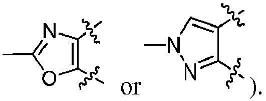

In one preferred embodiment of the present disclosure, 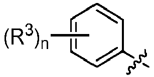 can be replaced by a substituted or unsubstituted heteroaromatic ring, in the heteroaromatic ring, the heteroatom(s) is(are) selected from nitrogen, oxygen and sulfur, and the number of the heteroatom(s) is 1 to 4; the substituent(s) in the substituted heteroaromatic ring is(are) one or more selected from halogen, C₁₋₄ alkyl, hydroxyl, 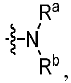 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 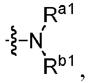 C₁-C₄ alkoxy, C₁-C₄ carboxyl, C₁-C₄ ester group and C₁-C₄ amide group; R^{a1} and R^{b1} are independently hydrogen or C₁-C₄ alkyl; the substituent(s) in the substituted heteroaromatic ring is(are) preferably one or more selected from C₁₋₄ alkyl.

In one preferred embodiment of the present disclosure, when 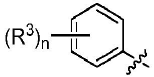 is replaced by a substituted or unsubstituted heteroaromatic ring, then in the heteroaromatic ring, the heteroatom(s) is(are) selected from nitrogen and oxygen, and the number of the heteroatom(s) is 1 to 3. Further preferably, when the heteroaromatic ring is a monocyclic ring, then the heteroatom(s) is(are) nitrogen, and the number of the heteroatom(s) is 1 or 2; when the heteroaromatic ring is a dicyclic heteroaromatic ring and the heteroatom(s) is(are) nitrogen, then the number of the heteroatom(s) is preferably 3; when the heteroaromatic ring is a dicyclic heteroaromatic ring, the heteroatom(s) is(are) selected from nitrogen and oxygen and the number of the heteroatoms is 2, then the heteroatoms are not adjacent.

In one preferred embodiment of the present disclosure, n is 0, 1, 2, 3, 4 or 5, preferably 0, 1, 2, or 3. When n is 1, then R³ is preferably halogen, alkylthio or alkoxy; and R³ is located on ortho, meta or para position of the phenyl, such as 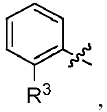 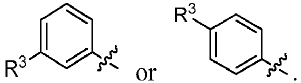 When n is 2, then preferably, two R³ are located on ortho and meta positions of the phenyl, such as 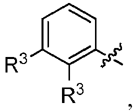 wherein, the two R³ are identical or different; or two R³ are adjacent, and the two adjacent R³ together with the two carbon atoms to which they are attached form a 5- to 7- membered heterocarbocyclic ring, such as 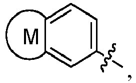 wherein, ring M is a 5- to 7- membered heterocarbocyclic ring. When n is 3, then preferably, two of R³ are adjacent, and the two adjacent R³ together with the two carbon atoms to which they are attached form a 5- to 7- membered heterocarbocyclic ring, such as 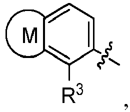 wherein, ring M is a 5- to 7- membered heterocarbocyclic ring (such as 2,3-dihydro-1,4-dioxane 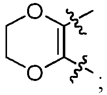 oxazole ring, such as 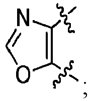 isoxazole ring, such as 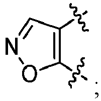 pyrazole ring, such as 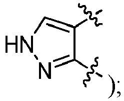 ring M can be further substituted by one or more than one C₁₋₄ alkyl (such as 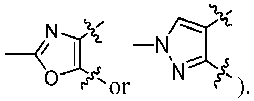 When n is 4 or 5, then preferably, R³ is deuterium.

In one preferred embodiment of the present disclosure, m is 2 or 3. When m is 2, then two R¹ are preferably located on ortho and meta positions of the phenyl, respectively, such as 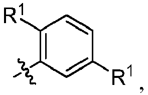 and the two R¹ are identical or different. When m is 3, then preferably, two of R¹ are adjacent, and the two adjacent R¹ together with the two carbon atoms to which they are attached form a 5- to 7- membered heterocarbocyclic ring, such as 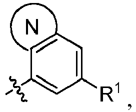 wherein, ring N is a 5- to 7- membered heterocarbocyclic ring, such as 2,3-dihydrofuran ring

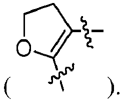

In one preferred embodiment of the present disclosure, when m is 2, then two R¹ are located on ortho and meta positions of the phenyl respectively, the two R¹ are identical or different; the R¹ located on ortho position of the phenyl is F, substituted or unsubstituted alkyl, or, substituted or unsubstituted alkoxy.

In one preferred embodiment of the present disclosure, when m is 2, then one of R¹ (preferably located on ortho position of the phenyl) is preferably alkyl or alkyl substituted by halogen; the other of R¹ (preferably located on meta position of the phenyl) is preferably alkyl substituted by 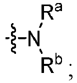 when m is 3, then two of R¹ are adjacent, and the two adjacent R¹ together with the two carbon atoms to which they are attached form a 5- to 7- membered heterocarbocyclic ring, and the third R¹ is alkyl substituted by 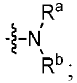 wherein, the alkyl is preferably C₁₋₄ alkyl. The alkyl substituted by halogen is preferably C₁-C₄ alkyl substituted by one or more halogen, such as trifluoromethyl. The alkyl substituted by 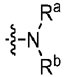 is preferably C₁-C₄ alkyl substituted by

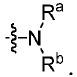

In one preferred embodiment of the present disclosure, the C₁-C₄ alkyl substituted by 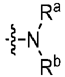 is preferably 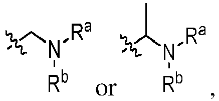 wherein, one of R^{a} and R^{b} is H, and the other is alkyl substituted by hydroxyl and/or carboxyl.

In one preferred embodiment of the present disclosure, the C₁-C₄ alkyl substituted by 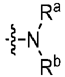 is preferably 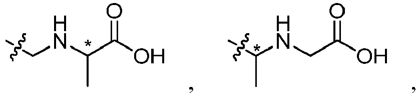 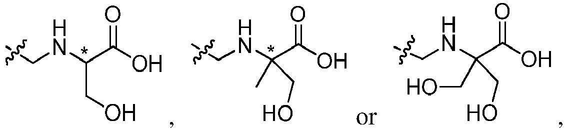 wherein, the carbon labelled by ^{∗} is an S-configuration chiral carbon, an *R*-configuration chiral carbon or an achiral carbon. Wherein, 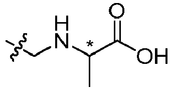 is preferably 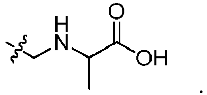 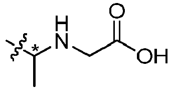 is preferably 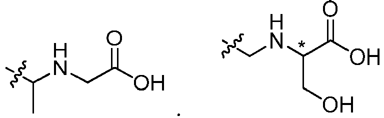 is preferably 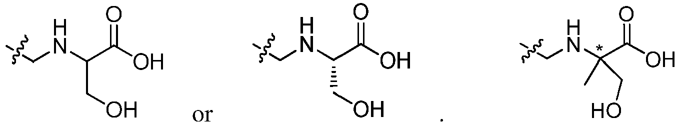 is preferably

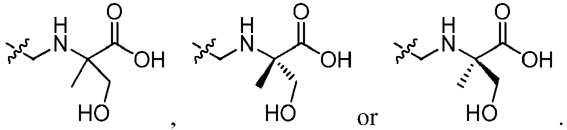

In one preferred embodiment of the present disclosure,
each of R¹ is independently halogen, or, substituted or unsubstituted alkyl; or two adjacent R¹ together with the two carbon atoms to which they are attached form a 5- to 7- membered heterocarbocyclic ring;
R² is alkyl or halogen;
each of R³ is independently deuterium, halogen, alkylthio, or alkoxy; or two adjacent R³ together with the two carbon atoms to which they are attached form a 5- to 7- membered heterocarbocyclic ring;
n is 0, 1, 2, 3, 4 or 5, preferably 0, 1, 2 or 3;
and m is 2.
In one preferred embodiment of the present disclosure,
each of R¹ is independently deuterium, or, substituted or unsubstituted alkyl; or two adjacent R¹ together with the two carbon atoms to which they are attached form a 5- to 7- membered heterocarbocyclic ring; the substituent(s) in the substituted alkyl is(are) one or more selected from halogen and 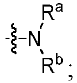
R^{a} and R^{b} are independently hydrogen, or, substituted or unsubstituted alkyl; in R^{a} or R^{b}, the substituent(s) in the substituted alkyl is(are) one or more selected from hydroxyl and C₁-C₄ carboxyl;
R² is alkyl or halogen;
each of R³ is independently deuterium, halogen, alkylthio or alkoxy; or two adjacent R³ together with the two carbon atoms to which they are attached form a 5- to 7- membered heterocarbocyclic ring;
n is 0, 1, 2, 3, 4 or 5, preferably 0, 1, 2 or 3, when n is 1, then R³ is preferably halogen, alkylthio or alkoxy; and R³ is located on ortho, meta or para position of the phenyl, such as 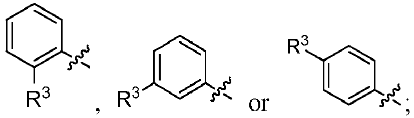 when n is 2, then preferably, two R³ are located on ortho and meta positions of the phenyl, such as 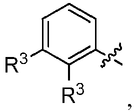 wherein, the two R³ are identical or different; or two R³ are adjacent, and the two adjacent R³ together with the two carbon atoms to which they are attached form a 5- to 7- membered heterocarbocyclic ring, such as 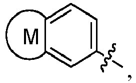 wherein, ring M is a 5- to 7- membered heterocarbocyclic ring; when n is 3, then preferably, one of R³ is halogen, the other two R³ are adjacent, and the two adjacent R³ together with the two carbon atoms to which they are attached form a 5- to 7- membered heterocarbocyclic ring, such as 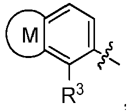 wherein, ring M is a 5- to 7- membered heterocarbocyclic ring;
and m is 2 or 3; when m is 2, then two R¹ are preferably located on ortho and para positions of the phenyl, respectively, such as 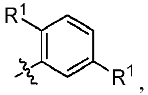 R¹ located on ortho position of the phenyl is preferably alkyl or alkyl substituted by halogen; R¹ located on meta position of the phenyl is preferably alkyl substituted by 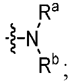 when m is 3, then two of R¹ are adjacent, and the two adjacent R¹ together with the two carbon atoms to which they are attached form a 5- to 7- membered heterocarbocyclic ring, and the third R¹ is alkyl substituted by 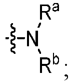 when n is 4 or 5, R³ is deuterium.

In one preferred embodiment of the present disclosure,
each of R¹ is independently halogen, substituted or unsubstituted alkyl, or, substituted or unsubstituted alkoxy;
R² is substituted or unsubstituted alkyl, or, halogen;
each of R³ is independently deuterium, halogen, substituted or unsubstituted alkylthio, substituted or unsubstituted hydroxyl, substituted or unsubstituted amino group, substituted or unsubstituted alkyl, substituted or unsubstituted alkoxy, 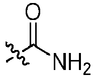 or 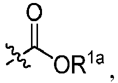 wherein, R^{1a} is C₁-C₄ alkyl;
in each of R¹, the substituent(s) in the substituted alkyl, the substituted alkoxy, the substituted hydroxyl, or, the substituted amino group is(are) substituted by one or more than one halogen, preferably substituted by one or more than one F;
the subsitituent(s) in the substituted alkyl in each of R² and R³, the substituted alkoxy and the substituted alkylthio in each of R³ is(are) one or more selected from halogen, C₁₋₄ alkyl, hydroxyl, 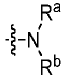 , C₁₋₄ alkoxy, C₁₋₄ carboxyl, C₁₋₄ ester group and C₁₋₄ amide group; when there are more subsitituents than one, then the subsitituents are identical or different; R^{a} and R^{b} are independently hydrogen, or, substituted or unsubstituted alkyl; in R^{a} or R^{b}, the subsitituent(s) in the substituted alkyl is(are) one or more selected from halogen, C₁-C₄ alkyl, hydroxyl, 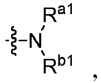 C₁-C₄ alkoxy, C₁-C₄ carboxyl, C₁-C₄ ester group or C₁-C₄ amide group; R^{a1} and R^{b1} are independently hydrogen, or, C₁-C₄ alkyl;
in each of R³, the substituent(s) in the substituted hydroxy or the substituted amino group is(are) one or more selected from C₁₋₄ alkyl, C₁₋₄ alkoxy, C₁₋₄ carboxyl, C₁₋₄ ester group and C₁₋₄ amide group;
m is 2 or 3;
n is 0, 1, 12, 3, 4 or 5, preferably 0, 1, 2 or 3;
when m is 2, then two R¹ are located on ortho and meta positions of the phenyl, respectively, two R¹ are identical or different; R¹ located on ortho position of the phenyl is F, substituted or unsubstituted alkyl, or substituted or unsubstituted alkoxy; when 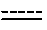 is a double bond and m is 3, then two R¹ are adjacent, and the two adjacent R¹ together with the two carbon atoms to which they are attached form a 5- to 7- membered carbocyclic ring;
or, in the aromatic vinyl or aromatic ethyl derivate represented by general formula (I), 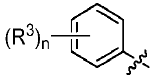 is replaced by a substituted or unsubstituted heteroaromatic ring, the heteroatom(s) in the heteroaromatic ring is(are) one or more selected from N, O and S, and the number of the heteroatom(s) is 1 to 4; the substituent(s) in the substituted heteroaromatic ring is(are) one or more selected from halogen, C₁₋₄ alkyl, hydroxyl, 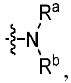 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 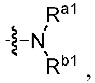 C₁-C₄ alkoxy, C₁-C₄ carboxyl, C₁-C₄ ester group and C₁-C₄ amide group; R^{a1} and R^{b1} are independently hydrogen or C₁-C₄ alkyl.

In one preferred embodiment of the present disclosure, 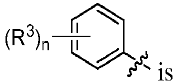 preferably

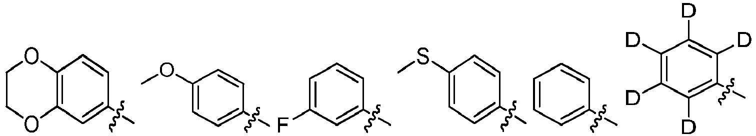

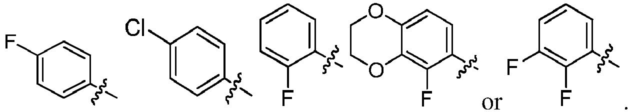

In one preferred embodiment of the present disclosure, 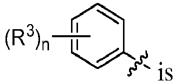 preferably

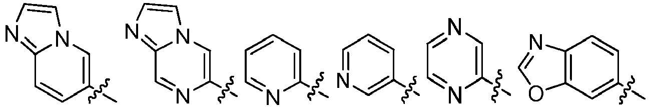

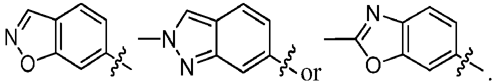

In one preferred embodiment of the present disclosure, 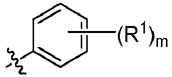 is preferably 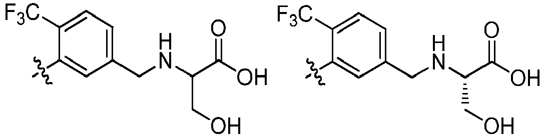 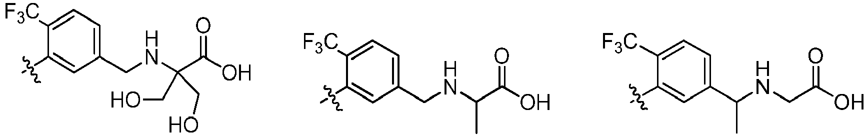 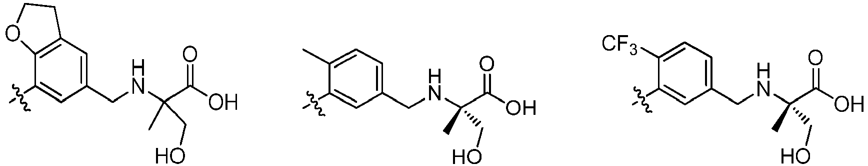 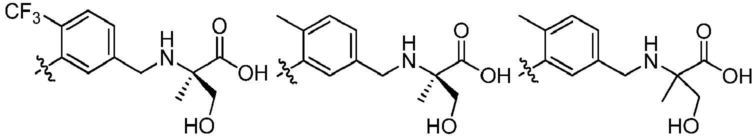 or

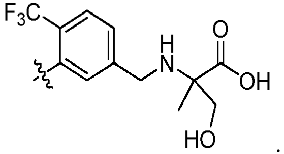

In one preferred embodiment of the present disclosure, when n is 1, R³ is halogen or alkoxy, and R³ is located on para position of the phenyl, then m is 2 or 3; when m is 2, then R¹ located on ortho position of the phenyl is preferably alkyl substituted by halogen.

In one preferred embodiment of the present disclosure, when n is 1 and R³ is halogen, then R³ is preferably F or Cl. When R³ is F, then R³ is preferably located on meta position of the phenyl.

In one preferred embodiment of the present disclosure, when n is 5, R³ is deuterium.

In one preferred embodiment of the present disclosure, in the aromatic vinyl or aromatic ethyl derivative represented by general formula (I), the pharmaceutically acceptable salt, the metabolite, the metabolic precursor or the prodrug thereof, 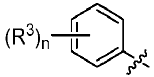 can be replaced by a substituted or unsubstituted heteroaromatic ring, and other variables and substituents are defined above.

In the present disclosure, the aromatic vinyl or aromatic ethyl derivative represented by general formula (I) is preferably any one of the compounds as followed: 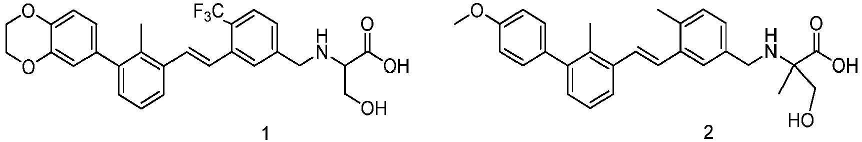 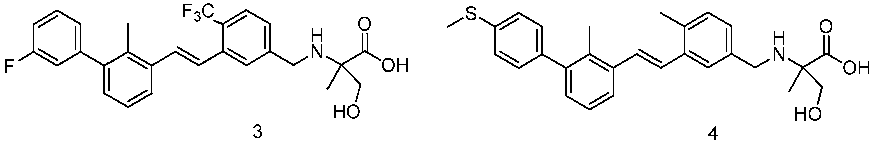 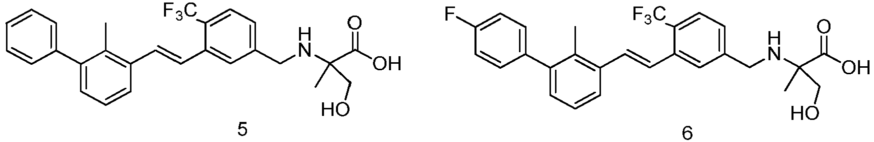 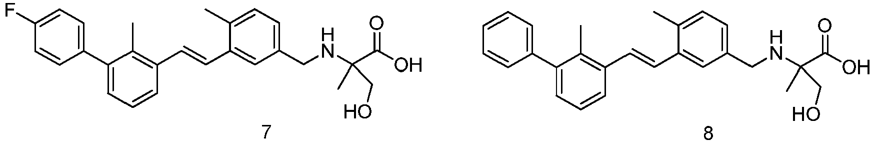 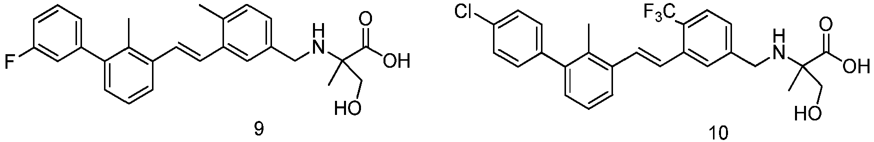 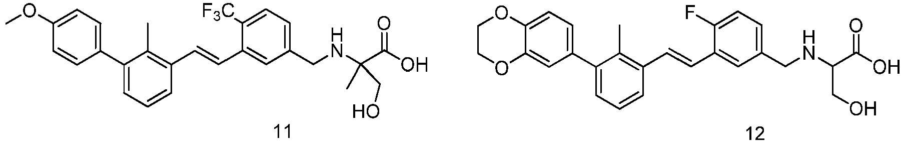 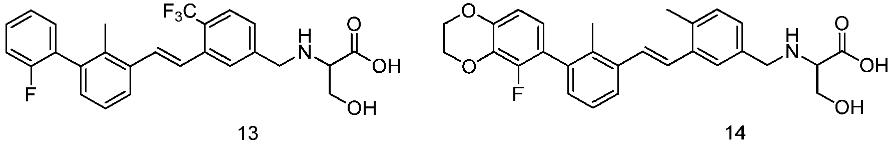 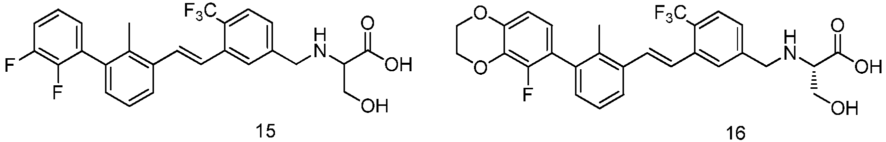 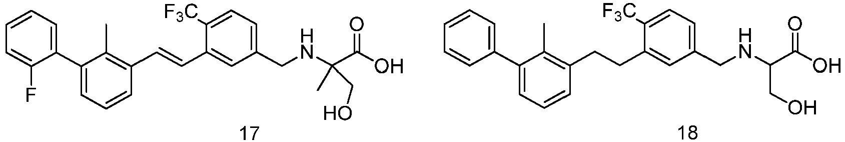 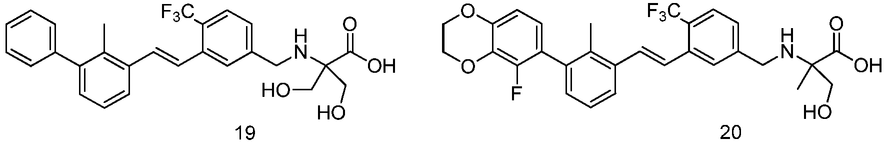 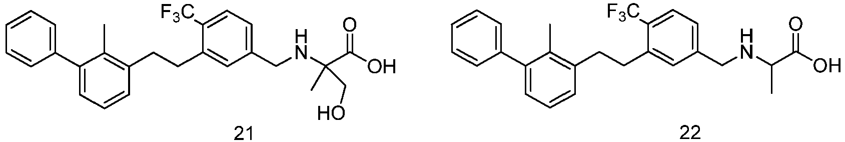 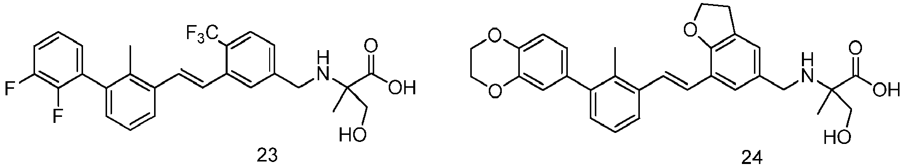 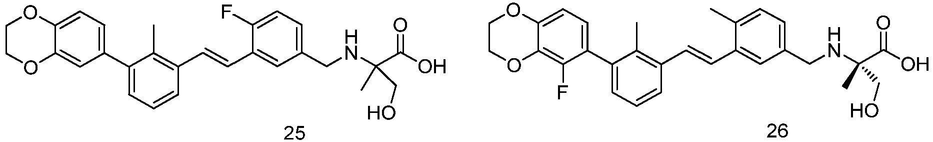 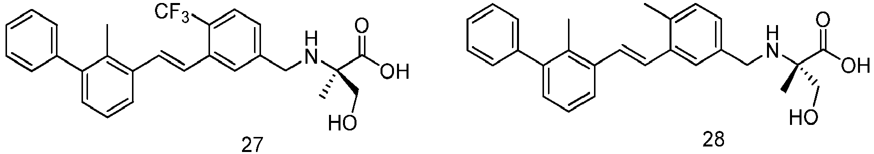 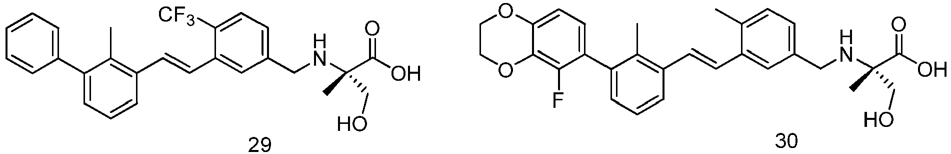 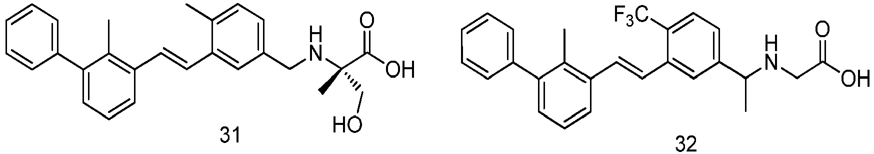 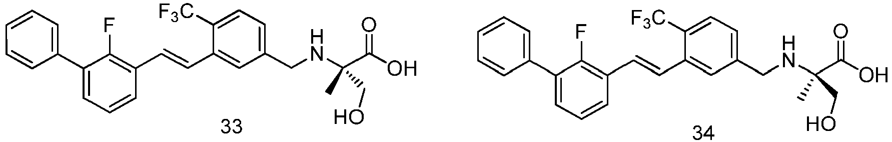 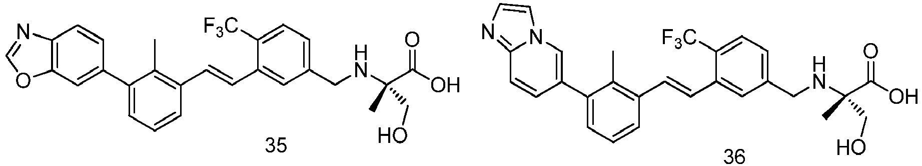 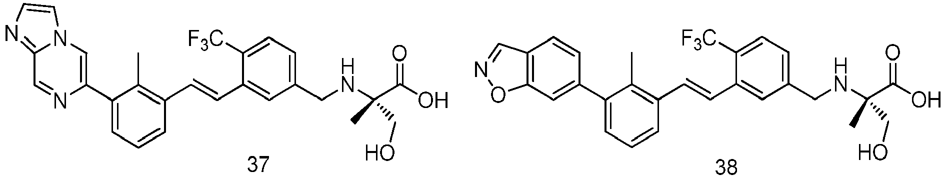 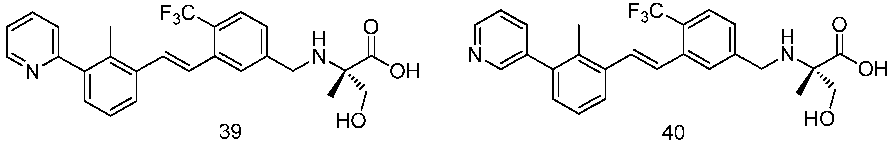 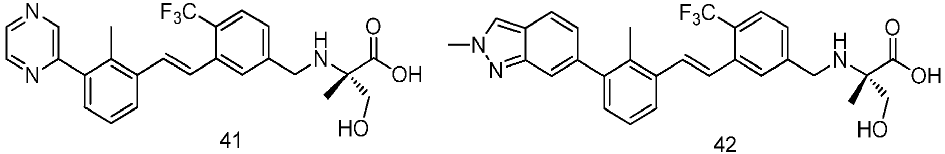 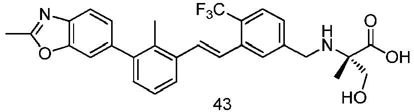 and

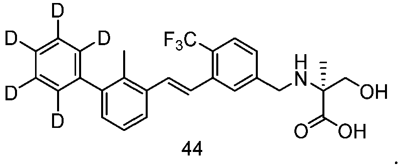

In one preferred embodiment of the present disclosure, the aromatic vinyl or aromatic ethyl derivative represented by general formula (I) is preferable an aromatic vinyl or aromatic ethyl derivative represented by general formula (II) 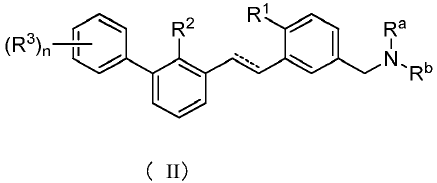 wherein, the definitions of R¹, R², R³, n, R^{a} and R^{b} are as defined above.

The present disclosure further provides a method for preparing the aromatic vinyl or aromatic ethyl derivative represented by general formula (II), which comprises the following step: conducting a reductive amination reaction of compound (I-a) with 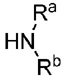 as followed to obtain the compound represented by general formula (II);

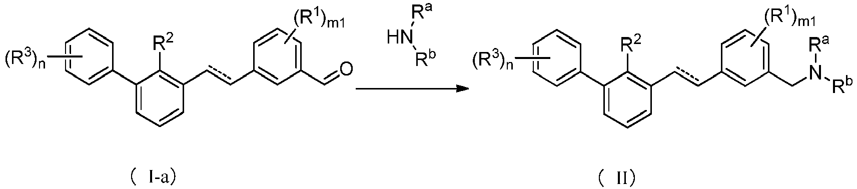

in the general formula above, the definitions of R¹, R², R³, n, R^{a} and R^{b} are as defined above, and m1 is 0, 1, 2, 3 or 4, preferably 0, 1 or 2. The methods and conditions of the reductive amination reaction are conventional methods and conditions for such reactions in the art.

An acid can be added in the reductive amination reaction. The acid is preferable an inorganic acid and/or an organic acid. The inorganic acid is preferable hydrochloric acid and/or sulfuric acid. The organic acid is preferable acetic acid. The molar ratio of the acid to the compound (I-a) is preferably 0.2:1 to 5:1 (such as 2:1).

The solvent is preferably an organic solvent and/or water. The organic solvent can be an organic solvent commonly used in such reactions in the art, preferably one or more selected from an alcohol solvent, an chlorinated hydrocarbon solvent, an ether solvent and an amide solvent. The alcohol solvent is preferably methanol and/or ethanol. The chlorinated hydrocarbon solvent is preferably dichloromethane. The ether solvent is preferably 1,4-dioxane. The amide solvent is preferably N,N-dimethylformamide. The solvent is preferably a mixed solvent of an alcohol solvent and a chlorinated hydrocarbon solvent, such as a mixed solvent of methanol and dichloromethane. In the mixed solvent of the alcohol solvent and the chlorinated hydrocarbon solvent, the volume ratio of the alcohol solvent to the chlorinated hydrocarbon solvent is preferably 1:0.1 to 1:5 (such as 1:1). The amount of the solvent can not be specifically limited as long as it does not affect the progress of the reaction, the volume-to-mass ratio of the solvent to the compound represented by compound (I-a) is preferably 10 mL/g to 200 mL/g.

The reductant can be a reductant commonly used in such reactions in the art, preferably one or more selected from sodium cyanoborohydride, sodium acetate borohydride, sodium borohydride and lithium borohydride, and preferably sodium cyanoborohydride. The molar ratio of the reductant to the compound (I-a) is preferably 0.3:1 to 10:1 (such as 5:1).

In the reductive amination reaction, the molar ratio of the compound (I-a) to 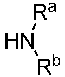 is 1:1 to 1:3 (such as 1:2).

The temperature of the reductive amination reaction is preferably 0 °C to 120 °C, more preferably 0 °C to 50 °C, further more preferably room temperature (10 °C to 30 °C).

The progress of the reductive amination reaction can be monitored by TLC or HPLC, generally the disappearance of the compound (I-a) is seen as completion of the reaction.

After the completion of the reductive amination reaction, the product can be further purified by a post-treatment. The methods of the post-treatment preferably comprises one or more methods selected from recrystallization, silica gel thin layer chromatography preparative plate purification (such as dichloromethane : methanol = 15:1), silica gel column chromatography purification and preparative high performance liquid chromatography purification (mobile phase: water (10 mM ammonium bicarbonate) and acetonitrile; the gradient: 25% to 55%).

A method for preparing the compound (I-a) preferably comprises the following step: in a solvent, in the action of a palladium catalyst, conducting a coupling reaction of compound (II-b) with 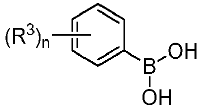 to obtain the compound (I-a); 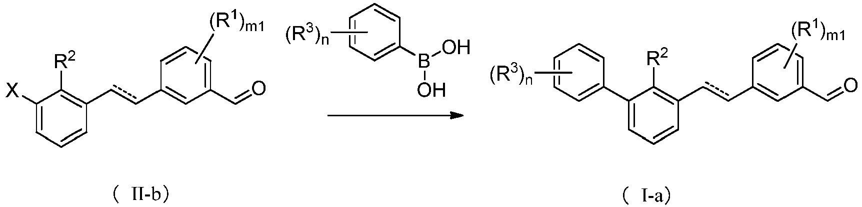 wherein the definitions of R¹, R², R³, n, R^{a} and R^{b} are as defined above, X is halogen, m1 is 0, 1, 2, 3 or 4, preferably 0, 1 or 2.

The methods and conditions of the coupling reaction are conventional methods and conditions for such reactions in the art.

In the method for preparing the compound (I-a), a base can also be added in the coupling reaction. The base is preferably an alkali metal carbonate, more preferably sodium carbonate, potassium carbonate or cesium carbonate. The molar ratio of the base to the compound (II-b) is preferably 1:1 to 5:1.

In the method for preparing the compound (I-a), the solvent is preferably an organic solvent and/or water. The organic solvent can be an organic solvent commonly used in such reactions in the art, preferably one or more selected from an ether solvent, an aromatic hydrocarbon solvent and an amide solvent. The ether solvent is preferably 1,4-dioxane and dimethoxyethane. The aromatic hydrocarbon solvent is preferably toluene. The amide solvent is preferably N,N-dimethylformamide. The solvent is preferably an aromatic hydrocarbon solvent. The volume-to-mass ratio of the solvent and the compound (II-b) is preferably 10 mL/g to 110 mL/g.

In the method for preparing the compound (I-a), the palladium catalyst can be a palladium catalyst commonly used in such coupling reactions, preferably selected from [1,1'-bis(diphenylphosphino)ferrocene] palladium dichloride, tris(dibenzylideneacetone)dipalladium, palladium acetate and tetrakis(triphenylphosphine)palladium. The molar ratio of the palladium catalyst to the compound (II-b) is preferably 0.005:1 to 0.5:1, more preferably 0.01:1 to 0.10:1.

In the method for preparing the compound (I-a), the coupling reaction is preferably carried out in the presence of a ligand, and the ligand is preferably 2-dicyclohexylphosphino-2',4',6'-triisopropylbiphenyl.

In the method for preparing the compound (I-a), the temperature of the coupling reaction is preferably 50°C to 150°C (such as 80°C to 90°C).

In the method for preparing the compound (I-a), the progress of the coupling reaction can be monitored by TLC or HPLC, generally the disappearance of the compound (II-b) is seen as completion of the reaction.

In the method for preparing the compound (I-a), after the completion of the coupling reaction, the product can be further purified by a post-treatment. The methods of the post-treatment preferably comprises one or more methods selected from recrystallization, silica gel thin layer chromatography preparative plate purification, silica gel column chromatography purification(eluent=petroleum ether: ethyl acetate) and preparative high performance liquid chromatography purification.

Those skilled in the art should understand that, after knowing the structure of the compounds of the present disclosure, the compounds of the present disclosure can be obtained by various methods well-known in the art and using well-known raw materials, such as chemical synthesis or extraction from plants, and these methods are included in the present disclosure. Unless otherwise stated or provided a preparation method, the raw materials used to prepare the compounds of the present disclosure or intermediates thereof are known in the art or are commercially available.

The present disclosure further provides a use of the aromatic vinyl or aromatic ethyl derivative represented by general formula (I), the pharmaceutically acceptable salt, the deuterated compound, the metabolite, the metabolic precursor or the prodrug thereof in manufacturing PD-1 inhibitors and/or PD-L1 inhibitors.

The present disclosure further provides a use of the aromatic vinyl or aromatic ethyl derivative represented by general formula (I), the pharmaceutically acceptable salt, the deuterated compound,the metabolite, the metabolic precursor or the prodrug thereof in manufacturing a medicament for preventing, alleviating or treating cancer, infection, autoimmune diseases or the related diseases thereof.

The cancer is preferably one or more selected from lung cancer, esophageal cancer, stomach cancer, colorectal cancer, liver cancer, nasopharyngeal cancer, brain tumor, breast cancer, cervical cancer, blood cancer and bone cancer.

The present disclosure also provides a pharmaceutical composition, which comprises a therapeutically and/or prophylactically effective amount of the aromatic vinyl or aromatic ethyl derivative represented by general formula (I), a pharmaceutically acceptable salt, a deuterated compound, a metabolite, a metabolic precursor or a prodrug thereof, and a pharmaceutically acceptable carrier and/or a diluent.

On the other hand, the present disclosure also provides a pharmaceutical composition, which comprises a PD-L1 antibody; and

a small-molecule PD-1/PD-L1 inhibitor or the pharmaceutically acceptable salt thereof.

The PD-L1 antibody is one or more selected from Atezolizumab, Durvalumab, Avelumab, Cemiplimab, KN035, CS1001, MBS2311, BGB-A333, KL-A167, SHR-1316 and STI-A1014.

In some embodiments of the present disclosure, the PD-L1 antibody is Durvalumab.

In some embodiments of the present disclosure, the small-molecule PD-1/PD-L1 inhibitor is an aromatic vinyl or aromatic ethyl derivative, a deuterated compound, a pharmaceutically acceptable salt, a metabolite, a metabolic precursor or a prodrug thereof.

In some embodiments of the present disclosure, the molecular weight of small-molecule PD-1/PD-L1 inhibitor is less than 1500 daltons, such as, less than 1400, 1300, 1200, 1100, 1000, 900, 800, 700, 600 or 500 daltons.

In some embodiments of the present disclosure, the IC₅₀ value of small-molecule PD-1/PD-L1 inhibitor in PD-1/PD-L1 binding experiments (such as the PD-1/PD-L1 binding experiments in Effect Example 1) is less than 100 nM, such as, less than 90 nM, 80 nM, 70 nM, 60 nM, 50 nM, 40 nM, 30 nM, 20 nM, 10 nM or 1 nM.

In some embodiments of the present disclosure, the small-molecule PD-1/PD-L1 inhibitor binds to PD-L1. In some embodiments of the present disclosure, the small-molecule PD-1/PD-L1 inhibitor binds to PD-1.

In some embodiments of the present disclosure, the small-molecule PD-1/PD-L1 inhibitor is a PD-L1 inhibitor. In some embodiments of the present disclosure, the small-molecule PD-1/PD-L1 inhibitor is a PD-1 inhibitor.

The small-molecule inhibitors of PD-1/PD-L1 disclosed in the present disclosure include the compounds disclosed in WO2015034820 (the contents of which are incorporated herein by reference in their entirety), such as

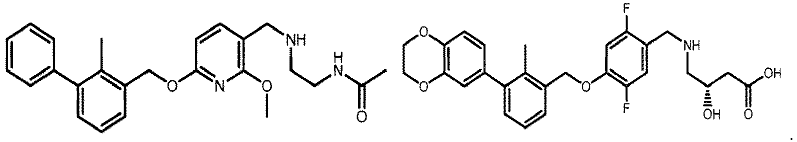

The small-molecule inhibitors of PD-1/PD-L1 disclosed in the present disclosure further include the compounds disclosed in WO2018009505 (the contents of which are incorporated herein by reference in their entirety), such as

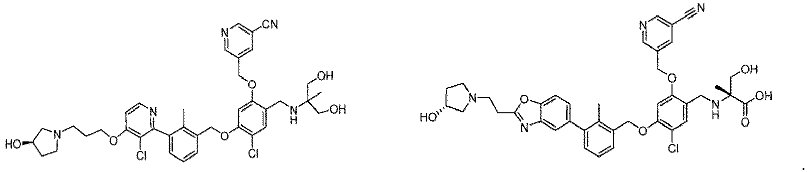

The small-molecule inhibitors of PD-1/PD-L1 disclosed in the present disclosure further include the compounds disclosed in WO2018195321 (the contents of which are incorporated herein by reference in their entirety), such as

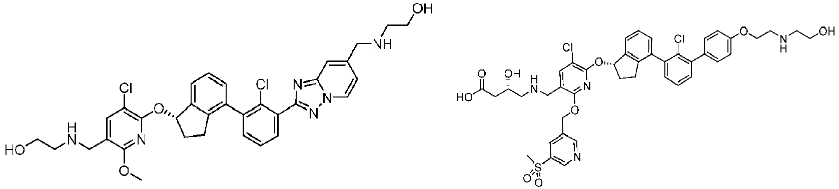

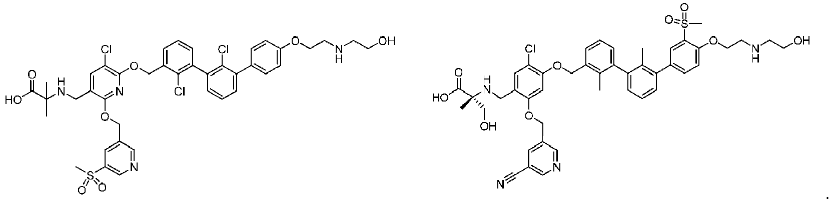

The small-molecule inhibitors of PD-1/PD-L1 disclosed in the present disclosure further include the compounds disclosed in WO2018119263 (the contents of which are incorporated herein by reference in their entirety), such as

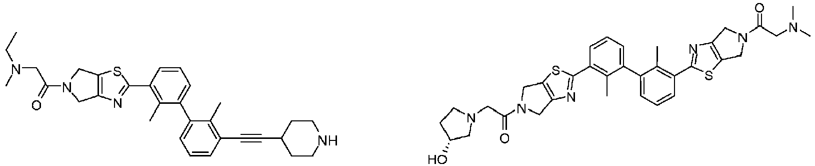

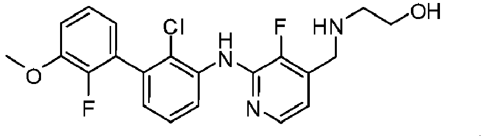

The small-molecule inhibitors of PD-1/PD-L1 disclosed in the present disclosure further include the compounds disclosed in WO2018119221 (the contents of which are incorporated herein by reference in their entirety), such as

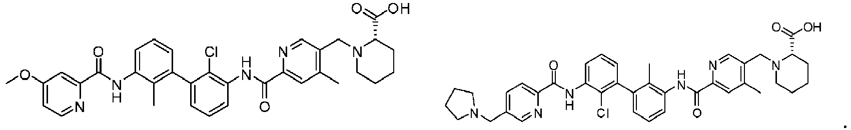

The small-molecule inhibitors of PD-1/PD-L1 disclosed in the present disclosure further include the compounds disclosed in WO2018119224 (the contents of which are incorporated herein by reference in their entirety), such as

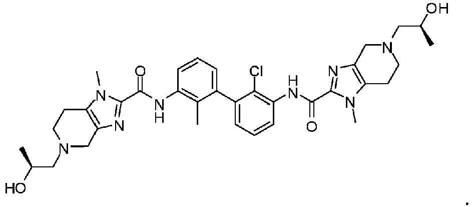

The small-molecule inhibitors of PD-1/PD-L1 disclosed in the present disclosure further include the compounds disclosed in WO2018119236 (the contents of which are incorporated herein by reference in their entirety), such as

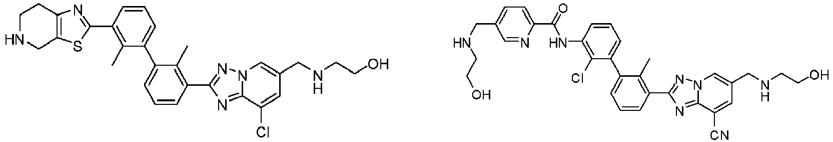

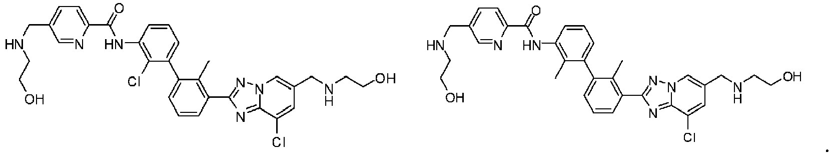

The small-molecule inhibitors of PD-1/PD-L1 disclosed in the present disclosure further include the compounds disclosed in WO2018119266 (the contents of which are incorporated herein by reference in their entirety), such as

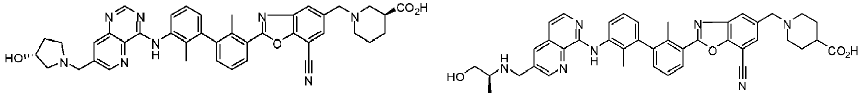

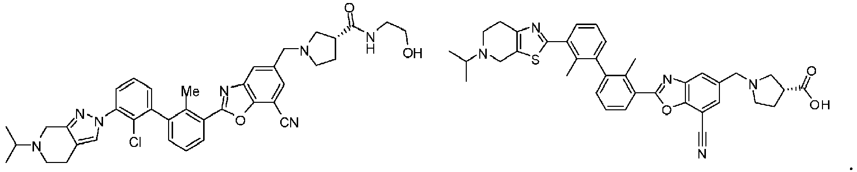

The small-molecule inhibitors of PD-1/PD-L1 disclosed in the present disclosure further include the compounds disclosed in WO2018119286 (the contents of which are incorporated herein by reference in their entirety), such as

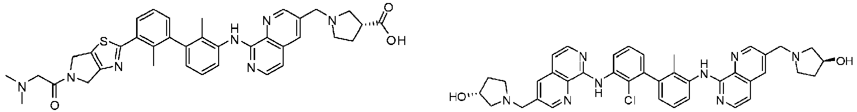

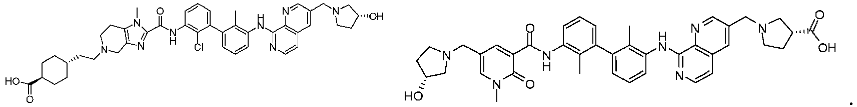

The small-molecule inhibitors of PD-1/PD-L1 disclosed in the present disclosure further include the compounds disclosed in WO2018044783 (the contents of which are incorporated herein by reference in their entirety), such as

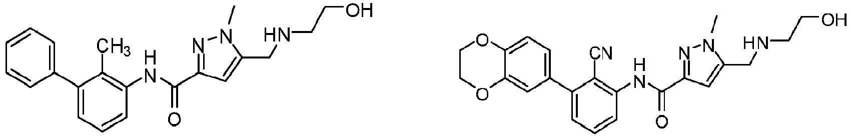

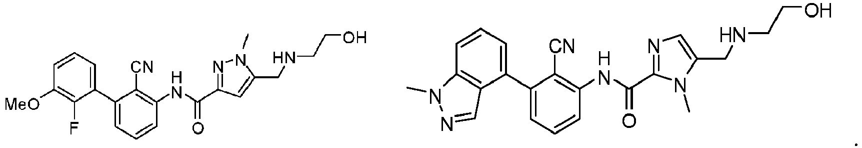

The small-molecule inhibitors of PD-1/PD-L1 disclosed in the present disclosure further include the compounds disclosed in WO2018013789 (the contents of which are incorporated herein by reference in their entirety), such as

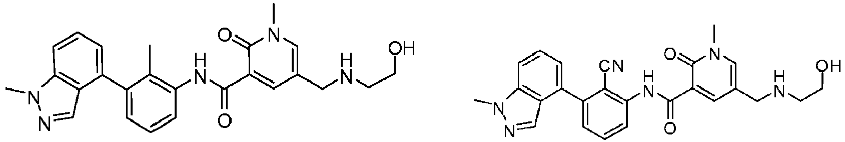

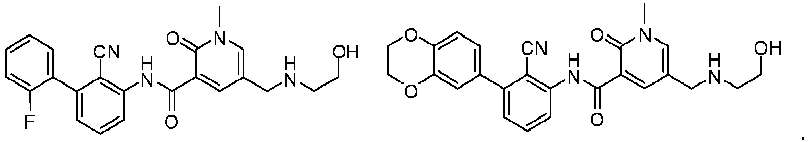

The small-molecule inhibitors of PD-1/PD-L1 disclosed in the present disclosure further include the compounds disclosed in WO2017222976 (the contents of which are incorporated herein by reference in their entirety), such as

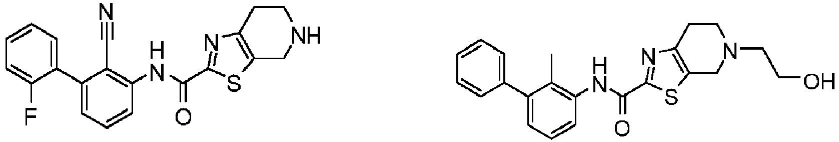

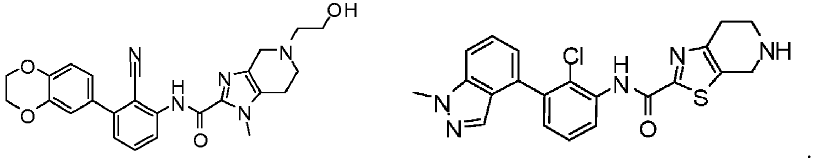

The small-molecule inhibitors of PD-1/PD-L1 disclosed in the present disclosure further include the compounds disclosed in WO2017205464 (the contents of which are incorporated herein by reference in their entirety), such as

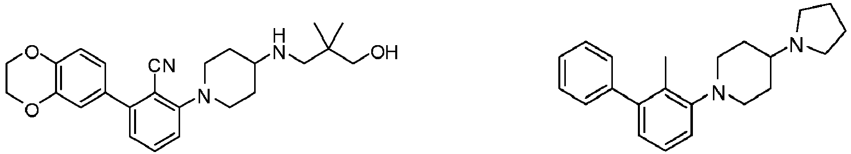

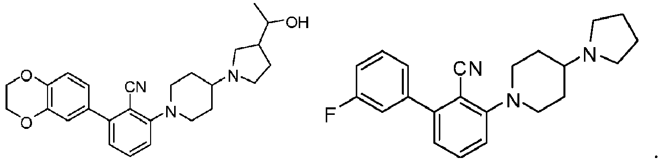

The small-molecule inhibitors of PD-1/PD-L1 disclosed in the present disclosure further include the compounds disclosed in WO2017192961 (the contents of which are incorporated herein by reference in their entirety), such as

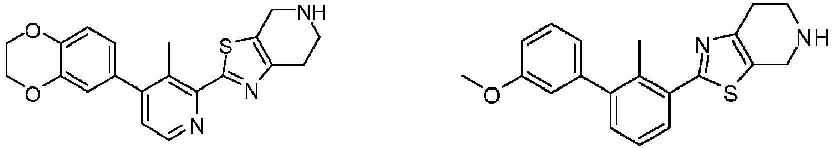

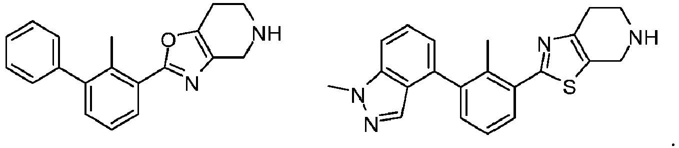

The small-molecule inhibitors of PD-1/PD-L1 disclosed in the present disclosure further include the compounds disclosed in WO2017112730 (the contents of which are incorporated herein by reference in their entirety), such as

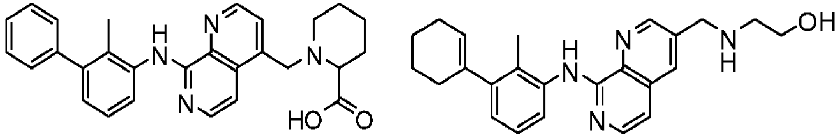

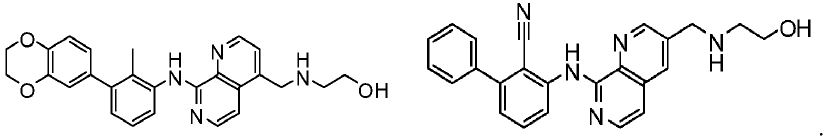

The small-molecule inhibitors of PD-1/PD-L1 disclosed in the present disclosure further include the compounds disclosed in WO2017106634 (the contents of which are incorporated herein by reference in their entirety), such as

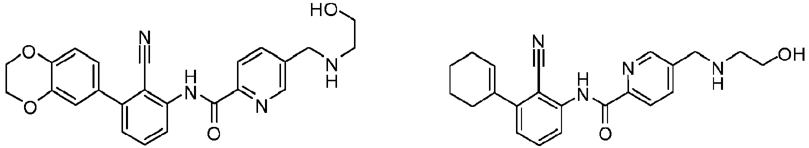

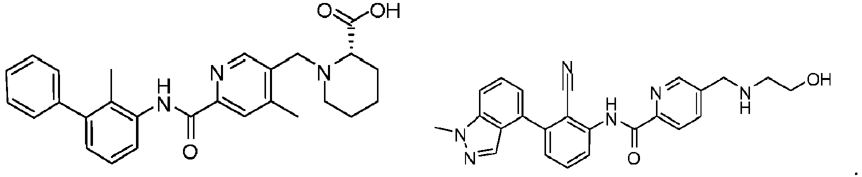

The small-molecule inhibitors of PD-1/PD-L1 disclosed in the present disclosure further include the compounds disclosed in WO2017087777 (the contents of which are incorporated herein by reference in their entirety), such as

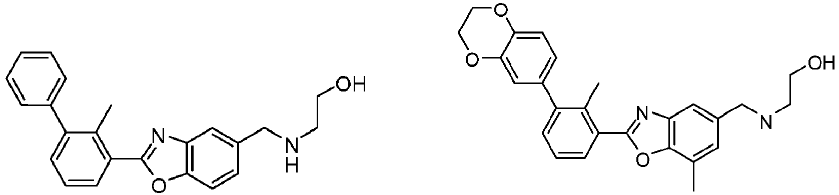

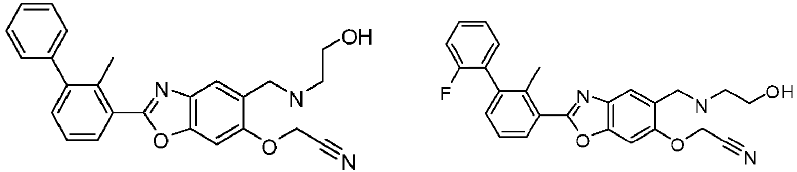

The small-molecule inhibitors of PD-1/PD-L1 disclosed in the present disclosure further include the compounds disclosed in WO2017070089(the contents of which are incorporated herein by reference in their entirety), such as

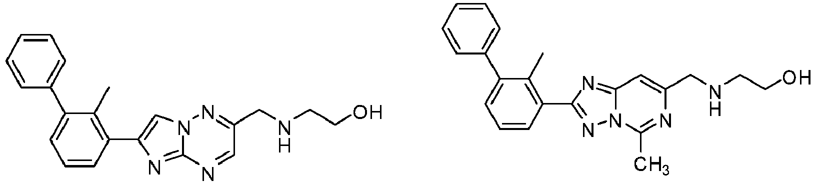

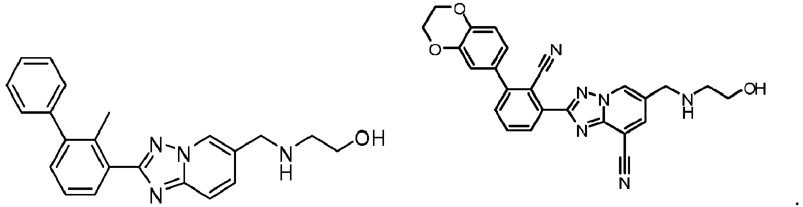

The small-molecule inhibitors of PD-1/PD-L1 disclosed in the present disclosure further include the compounds disclosed in WO2018026971 (the contents of which are incorporated herein by reference in their entirety), such as

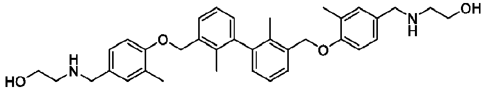

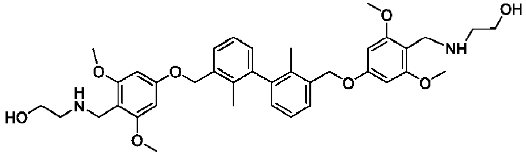

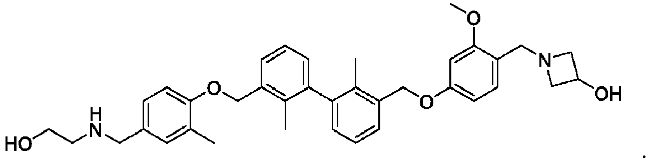

The small-molecule inhibitors of PD-1/PD-L1 disclosed in the present disclosure further include the compounds disclosed in WO2018005374 (the contents of which are incorporated herein by reference in their entirety), such as

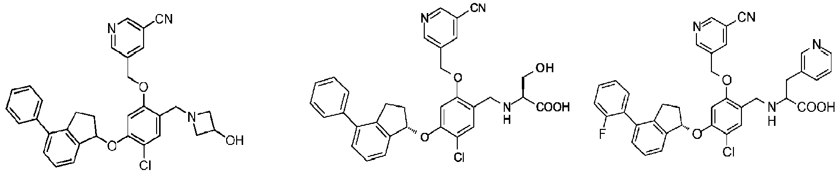

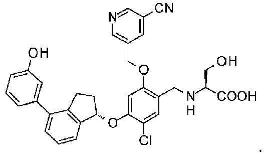

The small-molecule inhibitors of PD-1/PD-L1 disclosed in the present disclosure further include the compounds disclosed in WO2018051254 (the contents of which are incorporated herein by reference in their entirety), such as

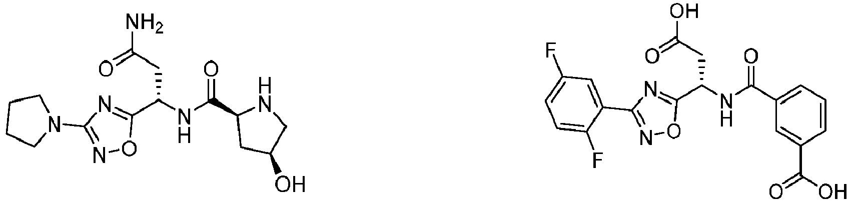

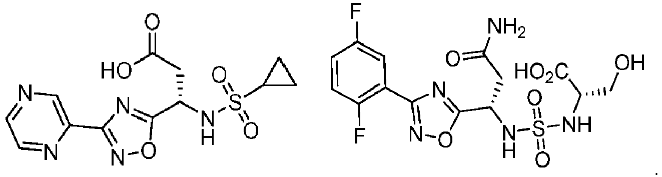

The small-molecule inhibitors of PD-1/PD-L1 disclosed in the present disclosure further include the compounds recorded in CN201711445262.9 (the contents of which are incorporated herein by reference in their entirety), such as

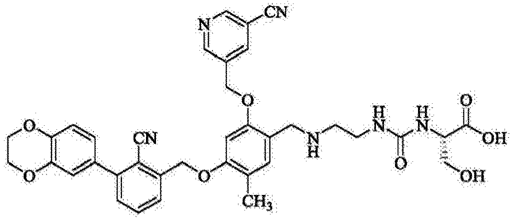

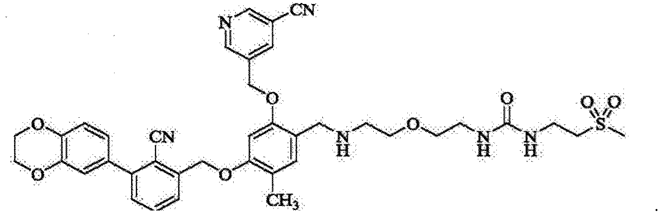

The small-molecule inhibitors of PD-1/PD-L1 disclosed in the present disclosure further include the compounds recorded in CN201710064453.4 (the contents of which are incorporated herein by reference in their entirety), such as

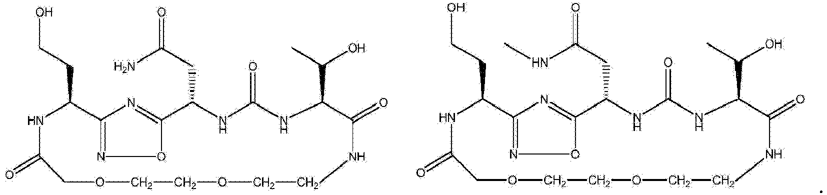

The small-molecule inhibitors of PD-1/PD-L1 disclosed in the present disclosure further include the compounds disclosed in WO2017202273 (the contents of which are incorporated herein by reference in their entirety), such as

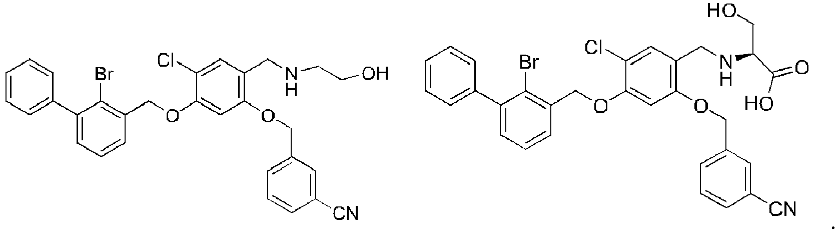

The small-molecule inhibitors of PD-1/PD-L1 disclosed in the present disclosure further include the compounds disclosed in WO2017202275 (the contents of which are incorporated herein by reference in their entirety), such as

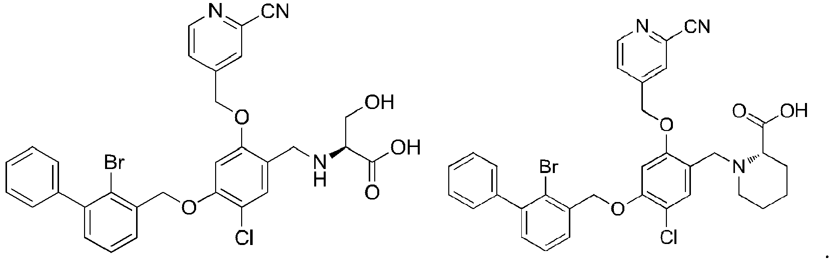

The small-molecule inhibitors of PD-1/PD-L1 disclosed in the present disclosure further include the compounds disclosed in WO2017202276 (the contents of which are incorporated herein by reference in their entirety), such as

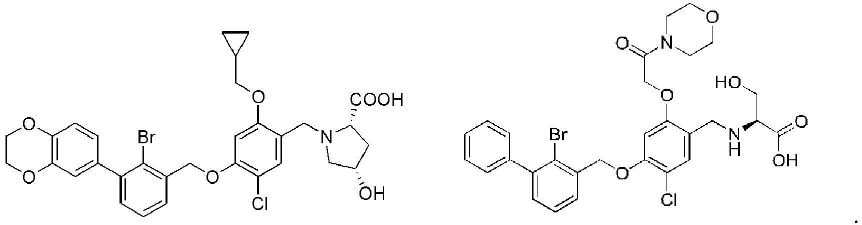

The small-molecule inhibitors of PD-1/PD-L1 disclosed in the present disclosure further include the compounds disclosed in WO2018006795 (the contents of which are incorporated herein by reference in their entirety).

The small-molecule inhibitors of PD-1/PD-L1 disclosed in the present disclosure further include the compounds disclosed in CN201710539028.6 (the contents of which are incorporated herein by reference in their entirety), such as 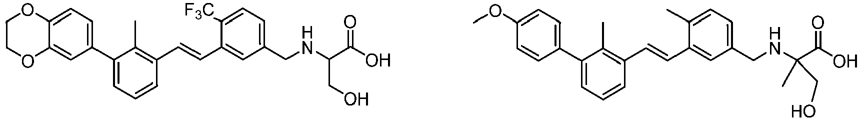 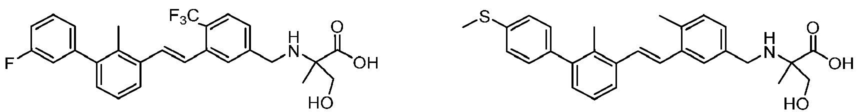 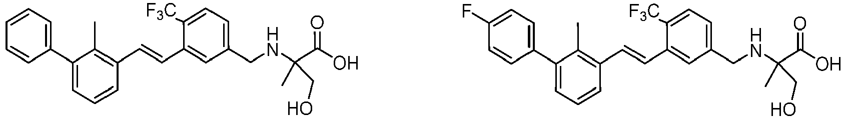 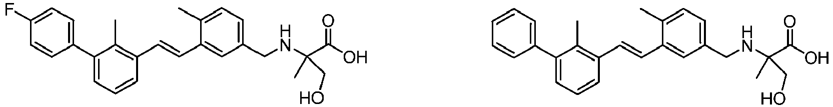 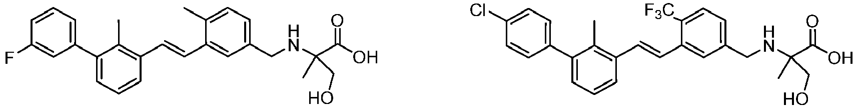 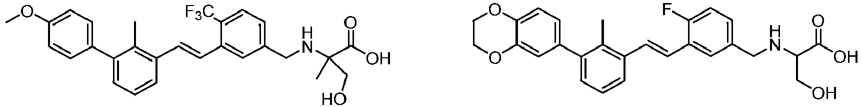 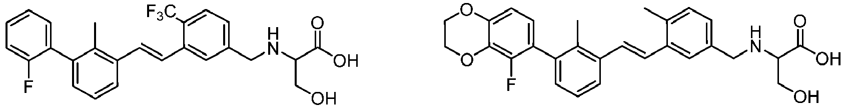 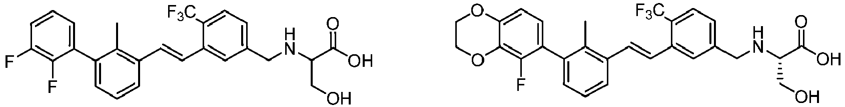 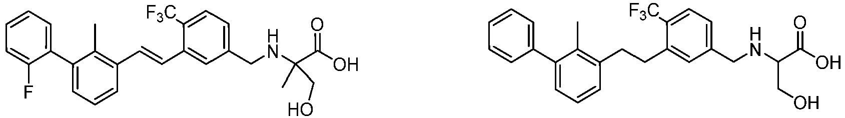 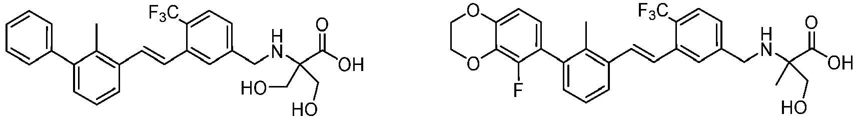 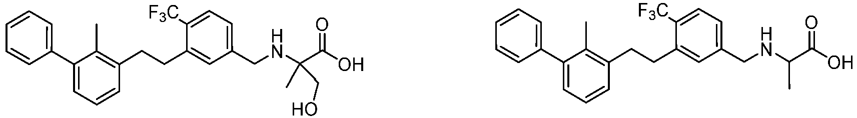 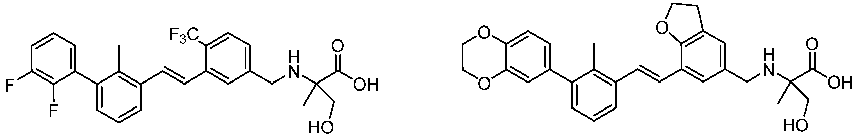 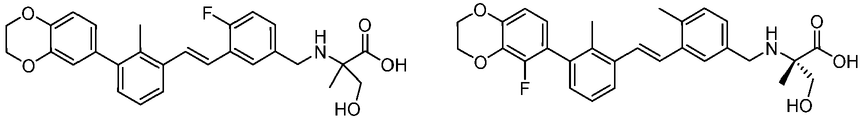 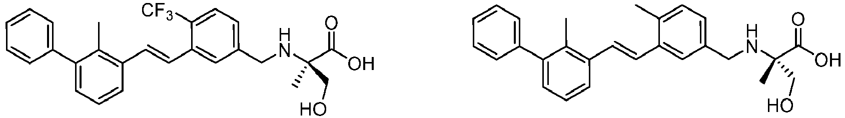 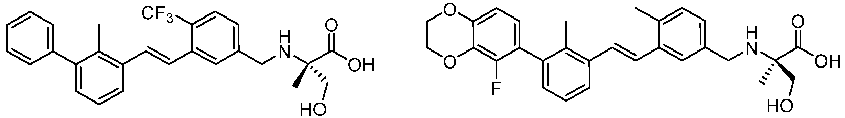 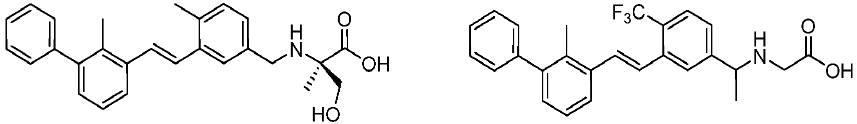 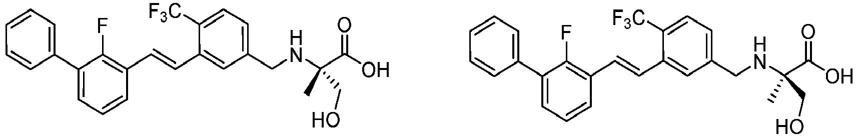 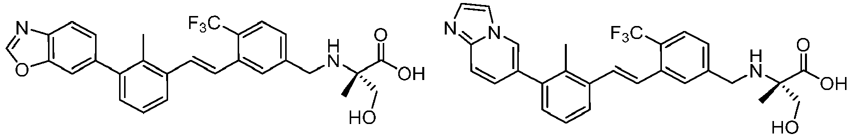 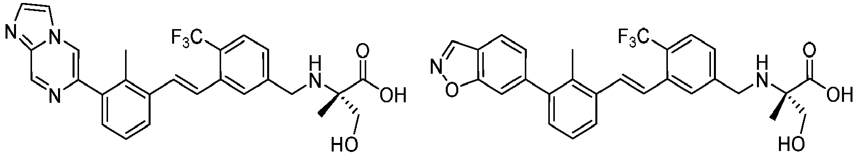 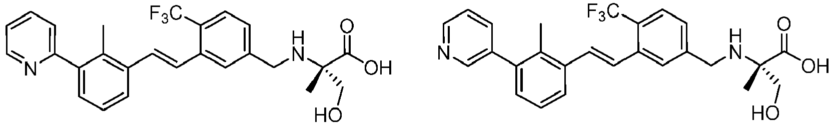 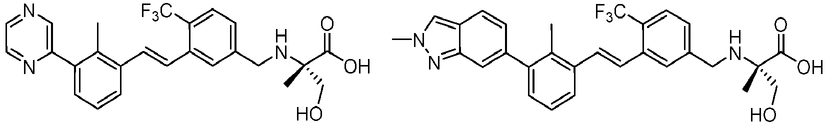 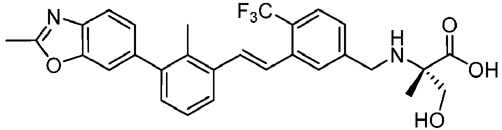 and

In some embodiments of the present disclosure, the small-molecule PD-1/PD-L1 inhibitor can be the aromatic vinyl or aromatic ethyl derivative represented by general formula (I), the pharmaceutically acceptable salt, the deuterated compound, the metabolite, the metabolic precursor or the prodrug thereof.

In some embodiments of the present disclosure, the small-molecule PD-1/PD-L1 inhibitor is compound 29 or the pharmaceutically acceptable salt thereof.

In some embodiments of the present disclosure, the PD-L1 antibody is Durvalumab; and, the small-molecule PD-1/PD-L1 inhibitor is compound 29 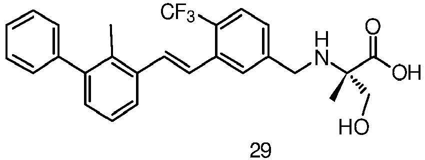 or the pharmaceutically acceptable salt thereof.

The PD-L1 antibody and small-molecule PD-1/PD-L1 inhibitor can exist in a single pharmaceutical composition or in separate pharmaceutical compositions. When the PD-L1 antibody and small-molecule PD-1/PD-L1 inhibitors need different routes of administration or different administration intervals, the PD-L1 antibody and small-molecule PD-1/PD-L1 inhibitor existed in separate individual pharmaceutical compositions are preferable.

The pharmaceutical composition further includes a pharmaceutically acceptable carrier.

In some embodiments of the present disclosure, the pharmaceutical composition includes:
a PD-L1 antibody;
a small-molecule PD-1/PD-L1 inhibitor or a pharmaceutically acceptable salt thereof; and,
a pharmaceutically acceptable carrier.

In some embodiments of the present disclosure, the pharmaceutical composition includes:
a first pharmaceutical composition, which includes a PD-L1 antibody and a pharmaceutically acceptable carrier;
a second pharmaceutical composition, which includes a small-molecule PD-1/PD-L1 inhibitor or a pharmaceutically acceptable salt thereof, and, a pharmaceutically acceptable carrier.

The pharmaceutical composition can be formulated into various dosage forms according to different administration methods, including gastrointestinal administration dosage form (such as oral dosage form) and non-gastrointestinal dosage form (such as injection dosage form). Wherein, the PD-L1 antibody and small-molecule PD-1/PD-L1 inhibitor can exist in a single unit dosage form or in different unit dosage forms. When the route of administration and administration interval of PD-L1 antibody and small-molecule PD-1/PD-L1 inhibitor are different, the PD-L1 antibody and small-molecule PD-1/PD-L1 inhibitor existed in different unit dosage forms are preferable.

In some embodiments of the present disclosure, the PD-L1 antibody is an injection, such as subcutaneous injection, intramuscular injection or intravenous injection.

In some embodiments of the present disclosure, the PD-L1 antibody is a subcutaneous injection.

In some embodiments of the present disclosure, the PD-L1 antibody is an intravenous injection.

In some embodiments of the present disclosure, the PD-L1 antibody is Durvalumab, which is an injection.

In some embodiments of the present disclosure, the PD-L1 antibody is Durvalumab, which is a subcutaneous injection.

In some embodiments of the present disclosure, the PD-L1 antibody is Durvalumab, which is an intravenous injection.

In some embodiments of the present disclosure, the small-molecule PD-1/PD-L1 inhibitor is in oral dosage form, such as solid oral dosage form (such as tablets, capsules, pills, granules) and liquid oral dosage form (such as oral solution, oral suspension, or, syrup).

In some embodiments of the present disclosure, the small-molecule PD-1/PD-L1 inhibitor is in liquid oral dosage form, such as oral suspension.

In some embodiments of the present disclosure, the small-molecule PD-1/PD-L1 inhibitor is Compound 29, which is in liquid oral dosage form, such as oral suspension.

In some embodiments of the present disclosure, the small-molecule PD-1/PD-L1 inhibitor is Compound 29, which is a suspension of Compound 29 in 10%(w/v) polyoxyl 40 hydrogenated caster oil (Cremophor RH40), 20%(w/v) sulfobutyl ether-*β-*cyclodextrin(SBE-*β*-CD) and 70%(w/v) water (i.e., suspending compound 29 in the mixed solution of 10%(w/v) polyoxyl 40 hydrogenated caster oil, 20%(w/v) sulfobutyl ether-*β*-cyclodextrin and 70%(w/v) water).

In some embodiments of the present disclosure, the PD-L1 antibody is Durvalumab; the small-molecule PD-1/PD-L1 inhibitor is Compound 29, or, the pharmaceutically acceptable salt thereof;
wherein, Durvalumab is an injection; Compound 29 is in oral dosage form.

In some embodiments of the present disclosure, the PD-L1 antibody is Durvalumab; the small-molecule PD-1/PD-L1 inhibitor is Compound 29, or, the pharmaceutically acceptable salt thereof;
wherein, Durvalumab is a subcutaneous injection; Compound 29 is in liquid oral dosage form.

In some embodiments of the present disclosure, the PD-L1 antibody is Durvalumab; the small-molecule PD-1/PD-L1 inhibitor is Compound 29;
wherein, Durvalumab is an intravenous injection; Compound 29 is in liquid oral dosage form.

In some embodiments of the present disclosure, the PD-L1 antibody is Durvalumab; the small-molecule PD-1/PD-L1 inhibitor is Compound 29;
wherein, Durvalumab is a subcutaneous injection; Compound 29 is a suspension of Compound 29 in 10%(w/v) polyoxyl 40 hydrogenated caster oil (Cremophor RH40), 20%(w/v) sulfobutylether-β-cyclodextrin (SBE-β-CD) and 70%(w/v) water.

The pharmaceutical composition can be presented in a unit dosage form containing a predetermined amount of active components. As those skilled in the art have known, the amount of active components per dose depends on the disease treated, the route of administration, and the age, weight and condition of the patients. The preferable unit dose composition contains a single dose, daily dose or subdose of active constituents or an appropriate dose thereof. In addition, the pharmaceutical composition can be prepared by any method known to the pharmaceutical field.

In some embodiments of the present disclosure, the content of PD-L1 antibody and small-molecule PD-1/PD-L1 inhibitor in pharmaceutical composition unit dosage form is therapeutically effective amount.

In the pharmaceutical composition unit dosage form, the content ratio of PD-L1 antibody to small-molecule PD-1/PD-L1 inhibitor can be 1:1-1:9, such as 1:1, 1:2, 1:3, 1:4, 1:5, 1:6, 1:7, 1:8, or, 1:9.

On the other hand, the present disclosure provides a use of the pharmaceutical composition in manufacturing a medicament for treating cancer.

On the other hand, the present disclosure provides a method for treating cancer, which includes administering therapeutically effective amount of the pharmaceutical composition to the subject in need thereof.

In some embodiments of the present disclosure, the cancer is lung cancer, stomach cancer, colorectal cancer, cervical cancer, ovarian cancer, prostate cancer, breast cancer, pancreatic cancer, liver cancer, bladder cancer, renal cancer, bone cancer, skin cancer, melanoma, glioma, neuroblastoma, leukemia, or, lymphoma.

In some embodiments of the present disclosure, the cancer is lung cancer, colorectal cancer, breast cancer, melanoma, leukemia, or, lymphoma.

In some embodiments of the present disclosure, the cancer is colorectal cancer.

In some embodiments of the present disclosure, the cancer is colon cancer.

The dosage regimen (such as, routes of administration, dosage, administration interval) of the PD-L1 antibody and small-molecule PD-1/PD-L1 inhibitor can be adjusted by those skilled in the art as required to provide the optimal therapeutic effect.

The way to administer the PD-L1 antibody and small-molecule PD-1/PD-L1 inhibitor can be simultaneous administration or separate administration (such as, sequential administration). The "simultaneous administration" means PD-L1 antibody and small-molecule PD-1/PD-L1 inhibitor can be simultaneously administered in a single pharmaceutical composition containing PD-L1 antibody and small-molecule PD-1/PD-L1 inhibitor. The "separate administration" means PD-L1 antibody and small-molecule PD-1/PD-L1 inhibitor can be administered sequentially in the pharmaceutical composition containing one of the PD-L1 antibody and small-molecule PD-1/PD-L1 inhibitor separately, such as, PD-L1 antibody or small-molecule PD-1/PD-L1 inhibitor is administered first and the other is administered thereafter. The time interval between two administrations can be short (such as, simultaneously) or long.

The routes of administration of PD-L1 antibody can be any route of administration in the art, which includes oral administration, injection (such as, intravenous, intramuscular, subcutaneous) etc.

In some embodiments of the present disclosure, the PD-L1 antibody is administered by injection.

In some embodiments of the present disclosure, the PD-L1 antibody is administered by intravenous injection.

In some embodiments of the present disclosure, the PD-L1 antibody is administered by subcutaneous injection.

In some embodiments of the present disclosure, the PD-L1 antibody is Durvalumab, which is administered by injection.

In some embodiments of the present disclosure, the PD-L1 antibody is Durvalumab, which is administered by intravenous injection.

In some embodiments of the present disclosure, the PD-L1 antibody is Durvalumab, which is administered by subcutaneous injection.

The PD-L1 antibody (such as Durvalumab) can be administered according to the body weight of the subjects, non-restricted case range can be 1.0-1000 mg/kg, such as, 10-150 mg/kg, such as, 5 mg/kg, 10 mg/kg, 15 mg/kg, 20 mg/kg, 30 mg/kg, 40 mg/kg, 50 mg/kg, 60 mg/kg, 70 mg/kg, 80 mg/kg, 90 mg/kg, 100 mg/kg, 110 mg/kg, 120 mg/kg, or, 150 mg/kg. In some embodiments of the present disclosure, the dose of PD-L1 antibody is 20 mg/kg. These doses can be administered QD (once a day), BID (twice a day), TID (three times a day), QOD (every other day), QW (once a week), BIW (twice a week) or Q2W (once every two weeks). In some embodiments of the present disclosure, the PD-L1 antibody is administered BIW. In some embodiments of the present disclosure, the PD-L1 antibody (such as Durvalumab) is administered by injection (such as intravenous injection, subcutaneous injection) according to the dose and frequency above. In some embodiments of the present disclosure, the PD-L1 antibody is Durvalumab, administered at a dose of 20 mg/kg BIW by injection (such as intravenous injection, subcutaneous injection).

The PD-L1 antibody (such as Durvalumab) can also be applied at a fixed dose in subjects, which means given at a fixed dose or predetermined dose to subjects.

The small-molecule PD-1/PD-L1 inhibitor can be administered by any appropriate routes in the art, including oral administration, injection (such as intravenous, intramuscular, subcutaneous) etc..

In some embodiments of the present disclosure, the small-molecule PD-1/PD-L1 inhibitor is administered by oral administration.

In some embodiments of the present disclosure, the small-molecule PD-1/PD-L1 inhibitor is Compound 29, administered by oral administration.

The dosage regimen of small-molecule PD-1/PD-L1 inhibitor can be adjusted as needed to provide optimal therapeutic effect

The small-molecule PD-1/PD-L1 inhibitor (such as Compound 29) can be administered according to the body weight of the subjects, non-restricted case range can be 1.0-1000 mg/kg, such as, 5-180 mg/kg, such as, 5 mg/kg, 10 mg/kg, 15 mg/kg, 20 mg/kg, 30 mg/kg, 40 mg/kg, 50 mg/kg, 60 mg/kg, 70 mg/kg, 80 mg/kg, 90 mg/kg, 100 mg/kg, 110 mg/kg, 120 mg/kg, or, 150 mg/kg. In some embodiments of the present disclosure, the dose of PD-L1 antibody is 15-180 mg/kg, such as 30-120 mg/kg, 60-120 mg/kg. These doses can be administered QD (once a day), BID (twice a day), TID (three times a day), QOD (every other day), QW (once a week), BIW (twice a week) or Q2W (once every two weeks). In some embodiments of the present disclosure, the small-molecule PD-1/PD-L1 inhibitor is administered BID. In some embodiments of the present disclosure, the small-molecule PD-1/PD-L1 inhibitor (such as Compound 29) is administered orally according to the dose and frequency above. In some embodiments of the present disclosure, the small-molecule PD-1/PD-L1 inhibitor is Compound 29, administered orally at a dose of 60 mg/kg BID.

The small-molecule PD-1/PD-L1 inhibitor (such as Compound 29) can be applied at a fixed dose in subjects, which means given at a fixed dose or predetermined dose to subjects.

In some embodiments of the present disclosure, the PD-L1 antibody is Durvalumab, administered at a dose of 20 mg/kg BIW by injection(such as, intravenous injection, subcutaneous injection); and,
the small-molecule PD-1/PD-L1 inhibitor is Compound 29, administered orally at a dose of 60 mg/kg BID.

When the PD-L1 antibody and small-molecule PD-1/PD-L1 inhibitor are administered separately, the PD-L1 antibody and small-molecule PD-1/PD-L1 inhibitor can be administered continuously according to their respective administration-period separately. The administration-period of the PD-L1 antibody and small-molecule PD-1/PD-L1 inhibitor can start at the same or different time. For example, the PD-L1 antibody and small-molecule PD-1/PD-L1 inhibitor can be administered continuously at the same day according to their respective administration period. Alternatively, the small-molecule PD-1/PD-L1 inhibitor is administered on the second or third or more days after PD-L1 antibody is administered, and then the PD-L1 antibody and small-molecule PD-1/PD-L1 inhibitor are continuously administered according to their respective administration period.

The pharmaceutical composition can be presented in the form of a combined kit (or a set of kits). The term "a combined kit" or "a set of kits" is a container for applying one or more pharmaceutical compositions of the PD-L1 antibody and small-molecule PD-1/PD-L1 inhibitor. When the PD-L1 antibody and small-molecule PD-1/PD-L1 inhibitor are applied simultaneously, the combined kit could contain PD-L1 antibody and small-molecule PD-1/PD-L1 inhibitor in a single pharmaceutical composition. When the PD-L1 antibody and small-molecule PD-1/PD-L1 inhibitor are not applied simultaneously, the combined kit will contain a first pharmaceutical composition containing PD-L1 antibody and a second pharmaceutical composition containing small-molecule PD-1/PD-L1 inhibitor.

In some embodiments of the present disclosure, the combined kit contains:
a PD-L1 antibody;
a small-molecule PD-1/PD-L1 inhibitor; and,
a pharmaceutically acceptable carrier;
wherein, the PD-L1 antibody and small-molecule PD-1/PD-L1 inhibitor are provided in the form for applying simultaneously and/or applying separately.

In some embodiments of the present disclosure, the combined kit contains:
a first container, containing a first pharmaceutical composition, the first pharmaceutical composition comprises a PD-L1 antibody and a pharmaceutically acceptable carrier;
a second container, containing a second pharmaceutical composition, the second pharmaceutical composition comprises a small-molecule PD-1/PD-L1 inhibitor, or, a pharmaceutically acceptable salt thereof and a pharmaceutically acceptable carrier.

The pharmaceutical composition can be used individually or combined with one or more therapeutic agents (such as, immunomodulators, other antitumour agents etc). Therefore, the pharmaceutical composition can include PD-L1 antibody, small-molecule PD-1/PD-L1 inhibitor and other available therapeutic agents. Such pharmaceutical combinations can be applied together or separately, the time interval can be either short (even simultaneously) or long, and the order can be any when applied separately.

The pharmaceutical composition can be used together with other treatment methods (such as, chemotherapy, surgery and/or radiotherapy) for cancer treatment.

The term "small-molecule PD-1/PD-L1 inhibitor" refers to any small-molecule compound that can directly or indirectly reduce or inhibit (partially or completely) the activity of PD-L1/PD-1 pathway, which can be expressed as "small molecule PD-1/PD-L1 pathway inhibitor". Small-molecule PD-1/PD-L1 inhibitor can inhibit PD-1, PD-L1, and/or interact with PD-1/PD-L1, wherein, "small-molecule"should be understood as a concept relative to biomacromolecule, that is, small-molecule does not include biomacromolecule drug (such as antibody) and should not be understood as unclear.

The term "antibody" is intended to include complete molecules and fragments containing antigen binding sites that can bind epitopes

The term "PD-L1" includes homotypes, mammals, such as human PD-L1, homologous species of human PD-L1, and analogues containing at least one common epitope with PD-L1. The amino acid sequence of PD-L1, such as human PD-L1, is known in the art.

The term "homologous" refers to the homogeneity of subunit sequence between two polymer molecules, for example, between two nucleic acid molecules, such as between two DNA molecules, or, between two RNA molecules, or, between two polypetide molecules. When two molecules are occupied by the same monomeric subunit at one subunit position, for example, if each of the two DNA molecules is occupied by adenine in one position, they are homologous or identical in that position. Homology between two sequences is a direct function of the number of matching or homologous positions; for example, if half of the positions (for example, five positions in a polymer of ten subunits) are homologous, then the two sequences are 50% homologous; if 90% of the positions (for example, 9 out of 10) are matched or homologous, then the two sequences are 90% homologous.

The term "PD-L1 antibody" can be polyclonal or monoclonal. Wherein, the term "monoclonal antibody" only shows binding specificity and affinity to a single specific epitope. Monoclonal antibody can be prepared by hybridoma technique or without hybridoma technique (such as, recombination).

The PD-L1 antibody can be humanized. The term "humanized" refers to the form of non-human (e.g., mouse) antibodies containing the smallest sequence of non-human immunoglobulins, which can be chimeric immunoglobulins, immunoglobulin chains or fragments.

The PD-L1 antibody also includes an antibody molecule with more than 80% homologous, preferably more than 80%, more preferably more than 91%, 92%, 93%, 94%, 95%, 96%, 97%, 98% or 99%.

The term "treatment" refers to therapeutic treatment. When involving specific diseases, treatment refers to: (1) alleviating one or more biological manifestations of the disease or condition, (2) interfering with one or more points in the biological cascade of disease led or induced by (a) or one or more biological manifestations of (b) condition, (3) improving one or more symptoms, effects or side effects associated with the disease, or one or more symptoms, effects or side effects associated with the disease or the treatment of disease, or (4) mitigating disease or one or more progressions of biological manifestations of the disease.

In some embodiments of the present disclosure, the compound of the present disclosure is administered as a preventive measure. As used in the present disclosure, "prevention" or "ongoing prevention" means reducing the risk of acquiring a given disease or condition. In the preferred model of the embodiments, the specified compounds are given to subjects as preventive measures, such as subjects with a family history or tendency of cancer or autoimmune diseases.

The term "therapeutically effective amount" refers to an amount of the compound administered to a subject sufficient to treat the diseases involved in the present disclosure. The therapeutically effective amount of a compound will vary depending on the compound, condition and its severity, age of the subject to be treated, dosage form of compound, route of administration, administration interval, but it can be determined by those skilled in the art as needed.

The term "administration" refers to applying one or more substances (such as, PD-L1 antibody and/or small-molecule PD-1/PD-L1 inhibitor) to the subject, it can also be expressed as "application".

The term "pharmaceutical composition" can also be expressed as "pharmaceutical combination", both can be substituted for each other in the present disclosure.

Unless otherwise stated, the term "dosage" refers to the dosage of single administration.

The term "container" refers to any container and cover applicable to the storage, transportation, distribution and/or handling of drugs.

The term "pharmaceutical acceptable" refers to the salt, composition, excipient which are generally non-toxic, safe and suitable to be administered to the subject; the subject is preferably a mammal, more preferably human.

The term "subject" or "patient" refers to any animal to be administered or has been administered with the compound or the pharmaceutical composition according to the embodiment of the present disclosure, preferably a mammal, most preferably human. As used herein, the term "mammal" includes any mammal. The example of mammal includes but not limited to cattle, horse, sheep, pig, cat, dog, mouse, rat, rabbit, Guinea pig, monkey, human and so on, human is the most preferable. The term "subject" and "patient" are interchangeable herein.

The term "pharmaceutically acceptable salt" refers to a pharmaceutically acceptable organic or inorganic salt. Typical example includes but not limited to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, methylsulfonate, ethylsulfonate, benzene sulfonate, tosilate, embonate (i.e. 1-1-methylene-bis(2-hydroxyl-3-naphthoate)). The compound used in the present disclosure can form pharmaceutically acceptable salts with various amino acids. Suitable alkali salt includes but not limited to aluminum, calcium, lithium, magnesium, potassium, sodium, zinc, bismuth and diethanolaminate. A review of pharmaceutically acceptable salts refers to Handbook of Pharmaceutical Salts: Properties, Selection, and Use (P. Heinrich Stahl and Camille G. Wermuth, ed., Wiley-VCH, 2002*).*

The term "halogen" is preferably F, Cl, Br, or, I.

The term "substituted or unsubstituted alkyl" is preferably substituted or unsubstituted C₁-C₄ alkyl. The substituted or unsubstituted C₁-C₄ alkyl is preferably substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, or, substituted or unsubstituted tert-butyl.

The term "substituted or unsubstituted alkoxy" is preferably substituted or unsubstituted C₁-C₄ alkoxy. The substituted or unsubstituted C₁-C₄ alkoxy is preferably substituted or unsubstituted methoxy, substituted or unsubstituted ethoxy, substituted or unsubstituted n-propoxy, substituted or unsubstituted isopropoxy, substituted or unsubstituted n-butoxy, substituted or unsubstituted isobutoxy, or, substituted or unsubstituted tert-butoxy.

In the present disclosure, the term "alkylthio" is preferably -S-R^{s}, wherein, R^{s} is C₁-C₄ alkyl.

In the present disclosure, the term "C₁₋₄ alkyl" is preferably methyl, ethyl, n-propyl, isopropyl, n-butyl, isobutyl or tert-butyl.

In the present disclosure, the term "C₁₋₄ alkoxy" is preferably methoxy, ethoxy, n-propoxy, isopropoxy, n-butoxy, isobutoxy or tert-butoxy.

In the present disclosure, the term "C₁₋₄ carboxyl" is preferably

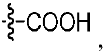

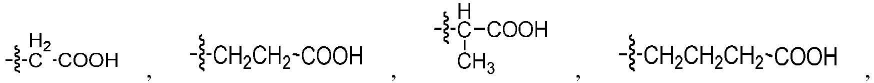

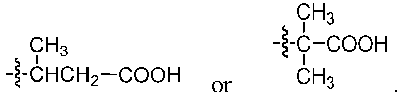

In the present disclosure, the term "C₁₋₄ ester group" is preferably 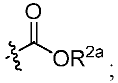 wherein, R^{2a} is C₁-C₄ alkyl.

In the present disclosure, the term "C₁₋₄ amide group" is preferably 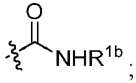 wherein, R^{1b} is hydrogen or C₁-C₄ alkyl.

In the present disclosure, the term "heteroaromatic ring" is preferably C₁-C₁₀ heteroaromatic ring, more preferably acridine ring, carbazole ring, cinnoline ring, carboline ring, quinoxaline ring, imidazole ring, pyrazole ring, pyrrole ring, indole ring, indoline ring, benzotriazole ring, benzimidazole ring, furan ring, thiophen ring, isothiazole ring, benzothiophene ring, dihydrobenzothiophene ring, benzofuran ring, isobenzofuran ring, benzoxazole ring, benzofuraxan ring, benzopyrazole ring, quinoline ring, isoindoline ring, isoquinoline ring, oxazole ring, oxadiazole ring, isoxazole ring, indole ring, pyrazine ring, pyridopyridine ring, tetrazolopyridine ring, imidazopyridine ring, imidazopyrazine ring, pyridazine ring, pyridine ring, naphthopyrimidine ring, pyrimidine ring, tetrazole ring, thiadiazole ring, thiazole ring, thiophene ring, triazole ring, quinazoline ring, tetrahydroquinoline ring, dihydrobenzimidazole ring, dihydrobenzofuran ring, dihydrobenzoxazole ring or dihydroquinoline ring.

In the present disclosure, the term "5- to 7- membered heterocarbocyclic ring" refers to 5- to 7- membered heterocarbocyclic ring wherein the heteroatom(s) is(are) selected from the heteroatoms which is oxygen and/or nitrogen and the number of the heteroatom(s) is 1 to 4. The number of ring atoms in the 5- to 7- membered heterocarbocyclic ring is 5, 6 or 7. In the present disclosure, the 5- to 7- membered heterocarbocyclic ring includes but are not limited to: azetidine ring, piperazine ring, piperidine ring, pyrrole ring, morpholine ring, thiomorpholine ring, 1,4-dioxane, pyran ring, dihydroimidazole ring, dihydroisoxazole ring, dihydroisothiazole ring, dihydrooxadiazole ring, dihydrooxazole ring, dihydropyrazine ring, dihydropyrazole ring, dihydropyridine ring, dihydropyrimidine ring, dihydropyrrole ring, dihydroquinoline ring, dihydrotetrazole ring, dihydrothiadiazole ring, dihydrothiazole ring, dihydrotriazole ring, dihydroazetidine ring, imidazole ring, pyrazole ring, pyrrole ring, furan ring, thiophene ring, isothiazole ring, oxazole ring, oxadiazole ring, isoxazole ring, pyrazine ring, pyridazine ring, pyridine ring, pyrimidine ring, tetrazole ring, thiadiazole ring, thiazole ring, thiophene ring and triazole ring; and the 5- to 7-membered heterocarbocyclic ring is preferably 2,3-dihydro-1,4-dioxane (preferably 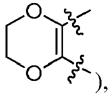 oxazole ring (preferably 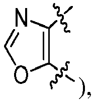 isoxazole ring (preferably 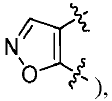 or, pyrazole ring (preferably

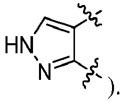

In the present disclosure, the term "pharmaceutically acceptable carrier" refers to any preparation or carrier medium that can deliver active substance, do not interfere with the biological activity of active substance and have no toxic and side effect on the host or patient.

In the present disclosure, the pharmaceutical composition can be formulated into various types of dosage forms such as tablets, pills, powders, liquids, suspensions, emulsions, granules, capsules, suppositories and injections (solutions and suspensions) etc., preferably liquids, suspensions, emulsions, suppositories and injections (solutions and suspensions) etc.

In order to form a pharmaceutical composition in the form of a tablet, any known and widely used excipients in the art can be used. For example, carriers such as lactose, white sugar, sodium chloride, glucose, urea, starch, calcium carbonate, kaolin, crystalline cellulose and silicic acid etc.; adhesives such as water, ethanol, propanol, common syrup, dextrose solution, starch solution, gelatin solution, carboxymethylcellulose, shellac, methylcellulose and potassium phosphate, polyvinylpyrrolidone etc.; disintegrants such as dry starch, sodium alginate, agar powder and kelp powder, sodium bicarbonate, calcium carbonate, polyethylene sorbitan fatty acid ester, sodium dodecyl sulfate, stearic acid monoglyceride, starch and lactose etc.; disintegration inhibitors such as white sugar, glyceryl tristearate, coconut oil and hydrogenated oil; adsorption accelerators such as quaternary ammonium base and sodium dodecyl sulfate etc.; wetting agents such as glycerol, starch etc.; adsorbents such as starch, lactose, kaolin, bentonite and colloidal silicic acid etc.; and lubricants such as pure talc, stearate, boric acid powder and polyethylene glycol etc. It is also possible to use conventional coating materials to prepare sugar-coated tablets, gelatin membrane-coated tablets, enteric-coated tablets, film-coated tablets, bilayer tablets and multilayered tablets.

In order to form the pharmaceutical composition in the form of a pill, any known and widely used excipients in the art can be used, for example, carriers such as lactose, starch, coconut oil, hardened vegetable oil, kaolin and talc etc., adhesives such as gum arabic, gum tragacanth, gelatin and ethanol etc.; disintegrating agents such as agar and kelp powder etc.

In order to form the pharmaceutical composition in the form of a suppository, any of the known and widely used excipients in the art can be used, for example, polyethylene glycol, coconut oil, higher alcohols, esters of higher alcohols, gelatin and semi-synthetic glycerides etc.

In order to prepare the pharmaceutical composition in the form of an injection, the solution or suspension can be sterilized (preferably by adding an appropriate amount of sodium chloride, glucose or glycerol etc.) to form an injection with the isotonic pressure of the blood. Any commonly used carrier in the art can also be used in the preparation of the injection. For example, water, ethanol, propanediol, ethoxylated isostearyl alcohol, polyoxylated isostearyl alcohol and polyethylene sorbitan fatty acid ester. In addition, ordinary solubilizers, buffers and analgesics etc. can be added.

In the pharmaceutical composition, the diluent can be a conventional diluent in the art.

In pharmaceutical composition can be in the form of oral or a sterile injectable aqueous solution, and oral and injectable composition can be prepared according to any method known in the art for preparing a pharmaceutical composition.

The term "prodrug" used herein refers to a derivative of a compound containing biological reactive functional groups, which can be cleaved from the compound or react in other ways to provide the compound under biological condition (in vivo or in vitro). Generally, the prodrug does not have activity, or have less activity than the compound itself, which makes the compound exhibit effects until the biological reactive functional group cleaved from the compound. The biological reactive functional group can hydrolyze or oxidize under biological condition to provide the compound. For example, the prodrug can include biologically hydrolysable groups. The biologically hydrolysable groups include but not limited to biologically hydrolysable phosphate, biologically hydrolysable ester, biologically hydrolysable amide, biologically hydrolysable carbonate, biologically hydrolysable carbamate and biologically hydrolysable ureide.

The compound of the present disclosure can contain one or more asymmetric centers ("stereoisomers"). As used herein, the term "stereoisomer" refers to *Cis-* and *Trans-* isomer, *R*- and *S*- enantiomer and diastereomer. These stereoisomers can be prepared by methods of asymmetric synthesis or chiral separation(e.g. separation, crystallization, thin layer chromatography, column chromatography, gas chromatography, high performance liquid chromatography). These stereoisomers can also be derived from a diastereomer obtained by reacting a mixture of the enantiomers or racemates with a proper chiral compound, followed by crystallizing or any other appropriate conventional method.

The compound of the present disclosure can also contain metabolite thereof. The term "metabolite" refers to the active substance produced after the chemical structure changes of drug molecules in vivo. The active substance is generally the derivative of the drug molecules, which can also be chemically modified.

The compound of the present disclosure can also contain polymorph thereof. The term "polymorph" refers to one or more crystal structures formed by different arrangement of molecules in lattice space during crystallization.

The compound of the present disclosure can also contain solvate thereof. The term "solvate" refers to a crystal form of compound, pharmaceutically acceptable salt, crystal form, eutectic, stereoisomer, isotopic compound, metabolite or prodrug thereof, It also contains one or more solvent molecules incorporated into the crystal structure. Solvate can include stoichiometric or non-stoichiometric solvent, and solvent molecule in solvent may exist in the form of ordered or non-ordered arrangement. Solvate containing non-stoichiometric solvent molecule may be obtained by losing at least part (but not all) of solvent molecule. In a particular embodiment, a solvate is a hydrate, meaning that the crystalline form of a compound further includes water molecule.

The compound of the present disclosure can also contain prodrug thereof. The term "prodrug" used herein refers to a derivative of a compound containing biological reactive functional groups, which can be cleaved from the compound or react in other ways to provide the compound under biological condition (in vivo or in vitro). Generally, the prodrug does not have activity, or have less activity than the compound itself, which makes the compound exhibit effects until the biological reactive functional group cleaved from the compound. The biological reactive functional group can hydrolyze or oxidize under biological condition to provide the compound. For example, the prodrug can include biologically hydrolysable groups. The biologically hydrolysable groups include but not limited to biologically hydrolysable phosphate, biologically hydrolysable ester, biologically hydrolysable amide, biologically hydrolysable carbonate, biologically hydrolysable carbamate and biologically hydrolysable ureide. An overview of prodrug refers to, for example, J. Rautio et al., Nature Reviews Drug Discovery (2008) 7, 255-270 *and* Prodrugs: Challenges and Rewards (V. Stella et al. ed., Springer, 2007***).***

The compound of the present disclosure can also contain isotopic derivatives thereof. The term "isotopic derivatives" used herein refers to the compound containing one or more natural or non-natural isotopes. Non-natural isotopes include but are not limited to deuterium (²H or D), tritium (³H or T), iodine-125 (¹²⁵I), phosphorus-32 (³²P), carbon-13 (¹³C) or carbon-14 (¹⁴C). All isotopic variants of the compound, whether radioactive or not, are included in the scope of the present disclosure.

Unless otherwise stated, the term "a/an", "one", or, "a kind" used herein also include plural form.

The code of compound used herein, such as, KN035, CS1001, MBS2311, BGB-A333, KL-A167, SHR-1316, STI-A1014, etc., is known in the art.

All publications, patent applications, patents and other references mentioned herein are incorporated herein by reference in their entirety.

Without departing from the common knowledege in the art, the optimum examples can be obtained by optionally combining the preferred conditions above.

The reagent and raw material used herein are commercially available.

The progressive effect of the present disclosure is: providing a small-molecule PD-1/PD-L1 inhibitor, or a pharmaceutical composition with PD-L1 antibody thereof, which can be used to treat cancer.

### Brief description of the drawings

Fig. 1 is a curve graphically depicting the weight change of mice in each group in effect example 3.
Fig.2 is a curve graphically depicting the weight change rate of mice in each group in effect example 3.
Fig.3 is a curve graphically depicting the tumor volume change of mice in each group in effect example 3.
Fig.4 is a curve graphically depicting the tumor inhibition rate change of mice in each group in effect example 3.

### Detailed description of the preferred embodiment

The following examples futher illustrate the present disclosure, but the present disclosure is not limited thereto. The experimental method without particular conditions being specified in the following examples is selected according to conventional methods and conditions, or product instructions.

In the following examples, room temperature refers to 10 °C to 30 °C; reflux refers to the solvent reflux temperature; overnight refers to 8 to 24 hours, preferably 12 to 18 hours.

Thestructure of the compounds were confirmed by NMR or MS. NMR was determined by Bruker Avance-500 apparatus using *d₆*-DMSO, CDCl₃ and CD₃OD etc. as a solvent, and TMS as an interior label. MS was determined by LC-MS Agilent Technologies 6110 using ESI as an ion source.

Microwave reaction was conducted in Explorer full automatic microwave irradiation equipment supplied by CEM, US Corporation. The magnetron frequency was 2450 MHz, and the continuous microwave output power was 300 W.

The instrument used for preparative high performance liquid chromatography was Gilson 281, and the preparative column was Shimadazu Shim-Pack, PRC-ODS, 20×250 mm, 15 µm.

### Example 1

(E)-2-(3-(3-(2,3-dihydrobenzo[b][1,4]dioxin-6-yl)-2-methylstyryl)-4-(trifluoromethyl)benzylamino)-3-hydroxypropanoic acid (compound 1)

Synthesis route:

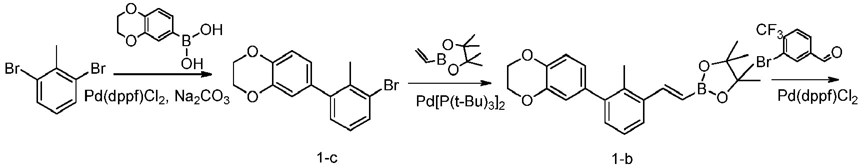

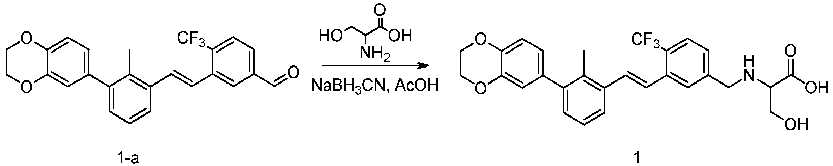

### Synthesis of compound 1-c

1,4-Benzodioxane-6-boronic acid (3.60 g, 20 mmol) and 2,6-dibromotoluene (7.50 g, 30 mmol) were dissolved in a mixed solution of 1,4-dioxane (100 mL) and water (15 mL), and [1,1'-bis(diphenylphosphino)ferrocene]palladium dichloride dichloromethane complex (817 mg, 1 mmol) and sodium carbonate (6.38 g, 60 mmol) were added. After the reaction system was replaced with nitrogen three times, the reaction solution was heated to 80 °C and stirred for 16 hours. The reaction solution was cooled to room temperature, concentrated under reduced pressure, and the residue was purified by silica gel column chromatography (petroleum ether) to obtain compound **1-c** (2.70 g, yield: 44%).

### Synthesis of compound 1-b

Compound **1-c** (915 mg, 3 mmol) and pinacol vinylboronate (924 mg, 6 mmol) were dissolved in toluene solution (10 mL), and bis(tri-*tert*-butylphosphine)palladium (120 mg, 0.24 mmol) and triethylamine (2.0 g, 20 mmol) were added. After the reaction system was replaced with nitrogen three times, the reaction solution was heated to 80 °C and stirred for 6 hours. The reaction solution was cooled to room temperature, concentrated under reduced pressure, and the residue was purified by silica gel column chromatography (petroleum ether: ethyl acetate = 10: 1) to obtain compound **1-b** (540 mg, yield: 48%).

### Synthesis of compound 1-a

Compound **1-b** (475 mg, 1.26 mmol) and 3-bromo-4-trifluoromethylbenzaldehyde (265.7 mg, 1.05 mmol) were dissolved in a mixed solution of 1,4-dioxane (20 mL) and water (1 mL), [1,1'-bis(diphenylphosphino)ferrocene]palladium dichloride (90.8 mg, 0.105 mmol) and sodium carbonate (277.8 mg, 2.62 mmol) were added. After the reaction system was replaced with nitrogen three times, the reaction solution was heated to 80 °C and stirred for 16 hours. The reaction solution was cooled to room temperature, concentrated under reduced pressure, and the residue was dissolved with ethyl acetate (50 mL), followed by washing with water (20 mL × 3) and saturated brine (20 mL) sequentially, and the organic phase was dried over anhydrous sodium sulfate, concentrated under reduced pressure, and the residue was purified by silica gel column chromatography (petroleum ether: ethyl acetate = 10: 1) to obtain compound **1-a** (366 mg, yield: 80.4%). [0273] ¹H NMR (500 MHz, CDCl₃) *δ*: 10.15 (s, 1H), 8.27 (s, 1H), 7.86 (s, 2H), 7.57-7.55 (d, *J* = 7.5 Hz, 1H), 7.52-7.49 (d, *J* = 16.0 Hz, 1H), 7.37-7.34 (m, 1H), 7.29-7.27 (m, 1H), 7.22-7.21 (m, 1H), 6.93-6.91 (d, *J* = 8.5 Hz, 1H), 6.84-6.83 (m, 1H), 6.79-6.77 (m, 1H), 4.31 (s, 4H), 2.35 (s, 3H) ppm.

### Synthesis of compound 1

Compound **1-a** (200 mg, 0.40 mmol) and serine (99 mg, 0.94 mmol) were dissolved in a mixed solution of methanol (15 mL) and dichloromethane (15 mL), and acetic acid (0.05 mL, 0.94 mmol) was added. After the reaction solution was stirred at room temperature for 2 hours, to the reaction solution was added sodium cyanoborohydride (119 mg, 1.89 mmol) and continued to be stirred for 12 hours. The reaction solution was concentrated under reduced pressure, and the residue was dissolved with ethyl acetate (50 mL), followed by washing with water (20 mL) and saturated brine (20 mL) sequentially, and the organic phase was dried over anhydrous sodium sulfate, and concentrated under reduced pressure, and the residue was purified by high performance liquid chromatography (mobile phase: water (10 mM ammonium bicarbonate), acetonitrile; gradient: 15% to 65% (the initial mobile phase was 15% water and 85% acetonitrile, the final mobile phase was 65% water and 35% acetonitrile, wherein, % refers to volume percentage) to obtain compound **1** (12 mg, yield: 5.8%). LC-MS (ESI): m/z = 514 [M+H]⁺.

¹H NMR (400 MHz, DMSO) *δ*: 8.05 (s, 1H), 7.74-7.73 (d, *J* = 6.4 Hz, 1H), 7.59-7.51 (m, 3H), 7.30-7.15 (m, 3H), 6.93-6.91 (d, *J* = 6.8 Hz, 1H), 6.80-6.75 (m, 2H), 4.28 (s, 4H), 4.09-4.06 (d, *J* = 11.2 Hz, 1H), 3.96-3.93 (d, *J* = 11.2Hz, 1H), 3.69-3.62 (m, 2H),3.20-3.18 (t, *J* = 4.0 Hz, 1H), 2.29 (s,3H) ppm.

### Example 2

(*E*)-2-((3-(2-(4'-methoxy-2-methylbiphenyl-3-yl)vinyl)-4-methylbenzyl)amino)-3-hydroxy-2-methylpropanoic acid (compound 2)

Synthesis route:

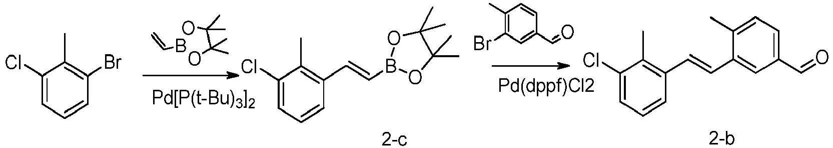

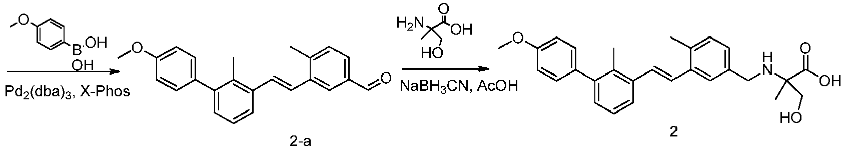

### Synthesis of compound 2-c

To a solution of 2-bromo-6-chlorotoluene (15.67 g, 76.26 mmol) and pinacol vinylboronate (14.30 g, 91.51 mmol) in toluene (300 mL) were added bis(tri-tert-butylphosphine)palladium (2.73 g, 5.34 mmol) and triethylamine (61.74 g, 610.08 mmol) at room temperature, and the reaction solution was heated to 80 °C and stirred overnight under nitrogen. After the completion of the reaction, the reaction solution was diluted with ethyl acetate (100 mL), washed with water (100 mL) and saturated brine (100 mL). The obtained organic phase was dried over anhydrous sodium sulfate, concentrated under reduced pressure, and the residue was purified by silica gel column chromatography (petroleum ether: ethyl acetate = 10: 1) to obtain compound 2-c (10.5 g, yield: 49.4%). [0282] ¹H NMR (500 MHz, CDCl₃) *δ*: 7.65-7.62 (d, *J* = 18.5 Hz, 1H), 7.42-7.41 (d, *J* = 7.5 Hz, 1H), 7.31-7.30 (d, *J* = 7.5 Hz, 1H), 7.12-7.09 (t, 1H), 6.06-6.02 (d, *J* = 18.0 Hz, 1H), 2.45 (s, 3H), 1.32 (s, 12H) ppm.

### Synthesis of compound 2-b

To a solution of 3-bromo-4-methylbenzaldehyde (5.0 g, 17.95 mmol) and **2-c** (2.98 g, 14.96 mmol) in 1,4-dioxane (40 mL) and water (2 mL) were added [1,1'-bis(diphenylphosphino)ferrocene]palladium dichloride (1.294 g, 1.496 mmol) and sodium carbonate (3.963 g, 37.39 mmol) at room temperature, and the reaction solution was heated to 80 °C and stirred overnight under nitrogen. After the completion of the reaction, the reaction solution was diluted with ethyl acetate (50 mL), washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **2-b** (3.31 g, yield: 81.9%).

¹H NMR (500 MHz, CDCl₃) *δ*: 10.03 (s, 1H), 8.08 (s, 1H), 7.72-7.70 (d, *J* = 8.0 Hz, 1H), 7.47-7.45 (d, *J* = 7.5 Hz, 1H), 7.37-7.31 (m, 3H), 7.18-7.12 (m, 2H), 2.50 (s, 6H) ppm.

### Synthesis of compound 2-a

To a solution of *p*-methoxyphenylboronic acid (202 mg, 1.329 mmol) and **2-b** (300 mg, 1.108 mmol) in toluene (20 mL) were added tris(dibenzylideneacetone)dipalladium (101.6 mg, 0.111 mmol), 2-dicyclohexylphosphino-2',4',6'-triisopropylbiphenyl (211.7 mg, 0.444 mmol) and potassium phosphate (705.6 mg, 3.324 mmol), and the reaction solution was heated to 90 °C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **2-a** (334 mg, yield: 87.9%).

¹H NMR (500 MHz, CDCl₃) *δ*: 10.03 (s, 1H), 8.10 (s, 1H), 7.71-7.69 (d, *J* = 7.5 Hz,1H), 7.58-7.56 (d, *J* = 7.5 Hz,1H), 7.42-7.36 (m, 4H), 7.21-7.17 (m, 2H), 6.98-6.96 (d, *J* = 8.5 Hz, 2H), 3.87 (s, 3H), 2.51 (s, 3H), 2.33 (s, 3H) ppm.

### Synthesis of compound 2

To a mixed solution of **2-a** (330 mg, 0.964 mmol) and 2-methylserine (229.9 mg, 1.93 mmol) in methanol (10 mL) and dichloromethane (10 mL) was added acetic acid (115.9 mg, 1.93 mmol) at room temperature, and the reaction solution was stirred at room temperature for 1 hour. Then, to the reaction solution was added sodium cyanoborohydride (302.9 mg, 4.82 mmol) and was stirred for 16 hours. After the completion of the reaction, the organic solvent was concentrated by rotary evaporation to dryness, and the residue was dissolved in ethyl acetate (50 mL), followed by washing with water (50 mL) and saturated brine (50 mL) sequentially. The obtained organic phase was dried over anhydrous sodium sulfate, concentrated under reduced pressure, and the residue was purified by silica gel thin-layer chromatography preparative plate (dichloromethane: methanol = 10: 1) to obtain compound 2 (85 mg, yield: 19.8%). LC-MS (ESI): m/z = 444.0 [M-H]⁺.

¹H NMR (500 MHz, DMSO-d6) *δ*: 7.79 (s, 1H), 7.67-7.65 (d, *J* = 7.5 Hz, 1H), 7.40-7.37 (d, *J* = 16.5 Hz, 1H), 7.30-7.23 (m, 6H), 7.14-7.13 (d, *J* = 7.5 Hz, 1H), 7.03-7.01 (d, *J* = 9.0 Hz, 2H), 4.01-3.94 (m, 2H), 3.81 (s, 3H), 3.68-3.66 (d, *J* = 11.5 Hz, 1H), 3.60-3.58 (d, *J* = 11.0 Hz, 1H), 2.41 (s, 3H), 2.29 (s, 3H), 1.31 (s, 3H) ppm.

### Example 3

(*E*)-2-(3-(2-(3'-fluoro-2-methylbiphenyl-3-yl)vinyl)-4-(trifluoromethyl)benzylamino)-3-hydroxy-2-methylpropanoic acid (compound 3)

Synthesis route:

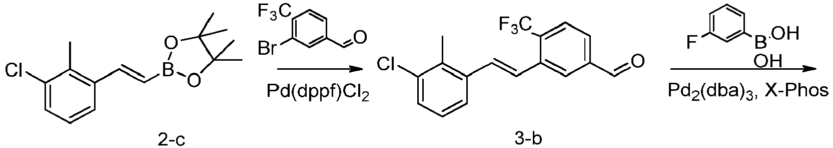

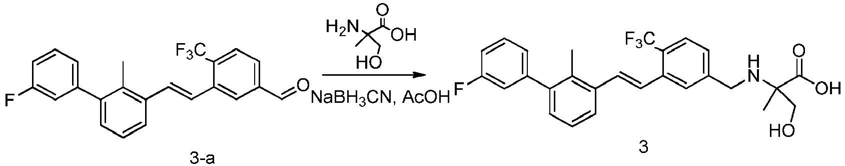

### Synthesis of compound 3-b

To a mixed solution of 3-bromo-4-trifluoromethylbenzaldehyde (4.16 g, 16.45 mmol) and compound 2-c (5.5 g, 19.74 mmol) in 1,4-dioxane (40 mL) and water (2 mL) were added [1,1'-bis(diphenylphosphino)ferrocene]palladium dichloride (1.423 g, 1.645 mmol) and sodium carbonate (4.36 g, 41.13 mmol) at room temperature, and the reaction solution was heated to 80 °C and stirred for 16 hours under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **3-b** (4.16 g, yield: 78%).

¹H NMR (500 MHz, CDCl₃) *δ*: 10.15 (s, 1H), 8.25 (s, 1H), 7.87 (s, 2H), 7.47-7.40 (m, 2H), 7.37-7.36 (d, *J* = 7.0 Hz, 1H), 7.32-7.29 (m, 1H), 7.20-7.17 (m, 1H), 2.50 (s, 3H) ppm.

### Synthesis of compound 3-a

To a solution of compound **3-b** (300 mg, 0.93 mmol) in toluene (20 mL) were added 3-fluorophenylboronic acid (156 mg, 1.11 mmol), potassium phosphate (590 mg, 2.78 mmol), 2-dicyclohexylphosphino-2',4',6'-triisopropylbiphenyl (66 mg, 0.132 mmol) and tris(dibenzylideneacetone)dipalladium (30 mg, 0.03 mmol). The reaction system was replaced with nitrogen three times, and the reaction solution was heated to 90 °C and stirred for 12 hours. The reaction solution was cooled to room temperature, concentrated under reduced pressure, and the residue was purified by silica gel column chromatography (petroleum ether: ethyl acetate = 100: 1) to obtain 3-a (240 mg, yield: 68%) as a yellow solid. LC-MS (ESI): m/z = 385 [M+H]⁺.

### Synthesis of compound 3

Compound **3-a** (240 mg, 0.625 mmol) and 2-methylserine (150 mg, 1.25 mmol) were dissolved in a mixed solution of methanol (15 mL) and dichloromethane (15 mL), and acetic acid (0.07 mL, 1.25 mmol) was added. After the reaction solution was stirred at room temperature for 2 hours, to the reaction solution was added sodium cyanoborohydride (157 mg, 2.5 mmol) and continued to be stirred for 12 hours. The reaction solution was concentrated under reduced pressure, and the residue was dissolved with ethyl acetate (50 mL), followed by washing with water (20 mL) and saturated brine (20 mL) sequentially, and the organic phase was dried over anhydrous sodium sulfate and concentrated under reduced pressure, and the residue was purified by high performance liquid chromatography (mobile phase: water (10 mM ammonium bicarbonate), acetonitrile; gradient: 15% to 65% (the initial mobile phase was 15% water and 85% acetonitrile, and the final mobile phase was 65% water and 35% acetonitrile, wherein % refers to volume percentage)) to obtain compound 3 (75 mg, yield: 24.5%). LC-MS (ESI): m/z = 488 [M+H]⁺.

¹H NMR (400 MHz, DMSO-d6) *δ*: 8.06 (s, 1H),7.76-7.74 (d, *J* = 6.4 Hz, 1H), 7.61-7.56 (m, 3H), 7.53-7.48 (m, 1H), 7.36-7.32 (t, *J* = 12.4 Hz, 1H), 7.07-7.17 (m, 5H), 3.99 (s, 2H), 3.64-3.61 (d, *J =* 8.8 Hz, 1H), 3.57-3.55 (d, *J* = 8.8 Hz, 1H), 2.29 (s, 3H), 1.27 (s, 3H) ppm.

### Example 4

(E)-2-(3-(2-(4'-methylthio-2-methylbiphenyl-3-yl)vinyl)-4-methylbenzylamino)-3-hydroxy-2-methylpropanoic acid (compound **4)**

Synthesis route:

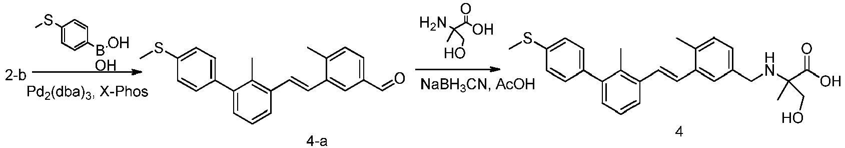

### Synthesis of compound 4-a

To a solution of *p*-methylthiophenylboronic acid (223.3 mg, 1.329 mmol) and **2-b** (300 mg, 1.108 mmol) in toluene (20 mL) were added tris(dibenzylideneacetone)dipalladium (101.6 mg, 0.111 mmol), 2-dicyclohexylphosphino-2',4',6'-triisopropylbiphenyl (211.7 mg, 0.444 mmol) and potassium phosphate (705.6 mg, 3.324 mmol) at room temperature, and the reaction solution was heated to 90°C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **4-a** (323 mg, yield: 81.2%).

¹H NMR (500 MHz, CDCl₃) *δ*: 10.03 (s, 1H), 8.10 (s, 1H), 7.71-7.69 (d, *J* = 7.0 Hz, 1H), 7.63-7.62 (m, 1H), 7.59-7.58 (d, *J* = 7.5 Hz, 2H), 7.43-7.36 (m, 4H), 7.33-7.32 (m, 2H), 7.21-7.18 (m, 2H), 2.54 (,s 3H), 2.51 (s, 3H), 2.33 (s, 3H) ppm.

### Synthesis of compound 4

To a mixed solution of **4-a** (323 mg, 0.901 mmol) and 2-methylserine (214.7 mg, 1.802 mmol) in methanol (10 mL) and dichloromethane (10 mL) was added acetic acid (108.2 mg, 1.802 mmol) at room temperature, and the reaction solution was stirred at room temperature for 1 hour. Then, to the reaction solution was added sodium cyanoborohydride (254.5 mg, 4.505 mmol) and was stirred for 16 hours. After the completion of the reaction, the organic solvent was concentrated by rotary evaporation to dryness. And the residue was dissolved in ethyl acetate (20 mL), followed by washing with water (20 mL) and saturated brine (20 mL) sequentially. The obtained organic phase was dried over anhydrous sodium sulfate, concentrated under reduced pressure, and the residue was purified by silica gel thin layer chromatography preparative plate (dichloromethane: methanol = 10: 1) to obtain compound **4** (112 mg, yield: 26.9%). LC-MS (ESI): m/z = 460.0 [M-H]⁺.
¹H NMR (500 MHz, CD₃OD) *δ*: 7.78 (s, 1H), 7.69-7.68 (d, *J* = 7.5 Hz, 1H), 7.39-7.33 (m, 3H), 7.31-7.26 (m, 5H), 7.24-7.22 (d, *J* = 8.0 Hz, 1H), 7.15-7.13 (d, *J* = 7.0 Hz, 1H), 4.00-3.92 (m, 2H), 3.67-3.65 (d, *J* = 11.5 Hz, 1H), 3.59-3.57 (d, *J* = 10.5 Hz, 1H), 2.52 (s, 3H), 2.41 (s, 3H), 2.29 (s, 3H), 1.30 (s, 3H) ppm

### Example 5

(*E*)-2-(3-(2-(2-methylbiphenyl-3-yl)vinyl)-4-(trifluoromethyl)benzylamino)-3-hydroxy-2-methylpropanoic acid (compound **5**)

Synthesis route:

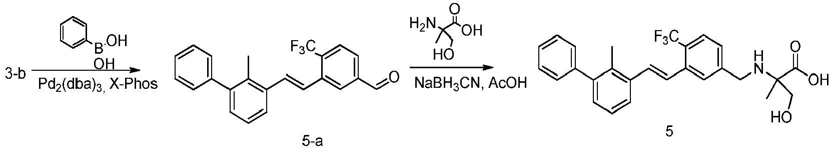

### Synthesis of compound 5-a

To a solution of phenylboronic acid (135.3 mg, 1.11 mmol) and compound **3-b** (300 mg, 0.924 mmol) in toluene (20 mL) were added tris(dibenzylideneacetone)dipalladium (84.2 mg, 0.092 mmol), 2-dicyclohexylphosphino-2',4',6'-triisopropylbiphenyl (175.4 mg, 0.368 mmol) and potassium phosphate (588.4 mg, 2.772 mmol) at room temperature, and the reaction solution was heated to 90 °C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate and concentrated under reduced pressure, and the residue was purified by silica gel column chromatography (petroleum ether: ethyl acetate = 40: 1) to obtain compound **5-a** (254 mg, yield: 74.9%).

¹H NMR (500 MHz, CDCl₃) *δ:* 10.15 (s, 1H), 8.28 (s, 1H), 7.87 (s, 2H), 7.60-7.59 (d, *J* = 7.5 Hz, 1H), 7.53-7.50 (d, *J* = 16.0 Hz, 1H), 7.45-7.42 (m, 2H), 7.39-7.35 (m, 2H), 7.33-7.29 (m, 3H), 7.25-7.23 (m, 1H), 2.33 (s, 3H) ppm.

### Synthesis of compound 5

To a mixed solution of **5-a** (254 mg, 0.693 mmol) and 2-methylserine (165.1 mg, 1.386 mmol) in methanol (10 mL) and dichloromethane (10 mL) was added acetic acid (83.2 mg, 1.386 mmol) at room temperature, and the reaction solution was stirred at room temperature for 1 hour. Then, to the reaction solution was added sodium cyanoborohydride (254.5 mg, 4.505 mmol) and was stirred for 16 hours. After the completion of the reaction, the organic solvent was concentrated by rotary evaporation to dryness, and the residue was dissolved in ethyl acetate (20 mL), followed by washing with water (20 mL) and saturated brine (20 mL) sequentially. The obtained organic phase was dried over anhydrous sodium sulfate and concentrated under reduced pressure, and the residue was purified by silica gel thin layer chromatography preparative plate (dichloromethane: methanol = 10: 1) to obtain compound **5** (95 mg, yield: 29.2%). LC-MS (ESI): m/z = 468.0 [M-H]+.
¹H NMR (500 MHz, CD₃OD) *δ*: 8.17 (s, 1H), 7.80-7.79 (d, *J* = 8.0 Hz, 1H), 7.68-7.62 (m, 2H), 7.57-7.56 (d, *J* = 7.5 Hz,1H), 7.46-7.43 (m, 2H), 7.39-7.29 (m, 5H), 7.21-7.19 (d, *J* = 7.5 Hz, 1H), 4.35-4.27 (m, 2H), 4.02-4.00 (d, *J* = 12.0 Hz, 1H), 3.86-3.83 (d, *J* = 12.0 Hz, 1H), 2.34 (s, 3H), 1.56 (s, 3H) ppm.

### Example 6

(*E*)-2-(3-(2-(4'-fluoro-2-methylbiphenyl-3-yl)vinyl)-4-(trifluoromethyl)benzylamino)-3-hydroxy-2-methylpropanoic acid (compound **6**)

Synthesis route:

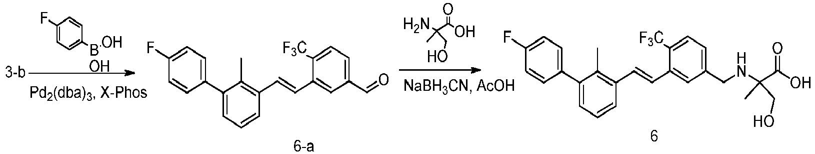

### Synthesis of compound 6-a

To a solution of compound **3-b** (300 mg, 0.93 mmol) in toluene (20 mL) were added 3-fluorophenylboronic acid (156 mg, 1.11 mmol), potassium phosphate (590 mg, 2.78 mmol), 2-dicyclohexylphosphino-2',4',6'-triisopropylbiphenyl (60 mg, 0.132 mmol) and tris(dibenzylideneacetone)dipalladium (30 mg, 0.03 mmol). The reaction system was replaced with nitrogen three times, and the reaction solution was heated to 90 °C and stirred for 12 hours. The reaction solution was cooled to room temperature, concentrated under reduced pressure, and the residue was purified by silica gel column chromatography (petroleum ether: ethyl acetate = 100: 1) to obtain **6-a** (240 mg, yield: 68%) as a yellow solid. LC-MS (ESI): m/z = 385 [M+H]⁺.

### Synthesis of compound 6

Compound **6-a** (240 mg, 0.625 mmol) and 2-methylserine (150 mg, 1.25 mmol) were dissolved in a mixed solution of methanol (15 mL) and dichloromethane (15 mL), and acetic acid (0.07 mL, 1.25 mmol) was added. After the reaction solution was stirred at room temperature for 2 hours, to the reaction solution was added sodium cyanoborohydride (157 mg, 2.5 mmol) and continued to be stirred for 12 hours. The reaction solution was concentrated under reduced pressure, and the residue was dissolved with ethyl acetate (50 mL), followed by washing with water (20 mL) and saturated brine (20 mL) sequentially. The organic phase was dried over anhydrous sodium sulfate, and concentrated under reduced pressure. The residue was purified by high performance liquid chromatography (mobile phase: water (10 mM ammonium bicarbonate), acetonitrile; gradient: 15% to 65% (the initial mobile phase was 15% water and 85% acetonitrile, and the final mobile phase was 65% water and 35% acetonitrile, wherein % refers to volume percentage)) to obtain compound 6 (65 mg, yield: 21.3%). LC-MS (ESI): m/z = 488 [M+H]⁺.

¹H NMR (400 MHz, DMSO-d6) *δ*: 8.06 (s, 1H), 7.76-7.74 (d, *J* = 6.8 Hz, 1H), 7.59-7.56 (m, 3H), 7.39-7.23 (m, 6H), 7.20-7.19 (m, 1H), 3.99 (s, 2H), 3.63-3.61 (d, *J* = 8.8 Hz, 1H), 3.56-3.54 (d, *J* = 8.8 Hz, 1H), 2.28 (s, 3H), 1.27 (s,3H) ppm.

### Example 7

(E)-2-(3-(2-(4'-fluoro-2-methylbiphenyl-3-yl)vinyl)-4-methylbenzylamino)-3-hydroxy-2-methylpropanoic acid (compound **7**)

Synthesis route:

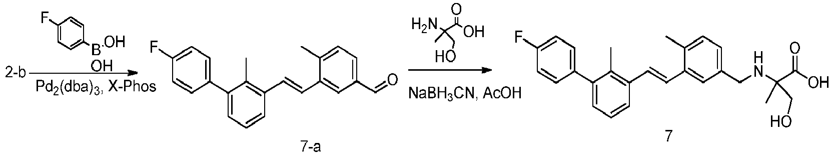

### Synthesis of compound 7-a

To a solution of compound **2-b** (300 mg, 1.11 mmol) in toluene (20 mL) were added p-fluorophenylboronic acid (187 mg, 1.33 mmol), potassium phosphate (708 mg, 3.33 mmol), 2-dicyclohexylphosphino-2',4',6'-triisopropylbiphenyl (60 mg, 0.132 mmol) and tris(dibenzylideneacetone)dipalladium (30 mg, 0.03 mmol). The reaction system was replaced with nitrogen three times, and the reaction solution was heated to 90 °C and stirred for 12 hours. The reaction solution was cooled to room temperature, concentrated under reduced pressure, and the residue was purified by silica gel column chromatography (petroleum ether: ethyl acetate = 100: 1) to obtain **7-a** (340 mg, yield: 92%) as a yellow solid. LC-MS (ESI): m/z = 331 [M+H]⁺.

### Synthesis of compound 7

Compound **7-a** (340 mg, 1.15 mmol) and 2-methylserine (274 mg, 2.3 mmol) were dissolved in a mixed solution of methanol (15 mL) and dichloromethane (15 mL), and acetic acid (0.07 mL, 1.25 mmol) was added. After the reaction solution was stirred at room temperature for 2 hours, to the reaction solution was added sodium cyanoborohydride (290 mg, 4.6 mmol) and continued to be stirred for 12 hours. The reaction solution was concentrated under reduced pressure, and the residue was dissolved with ethyl acetate (50 mL), followed by washing with water (20 mL) and saturated brine (20 mL) sequentially. The organic phase was dried over anhydrous sodium sulfate and concentrated under reduced pressure. The residue was purified by high performance liquid chromatography (mobile phase: water (10 mM ammonium bicarbonate), acetonitrile; gradient: 15% to 65% (the initial mobile phase was 15% water and 85% acetonitrile, and the final mobile phase was 65% water and 35% acetonitrile, wherein % refers to volume percentage)) to obtain compound **7** (35 mg, yield: 7%). LC-MS (ESI): m/z = 434 [M+H]⁺.

¹H NMR (400 MHz, DMSO-d6) *δ*: 7.77 (s, 1H), 7.70-7.68 (d, *J* = 6 Hz, 1H), 7.39-7.35 (m, 3H), 7.31-7.23 (m, 6H), 7.15-7.14 (m, 1H), 3.99-3.92 (m, 2H), 3.67-3.64 (d, *J* = 9.2 Hz, 1H), 3.58-3.56 (d, *J* = 9.2 Hz, 1H), 2.41(s, 3H), 2.26 (s, 3H), 1.29 (s, 3H) ppm.

### Example 8

(*E*)-2-(3-(2-(2-methylbiphenyl-3-yl)vinyl)-4-methylbenzylamino)-3-hydroxy-2-methylpropanoic acid (compound **8**)

Synthesis route:

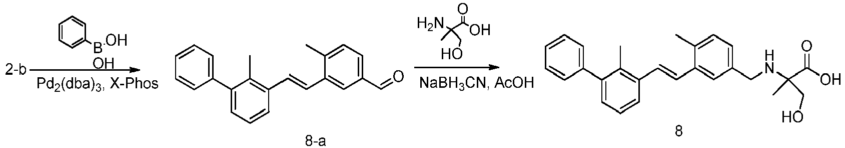

### Synthesis of compound 8-a

To a solution of compound **2-b** (300 mg, 1.108 mmol) in toluene (20 mL) were added phenylboronic acid (162.2 mg, 1.33 mmol), potassium phosphate (705.6 mg, 3.324 mmol), 2-dicyclohexylphosphino-2',4',6'-triisopropylbiphenyl (211.7 mg, 0.444 mmol) and tris(dibenzylideneacetone)dipalladium (101.6 mg, 0.111 mmol). The reaction system was replaced with nitrogen three times, and the reaction solution was heated to 90 °C and stirred for 12 hours. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **8-a** (323 mg, yield: 93.1%).

¹H NMR (500 MHz, CDCl₃) *δ*: 10.03 (s, 1H), 8.10 (s, 1H), 7.71-7.69 (d, *J* = 8.0 Hz, 1H), 7.60-7.59 (d, *J* = 7.0 Hz, 1H), 7.45-7.40 (m, 3H), 7.38-7.36 (m, 2H), 7.33-7.29 (m, 3H), 7.23-7.18 (m, 2H), 2.52 (s, 3H), 2.32 (s, 3H) ppm.

### Synthesis of compound 8

To a mixed solution of **8-a** (323 mg, 1.034 mmol) and 2-methylserine (246.3 mg, 2.068 mmol) in methanol (10 mL) and dichloromethane (10 mL) was added acetic acid (124.2 mg, 2.068 mmol) at room temperature, and the reaction solution was stirred at room temperature for 1 hour. Then, to the reaction solution was added sodium cyanoborohydride (324.9 mg, 5.17 mmol) and stirred for 16 hours. After the completion of the reaction, the organic solvent was concentrated by rotary evaporation to dryness, and the residue was dissolved with ethyl acetate (20 mL), followed by washing with water (20 mL) and saturated brine (20 mL) sequentially. The obtained organic phase was dried over anhydrous sodium sulfate, concentrated under reduced pressure, and the residue was purified by silica gel thin-layer chromatography preparative plate (dichloromethane: methanol = 10: 1) to obtain compound **8** (95 mg, yield: 22.1%). LC-MS (ESI): m/z = 414.0 [M-H]+.

¹H NMR (500 MHz, CD₃OD) *δ*: 7.87 (s, 1H), 7.64-7.63 (d, *J* = 7.5 Hz, 1H), 7.52-7.48 (d, *J* = 16.0 Hz, 1H), 7.46-7.43 (m, 2H), 7.39-7.36 (m, 2H), 7.32-7.26 (m, 5H), 7.16-7.15 (d, *J* = 6.5 Hz, 1H), 4.23-4.15 (m, 2H), 4.00-3.98 (d, *J* = 12.0 Hz, 1H), 3.84-3.81 (d, *J* = 12.0 Hz, 1H), 3.37 (s, 3H), 2.48 (s, 3H), 2.32 (s, 3H), 1.54 (s, 3H) ppm.

### Example 9

(E)-2-(3-(2-(3'-methyl-2-methylbiphenyl-3-yl)vinyl)-4-methylbenzylamino)-3-hydroxy-2-methylpropanoic acid (compound **9**)

Synthesis route:

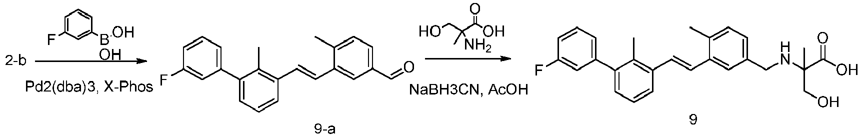

### Synthesis of compound 9-a

To a solution of compound **2-b** (300 mg, 1.11 mmol) in toluene (20 mL) were added 3-fluorophenylboronic acid (187 mg, 1.33 mmol), potassium phosphate (708 mg, 3.33 mmol), 2-dicyclohexylphosphino-2',4',6'-triisopropylbiphenyl (60 mg, 0.132 mmol) and tris(dibenzylideneacetone)dipalladium (30 mg, 0.03 mmol). The reaction system was replaced with nitrogen three times, and the reaction solution was heated to 90 °C and stirred for 12 hours. The reaction solution was cooled to room temperature, concentrated under reduced pressure, and the residue was purified by silica gel column chromatography (petroleum ether: ethyl acetate = 100: 1) to obtain **9-a** (330 mg, yield: 90%) as a yellow solid. LC-MS (ESI): m/z = 331 [M+H]⁺.

### Synthesis of compound 9

Compound **9-a** (330 mg, 1.0 mmol) and 2-methylserine (238 mg, 2.0 mmol) were dissolved in a mixed solution of methanol (15 mL) and dichloromethane (15 mL), and acetic acid (0.1 mL, 2.3 mmol) was added. After the reaction solution was stirred at room temperature for 2 hours, to the reaction solution was added sodium cyanoborohydride (251 mg, 4.0 mmol) and continued to be stirred for 12 hours. The reaction solution was concentrated under reduced pressure, and the residue was dissolved with ethyl acetate (50 mL), followed by washing with water (20 mL) and saturated brine (20 mL) sequentially. The organic phase was dried over anhydrous sodium sulfate and concentrated under reduced pressure. The residue was purified by high performance liquid chromatography (mobile phase: water (10 mM ammonium bicarbonate), acetonitrile; gradient: 15% to 65% (the initial mobile phase was 15% water and 85% acetonitrile, and the final mobile phase was 65% water and 35% acetonitrile, wherein % refers to volume percentage)) to obtain compound **9** (80 mg, yield: 18.4%). LC-MS (ESI): m/z = 434 [M+H]⁺.

¹1H NMR (400 MHz, DMSO-d6) *δ*: 7.78(s, 1H), 7.72-7.71 (d, *J* = 6 Hz, 1H), 7.52-7.48 (m, 1H), 7.39-7.36 (m, 1H), 7.32-7.26 (m, 3H), 7.22-7.16 (m, 5H), 3.99-3.92 (m, 2H), 3.67-3.65 (d, *J* = 9.2 Hz, 1H), 3.59-3.57 (d, *J* = 9.2 Hz, 1H), 2.41 (s, 3H), 2.28 (s, 3H), 1.29 (s,3H) ppm.

### Example 10

(*E*)-2-(3-(2-(4'-chloro-2-methylbiphenyl-3-yl)vinyl)-4-(trifluoromethyl)benzylamino)-3-hydroxy-2-methylpropanoic acid (compound **10**)

Synthesis route:

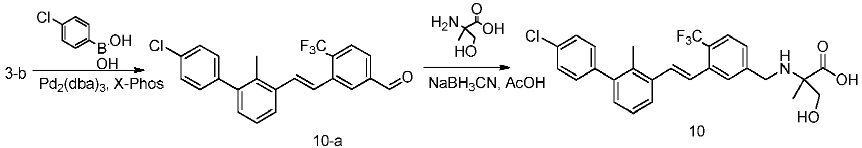

### Synthesis of compound 10-a

To a solution of *p*-chlorophenylboronic acid (173.6 mg, 1.11 mmol) and compound **3-b** (300 mg, 0.924 mmol) in toluene (20 mL) were added tris(dibenzylideneacetone)dipalladium (84.2 mg, 0.092 mmol), 2-dicyclohexylphosphino-2',4',6'-triisopropylbiphenyl (175.4 mg, 0.368 mmol) and potassium phosphate (588.4 mg, 2.772 mmol) at room temperature, and the reaction solution was heated to 90°C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **10-a** (144 mg, yield: 38.9%).

### Synthesis of compound 10

To a mixed solution of **10-a** (144 mg, 0.359 mmol) and 2-methylserine (85.5 mg, 0.718 mmol) in methanol (10 mL) and dichloromethane (10 mL) was added acetic acid (43.1 mg, 0.718 mmol) at room temperature, and the reaction solution was stirred at room temperature for 1 hour. Then, to the reaction solution was added sodium cyanoborohydride (112.8 mg, 1.795 mmol) and was stirred for 16 hours. After the completion of the reaction, the organic solvent was concentrated by rotary evaporation to dryness, and the residue was dissolved with ethyl acetate (20 mL), followed by washing with water (20 mL) and saturated brine (20 mL) sequentially. The obtained organic phase was dried over anhydrous sodium sulfate, concentrated under reduced pressure, and the residue was purified by silica gel thin layer chromatography preparative plate (dichloromethane: methanol = 10: 1) to obtain compound **10** (27 mg, yield: 14.9%). LC-MS (ESI): m/z = 502.0 [M-H]+.

¹H NMR (500 MHz, CD₃OD) *δ*: 8.16 (s, 1H), 7.80-7.78 (d, *J* = 8.0 Hz, 1H), 7.66-7.56 (m, 3H), 7.46-7.44 (m, 2H), 7.36-7.30 (m, 4H), 7.20-7.18 (m, 1H), 4.31-4.26 (m, 2H), 4.01-3.99 (d, *J* = 12.0 Hz, 1H), 3.85-3.83 (d, *J* = 12.0 Hz, 1H), 2.33 (s, 3H), 1.56 (s, 3H) ppm.

### Example 11

(*E*)-2-(3-(2-(4'-methoxy-2-methylbiphenyl-3-yl)vinyl)-4-(trifluoromethyl)benzylamino)-3-hydroxy-2-methylpropanoic acid (compound **11**)

Synthesis route:

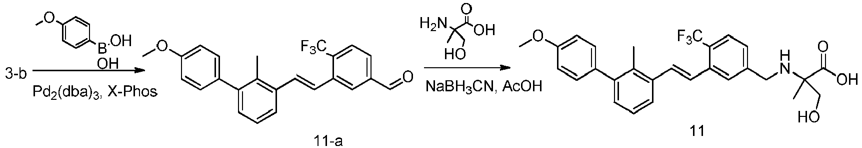

### Synthesis of compound 11-a

To a solution of p-methoxyphenylboronic acid (168.7 mg, 1.11 mmol) and compound **3-b** (300 mg, 0.924 mmol) in toluene (20 mL) were added tris(dibenzylideneacetone)dipalladium (84.2 mg, 0.092 mmol), 2-dicyclohexylphosphino-2',4',6'-triisopropylbiphenyl (175.4 mg, 0.368 mmol) and potassium phosphate (588.4 mg, 2.772 mmol) at room temperature, and the reaction solution was heated to 90 °C and stirred overnight under nitrogen. After the reaction solution was cooled to room temperature, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **11-a** (325 mg, yield: 74.9%).

¹H NMR (500 MHz, CDCl₃) *δ*: 10.15 (s, 1H), 8.28 (s, 1H), 7.86 (s, 2H), 7.76-7.73 (d, *J* = 15.5 Hz, 1H), 7.58-7.56 (d, *J* = 7.5 Hz,1H), 7.53-7.50 (d, *J* = 16.0 Hz, 1H), 7.44-7.41 (m, 2H), 7.38-7.34 (m, 1H), 7.11-7.08 (d, *J* = 16.0 Hz, 1H), 6.98-6.96 (d, *J* = 7.0 Hz, 2H), 3.87 (s, 3H), 2.34 (s, 3H) ppm.

### Synthesis of compound 11

To a mixed solution of **11-a** (325 mg, 0.82 mmol) and 2-methylserine (195.4 mg, 1.64 mmol) in methanol (10 mL) and dichloromethane (10 mL) was added acetic acid (98.5 mg, 1.64 mmol) at room temperature, and the reaction solution was stirred at room temperature for 1 hour. Then, to the reaction solution was added sodium cyanoborohydride (571.8 mg, 9.1 mmol) and was stirred for 16 hours. After the completion of the reaction, the organic solvent was concentrated by rotary evaporation to dryness, and the residue was dissolved with ethyl acetate (20 mL), followed by washing with water (20 mL) and saturated brine (20 mL) sequentially. The obtained organic phase was dried over anhydrous sodium sulfate, concentrated under reduced pressure, and the residue was purified by silica gel thin layer chromatography preparative plate (dichloromethane: methanol = 10: 1) to obtain compound **11** (76 mg, yield: 24.6%). LC-MS (ESI): m/z = 498.0 [M-H]+.

¹H NMR (500 MHz, CD₃OD) *δ*: 8.16 (s, 1H), 7.80-7.78 (d, *J* = 7.5 Hz, 1H), 7.68-7.62 (m, 2H), 7.54-7.53 (d, *J* = 7.5 Hz, 1H), 7.34-7.32 (m, 2H), 7.29-7.18 (m, 4H), 7.02-7.00 (m, 1H), 4.33-4.25 (m, 2H), 4.01-3.99 (d, *J* = 2.0 Hz,1H), 3.85-3.83 (d, *J* = 12.5 Hz, 1H), 3.86 (s, 3H), 2.35 (s, 3H), 1.55 (s, 3H) ppm.

### Example 12

(*E*)-2-(3-(3-(2,3-dihydrobenzo[b][1,4]dioxin-6-yl)-2-methylstyryl)-4-fluorobenzylamino)-3-hydroxypropanoic acid (compound **12**)

Synthesis route:

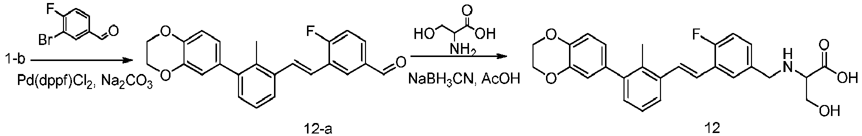

### Synthesis of compound 12-a

To a solution of 3-bromo-4-fluorobenzaldehyde (161 mg, 0.79 mmol) and **1-b** (300 mg, 0.79 mmol) in 1,4-dioxane (20 mL) and water (2 mL) were added [1,1'-bis (diphenylphosphino)ferrocene]palladium dichloride (57.8 mg, 0.079 mmol) and sodium carbonate (216.2 g, 2.4 mmol) at room temperature, and the reaction solution was heated to 80 °C and stirred under nitrogen for 16 hours. The reaction solution was cooled to room temperature and concentrated under reduced pressure, and the residue was purified by silica gel column chromatography (petroleum ether: ethyl acetate = 15: 1) to obtain compound **12-a** (140 mg, yield: 47%).

### Synthesis of compound 12

To a mixed solution of **12-a** (140 mg, 0.37 mmol) and serine (77.7 mg, 0.74 mmol) in methanol (5 mL) and dichloromethane (5 mL) was added acetic acid (0.04 mL, 0.65 mmol) at room temperature, and the reaction solution was stirred at room temperature for 6 hours. Then, to the reaction solution was added sodium cyanoborohydride (70 mg, 1.1 mmol) and was stirred for 18 hours. The reaction solution was concentrated under reduced pressure, and the residue was purified by high performance liquid chromatography (mobile phase: water (10 mM ammonium bicarbonate), acetonitrile; gradient: 30% to 60% (the initial mobile phase was 30% water and 70% acetonitrile, and the final mobile phase was 60% water and 40% acetonitrile, wherein % refers to volume percentage)) to obtain compound **12** (18 mg, yield: 11%). LC-MS (ESI): m/z = 464 [M-H]+.

¹H NMR (400 MHz,DMSO-d6) *δ*: 7.89 (d, *J* = 4.8 Hz, 1H), 7.64 (d, *J* = 6.0 Hz, 1H), 7.57 (d, *J* = 12.8 Hz, 1H), 7.40-7.37 (m, 1H), 7.28-7.22 (m, 2H), 7.16-7.12 (m, 2H), 6.92 (d, *J* = 6.4 Hz, 1H), 6.80 (d, *J* = 2.0 Hz, 1H), 6.77-6.75 (dd, *J₁* = 2.0 Hz, *J₂* = 6.4 Hz, 1H), 4.28 (s, 4H), 4.04-3.93 (q, 2H),3.69-3.65 (m, 2H), 3.19-3.17 (m, 1H), 2.29 (s, 3H) ppm.

### Example 13

(*E*)-2-(3-(2-(2'-fluoro-2-methylbiphenyl-3-yl)vinyl)-4-(trifluoromethyl)benzylamino)-3-hydroxypropanoic acid (compound **13**)

Synthesis route:

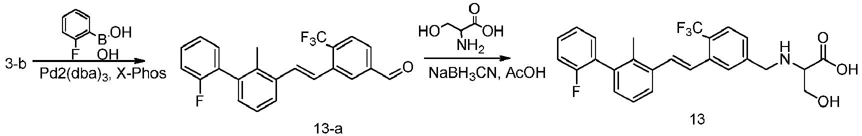

### Synthesis of compound 13-a

To a solution of 2-fluorophenylboronic acid (259 mg, 1.85 mmol) and compound **3-b** (500 mg, 1.54 mmol) in toluene (20 mL) were added tris(dibenzylideneacetone)dipalladium (50 mg, 0.05 mmol), 2-dicyclohexylphosphino-2',4',6'-triisopropylbiphenyl (100 mg, 0.21 mmol) and potassium phosphate (983 mg, 4.63 mmol) at room temperature, and the reaction solution was heated to 90 °C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 100: 1) to obtain **13-a** (250 mg, yield: 42%) as a yellow solid. LC-MS (ESI): m/z = 385 [M-H]⁺.

### Synthesis of compound 13

To a mixed solution of **13-a** (125 mg, 0.33 mmol) and serine (68 mg, 0.65 mmol) in methanol (10 mL) and dichloromethane (10 mL) was added acetic acid (0.04 mL, 0.65 mmol) at room temperature, and the reaction solution was stirred at room temperature for 2 hours. Then, to the reaction solution was added sodium cyanoborohydride (82 mg, 1.3 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein % refers to volume percentage)) to obtain compound **13** (13 mg, yield: 8.3%). LC-MS (ESI): m/z = 474 [M-H]+.

¹H NMR (400 MHz, DMSO-d6) *δ*: 8.07(s, 1H), 7.75-7.74 (m, 1H), 7.64-7.52 (m, 4H), 7.36-7.20 (m, 6H), 4.06-3.96 (m, 2H), 3.68-3.64 (m, 2H), 3.22-3.18 (m, 1H), 2.21 (s, 3H) ppm.

### Example 14

(*S,E*)-2-(3-(3-(5-fluoro-2,3-dihydrobenzo[b][1,4]dioxin-6-yl)-2-methylstyryl)-4-methylbenzylamino)-3-hydroxypropanoic acid (compound **14**)

Synthesis route:

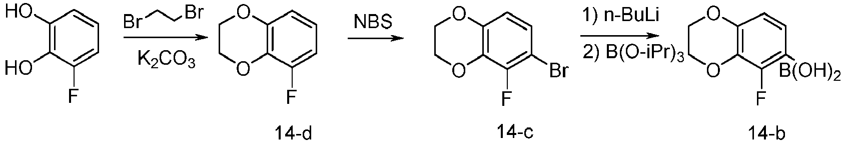

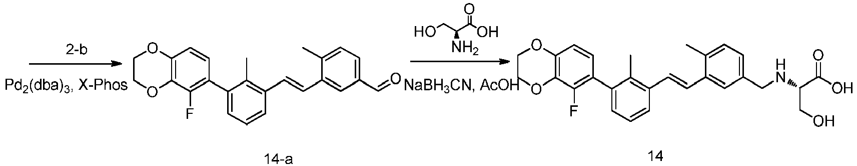

### Synthesis of compound 14-d

3-Fluorocatechol (2 g, 15.63 mmol) was dissolved in *N*, *N*-dimethylformamide (10 mL), and anhydrous potassium carbonate (6.5 g, 46.89 mmol) and 1,2-dibromoethane (14.7 g, 78.15 mmol) were added. The reaction mixture was heated to 80 °C and stirred for 24 hours. After the reaction mixture was cooled to room temperature, the reaction mixture was poured into ice water (100 mL), and extracted with petroleum ether (100 mL × 2). The organic phase was dried over anhydrous sodium sulfate, filtered, and the filtrate was concentrated under reduced pressure to obtain **14-d** as a light brown oil (2.3 g, yield: 95.8%). No further purification was required to this product.

### Synthesis of compound 14-c

Compound **14-d** (2.3 g, 14.93 mmol) was dissolved in *N, N-*dimethylformamide (20 mL). After the mixture was cooled to 0 °C, to the mixture was added N-bromosuccinimide (2.64 g, 14.93 mmol) in 10 portions. After the reaction mixture was stirred at room temperature for 16 hours, the reaction mixture was poured into ice water (200 mL), extracted with ethyl acetate (200 mL × 2). The organic phase was separated, dried over anhydrous sodium sulfate, filtered, the filtrate was concentrated under reduced pressure, and the residue was was purified by silica gel column chromatography (petroleum ether: ethyl acetate = 50: 1) to obtain **14-c** (2.5g , yield: 73%) as a light yellow solid.

¹H NMR (500MHz, CDCl₃) *δ*: 6.96 (dd, 1H), 6.59(dd, 1H), 4.27-4.32 (m, 4H) ppm.

### Synthesis of compound 14-b

Compound **14-c** (2.3 g, 10 mmol) was dissolved in anhydrous tetrahydrofuran (30 mL), and cooled to -70 °C, and a 2.5 M solution of *n*-butyl lithium in n-hexane (6 mL, 15 mmol) was added dropwise. After the reaction solution was stirred for 1 hour, to the reaction solution was added triisopropyl borate (2.7 g, 15 mmol) and continued to be stirred for 1 hour. The reaction solution was warmed to room temperature, followed by addition of 2N hydrochloric acid (20 mL), and stirred for 30 minutes. The reaction solution was extracted with ethyl acetate (100 mL), dried over anhydrous sodium sulfate, filtered, and the filtrate was concentrated under reduced pressure. To the residue was added petroleum ether (20 mL) and vigorously stirred to obtain **14-b** (1.2 g, yield: 61%) as a white solid.

¹H NMR (500 MHz, DMSO-*d*₆) *δ*: 7.97 (s, 2H), 6.99 (m, 1H), 6.65 (m, 1H), 4.27 (m, 4H) ppm.

### Synthesis of compound 14-a

Compound **14-b** (74 mg, 0.37 mmol) was dissolved in toluene (5 mL), and **2-b** (100 mg, 0.31 mmol), tris(dibenzylideneindeneacetone)dipalladium (27 mg, 0.04 mmol), 2-dicyclohexylphosphino-2,4,6-triisopropylbiphenyl (59 mg, 0.13 mmol) and anhydrous potassium phosphate (197 mg, 0.93 mmol) were added. The reaction mixture was heated to 105 °C and stirred for 16 hours under nitrogen. After the reaction mixture was cooled to room temperature, the reaction mixture was concentrated under reduced pressure, and the residue was purified by silica gel column chromatography (petroleum ether: ethyl acetate = 10: 1) to obtain **14-a** (60 mg, yield: 45%) as a yellow solid.

¹H NMR (500 MHz, CDCl₃) *δ*:10.12 (s, 1H), 8.09 (d, *J* = 1 Hz, 1H), 7.69 (dd, *J* = 8 Hz, *J* = 2 Hz, 1H), 7.37 (m, 2H), 7.28 (m, 1H), 7.17 (m, 2H), 6.78 (m, 1H), 6.70 (m, 2H), 4.34 (m, 4H), 2.51 (s, 3H), 2.28 (s, 3H) ppm.

### Synthesis of compound 14

Compound **14-a** (60 mg, 0.14 mmol) and L-serine (29 mg, 0.27 mmol) was dissolved in a mixed solution of tetrahydrofuran (3 mL), ethanol (3 mL) and water (3 mL), and sodium hydroxide (22 mg, 0.54 mmol) was added, and the reaction solution was stirred at 25 °C for 18 hours. Then, to the reaction solution was added sodium borohydride (20 mg, 0.53 mmol) and was stirred for half an hour. The reaction solution was concentrated under reduced pressure, and the pH of the residue was adjusted to 5 with 0.5 M hydrochloric acid, and then the mixture was extracted with ethyl acetate (50 mL × 2). The organic phase was dried over anhydrous sodium sulfate, concentrated under reduced pressure, and the residue was purified by silica gel column chromatography (dichloromethane: methanol = 10: 1) to obtain compound **14** (10 mg, yield: 14%). LC-MS (ESI): m/z = 478 [M-H]+.

¹H NMR (500 MHz, CDCl₃) *δ*: 7.39 (s, 2H), 6.89-7.11 (m, 6H), 6.57-6.65 (m, 2H), 4.24 (s, 4H), 3.64-3.81 (br, 4H), 3.17 (br, 2H), 2.17 (s, 3H), 2.11 (s, 3H) ppm.

### Example 15

(E)-2-(3-(2-(2',3'-difluoro-2-methylbiphenyl-3-yl)vinyl)-4-(trifluoromethyl)benzylamino)-3-hydroxypropanoic acid (compound **15**)

Synthesis route:

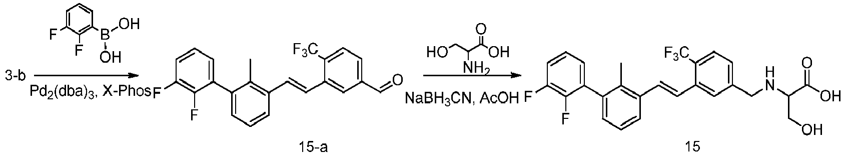

### Synthesis of compound 15-a

To a solution of 2,3-difluorophenylboronic acid (632 mg, 4.0 mmol) and compound **3-b** (648 mg, 2.0 mmol) in toluene (30 mL) were added tris(dibenzylideneacetone)dipalladium (183 mg, 0.2 mmol), 2-dicyclohexylphosphino-2',4',6'-triisopropylbiphenyl (200 mg, 0.42 mmol) and potassium phosphate (2120 mg, 10.0 mmol) at room temperature, and the reaction solution was heated to 100 °C and stirred overnight under nitrogen. After the completion of the reaction, the reaction solution was diluted with ethyl acetate (100 mL), and washed with water (100 mL) and saturated brine (100 mL) sequentially. The obtained organic phase was dried over anhydrous sodium sulfate, concentrated under reduced pressure, and the residue was purified by silica gel column chromatography (petroleum ether: ethyl acetate = 100: 1) to obtain **15-a** (300 mg, yield: 37%) as a canary solid.

¹H NMR (400 MHz, CD₃OD) *δ*:10.14 (s, 1H), 8.27 (s, 1H), 7.87 (s, 2H), 7.65 (d, *J* = 6.0 Hz, 1H), 7.50 (d, *J* = 12.4 Hz, 1H), 7.39-7.36 (m, 1H), 7.33 (t, *J* = 6.0 Hz, 1H), 7.22-7.14 (m, 3H), 7.03-7.01 (m, 1H), 2.28 (s, 3H) ppm.

### Synthesis of compound 15

To a mixed solution of **15-a** (120 mg, 0.3 mmol) and serine (63 mg, 0.6 mmol) in methanol (10 mL) and dichloromethane (10 mL) was added acetic acid (0.04 mL, 0.65 mmol) at room temperature, and the reaction solution was stirred at room temperature for 2 hours. Then, to the reaction solution was added sodium cyanoborohydride (56.7 mg, 0.9 mmol) and was stirred for 12 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein % refers to volume percentage)) to obtain compound **15** (26 mg, yield: 18%). LC-MS (ESI): m/z = 492 [M-H]+.

¹H NMR (400 MHz, DMSO-*d*₆) *δ*: 8.04 (s, 1H), 7.71 (d, *J* = 6.8 Hz, 1H), 7.66 (d, *J* = 6.0 Hz, 1H), 7.59 (d, *J* = 12.8 Hz, 1H), 7.52-7.47 (m, 2H), 7.37 (t, *J* = 6.0 Hz, 1H), 7.34-7.23 (m, 3H), 7.19-7.16 (m, 1H), 4.00 (d, *J* =12.0 Hz, 1H), 3.88 (d, *J* = 12.0 Hz, 1H), 3.55-3.49 (m, 2H), 2.94 (d, *J* = 4.8 Hz, 1H), 2.22 (s, 3H) ppm.

### Example 16

(*S,E*)-2-(3-(3-(5-fluoro-2,3-dihydrobenzo[b][1,4]dioxin-6-yl)-2-methylstyryl)-4-(trifluoromethyl)benzyl)-3-hydroxypropanoic acid (compound **16**)

Synthesis route:

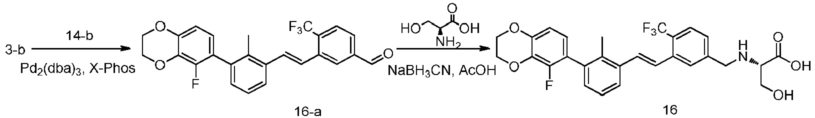

### Synthesis of compound 16-a

Compound **14-b** (300 mg, 1.48 mmol) was dissolved in toluene (5 mL), and compound **3-b** (400 mg, 1.24 mmol), tris(dibenzylideneindeneacetone)dipalladium (100 mg, 0.11 mmol), 2-dicyclohexylphosphino-2,4,6-triisopropylbiphenyl (100 mg, 0.21 mmol) and anhydrous potassium phosphate (790 mg, 3.72 mmol) were added. The reaction mixture was heated to 105 °C and stirred for 24 hours under nitrogen. After the reaction mixture was cooled to room temperature, the reaction mixture was concentrated under reduced pressure, and the residue was purified by silica gel column chromatography (petroleum ether: ethyl acetate = 10: 1) to obtain **16-a** (360 mg, yield: 66%) as a yellow solid.

### Synthesis of compound 16

Compound **16-a** (72 mg, 0.16 mmol) and L-serine (26 mg, 0.26 mmol) were dissolved in a mixed solution of tetrahydrofuran (2 mL), ethanol (2 mL) and water (2 mL), and sodium hydroxide (22 mg, 0.54 mmol) was added, and the reaction solution was stirred at 25 °C for 18 hours. Then, to the reaction solution was added sodium borohydride (20 mg, 0.53 mmol) and was stirred for half an hour. The reaction solution was concentrated under reduced pressure, and the pH of the residue was adjusted to 5 with 0.5 M hydrochloric acid, and the mixture was extracted with ethyl acetate (50 mL × 2). The organic phase was dried over anhydrous sodium sulfate and concentrated under reduced pressure. The residue was purified by silica gel thin-layer chromatography preparative plate (dichloromethane: methanol = 10: 1) to obtain compound **16** (8 mg, yield: 10%). LC-MS (ESI): m/z = 532 [M-H]+.

¹H NMR (500 MHz, CD₃OD) *δ*: 8.12 (s, 1H), 7.76 (d, *J* = 9 Hz, 1H), 7.57-7.65 (m, 3H), 7.28-7.36 (m, 2H), 7.16 (d, *J* = 8 Hz, 1H), 6.76 (dd, *J* = 9 Hz, *J* = 1 Hz,1H), 6.69 (m,1H), 4.35 (s, 4H), 4.31 (d, *J* = 13 Hz, 1H), 4.23 (d, *J* = 13 Hz, 1H), 3.98 (m, 1H), 3.87 (m, 1H), 3.52 (m, 1H), 2.28 (s, 3H) ppm.

### Example 17

(*E*)-2-(3-(2-(2'-fluoro-2-methylbiphenyl-3-yl)vinyl)-4-(trifluoromethyl)benzylamino)-3-hydroxy-2-methylpropanoic acid (compound **17**)

Synthesis route:

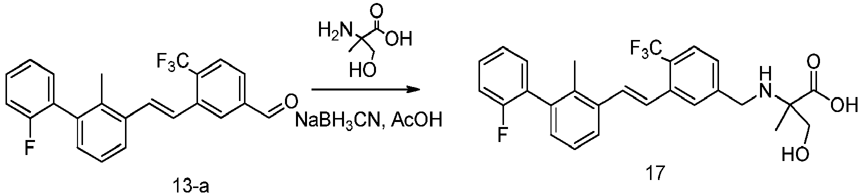

### Synthesis of compound 17

To a mixed solution of **13-a** (100 mg, 0.26 mmol) and 2-methylserine (62 mg, 0.52 mmol) in methanol (10 mL) and dichloromethane (10 mL) was added acetic acid (0.03 mL, 0.52 mmol) at room temperature, and the reaction solution was stirred at room temperature for 2 hours. Then, to the reaction solution was added sodium cyanoborohydride (65 mg, 1.04 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 42% to 72% (the initial mobile phase was 42% water and 58% acetonitrile, and the final mobile phase was 72% water and 28% acetonitrile, wherein, % refers to volume percentage) to obtain compound **17** (24 mg, yield: 19%). LC-MS (ESI): m/z = 486 [M-H]+.

¹H NMR (400 MHz, DMSO-d6) *δ*: 8.07 (s, 1H), 7.77-7.51 (d, *J* = 7.2 Hz, 1H), 7.64-7.57 (m, 3H), 7.49-7.45 (m, 1H), 7.39-7.26 (m, 6H), 3.99 (s, 2H), 3.60-3.54 (m, 2H), 2.22 (s, 3H), 1.27(s, 3H) ppm.

### Example 18

2-(3-(2-(2-Methylbiphenyl-3-yl)ethyl)-4-(trifluoromethyl)benzylamino)-3-hydroxypropanoic acid (compound **18**)

Synthesis route:

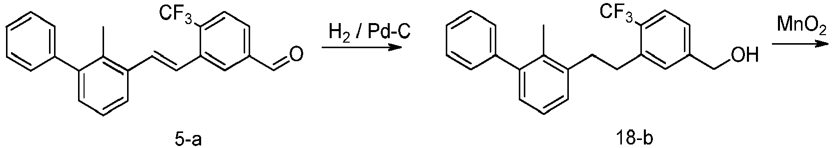

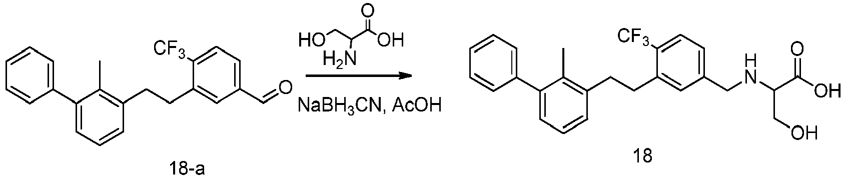

### Synthesis of compound 18-b

Compound 5-a (500 mg, 1.37 mmol) was dissolved in a mixed solution of isopropanol (10 mL) and tetrahydrofuran (20 mL), and to the solution was added 10% palladium-carbon (150 mg) at room temperature, and the reaction was stirred for 12 hours under the atmosphere of hydrogen (1 atm). The reaction solution was filtered, and the filtrate was concentrated under reduced pressure to obtain **18-b** (600 mg) as a white solid. The product was directly used in the next step without further purification. LC-MS (ESI): m/z = 371 [M+1]⁺.

### Synthesis of compound 18-a

Compound **18-b** (600 mg, 1.62 mmol) was dissolved in dioxane (20 mL), and active manganese dioxide (2.1 g, 24.3 mmol) was added at room temperature. The reaction mixture was stirred at 50 °C for 6 hours. The reaction mixture was filtrated, the filtrate was concentrated under reduced pressure, and the residue was purified by silica gel column chromatography (petroleum ether: ethyl acetate = 40: 1) to obtain **18-a** (300 mg, yield: 50%) as a yellow solid. LC-MS (ESI): m/z =369[M+H]⁺.

### Synthesis of compound 18

To a mixed solution of **18-a** (100 mg, 0.27 mmol) and serine (57 mg, 0.54 mmol) in methanol (10 mL) and dichloromethane (10 mL) was added acetic acid (0.03 mL, 0.54 mmol) at room temperature, and the reaction solution was stirred at room temperature for 2 hours. Then, to the reaction solution was added sodium cyanoborohydride (68 mg, 1.08 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase is 70% water and 30% acetonitrile, wherein, % refers to volume percentage) to obtain compound **18** (17 mg, yield: 13.8%). LC-MS (ESI): m/z = 456 [M-H]+.

¹H NMR (400 MHz, DMSO-d6) *δ*: 7.68-7.67 (m, 1H), 7.58(s, 1H), 7.46-7.43 (m, 3H), 7.38-7.35 (m, 1H), 7.31-7.29 (m, 2H), 7.22-7.19 (m, 2H), 7.07-7.05 (m, 1H),4.00-3.98 (d, *J* = 11.2 Hz, 1H), 3.87-3.84 (d, *J* = 11.2Hz, 1H), 3.62-3.67 (m, 2H), 3.13-3.11 (m, 1H), 2.99-2.92 (m, 4H), 2.17 (s, 3H) ppm.

### Example 19

(E)-2-(3-(2-(2-methylbiphenyl-3-yl)vinyl)-4-(trifluoromethyl)benzylamino)-3-hydroxy-2-hydroxymethylpropanoic acid (compound **19**)

Synthesis route:

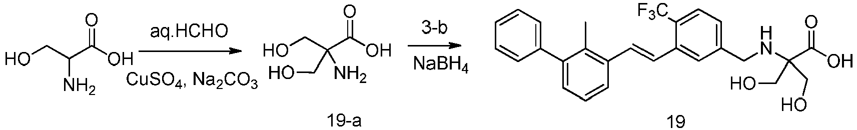

### Synthesis of compound 19-a

To a 500 mL reaction flask were added serine (2.95 g, 28 mmol), anhydrous copper sulfate (0.96 g, 6 mmol), sodium carbonate (11.9 g, 112 mmol), 37% aqueous formaldehyde (20 mL) and 400 mL of water. The mixture was heated to reflux for 2 hours, cooled to room temperature and filtered, and the filtrate was concentrated under reduced pressure. The residue was dissolved with an appropriate amount of water. After the pH of the aqueous solution was adjusted to 3 with 4 M hydrochloric acid, the aqueous solution was purified on a Dowex-50X ion exchange column (6.0 cm × 40 cm, 200 ~ 400 mesh, hydrogen type). The ion exchange column was first rinsed with water, and rinsed with 250 mL of water after the pH value of the effluent was changed from acidic to neutral, and then the product was eluted with 2M ammonia and detected with ninhydrin chromogenic agent. The effluent which was colored on ninhydrin was collected and concentrated under reduced pressure. To the residue was added anhydrous ethanol (10 mL) and stirred vigorously, filtered, and the filter cake was dried in vacuum to obtain α-(hydroxymethyl) serine (2.2 g, yield: 58%).

¹H NMR (500 MHz, CD₃OD) *δ*: 3.90 (d, *J* = 14.0 Hz, 2H), 3.76 (d, *J* = 14.0 Hz, 2H) ppm.

### Synthesis of compound 19

α-(Hydroxymethyl) serine (135 mg, 1 mmol) was dissolved in water (3 mL) and 1 M aqueous sodium hydroxide (2 mL), followed by addition of a mixed solution of compound **3-b** (110 mg, 0.3 mmol) in tetrahydrofuran (3 mL) and ethanol (5 mL), and the reaction solution was stirred at room temperature for 16 hours. The reaction solution was cooled to 0 °C, followed by addition of sodium borohydride (38 mg, 1 mmol), and further stirred for 1 hour. The reaction solution was concentrated under reduced pressure, and the residue was diluted with water (20 mL), and the pH of which was adjusted to 5 with citric acid. A solid precipitated out and was filtered, and the filter cake was dried to obtain a crude product, which was then recrystallized with ethyl acetate (10 mL) to obtain **19** (20 mg, yield: 14%) as a white solid. LC-MS (ESI): m/z = 486 [M-H]+.

¹H NMR (500 MHz, CD₃OD) *δ*: 8.18 (s, 1H), 7.79 (d, *J* = 8.0 Hz, 1H), 7.63-7.69 (m, 2H), 7.57 (d, *J* = 8.0 Hz, 1H), 7.44-7.47 (m, 2H), 7.29-7.33 (m, 5H), 7.20 (d, *J* = 8.0 Hz, 1H), 4.38 (s, 2H), 4.01 (d, *J* = 12.0 Hz, 2H), 3.97 (d, *J* = 12.0 Hz, 2H), 2.34 (s, 3H) ppm.

### Example 20

(*E*)-2-(3-(3-(5-fluoro-2,3-dihydrobenzo[b][1,4]dioxin-6-yl)-2-methylstyryl)-4-(trifluoromethyl)benzylamino)-3-hydroxy-2-methylpropanoic acid (compound **20**)

Synthesis route:

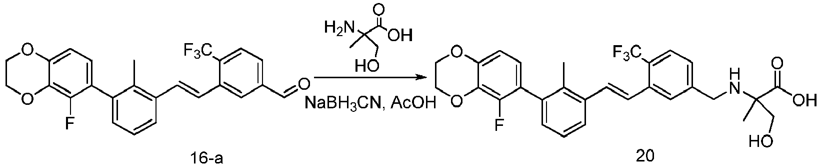

### Synthesis of compound 20

Compound **16-a** (80 mg, 0.18 mmol) and 2-methylserine (33 mg, 0.27 mmol) were dissolved in a mixed solution of tetrahydrofuran (2 mL), ethanol (2 mL) and water (2 mL), and sodium hydroxide (22 mg, 0.54 mmol) was added, and the reaction solution was stirred at 25 °C for 18 hours. Then, to the reaction solution was added sodium borohydride (20 mg, 0.53 mmol) and was stirred for half an hour. The reaction solution was concentrated under reduced pressure, and the pH of the residue was adjusted to 5 with 0.5 M hydrochloric acid, and then the mixture was extracted with ethyl acetate (50 mL × 2). The organic phase was dried over anhydrous sodium sulfate, concentrated under reduced pressure, and the residue was purified by silica gel thin-layer chromatography preparative plate (dichloromethane: methanol = 10: 1) to obtain compound **20** (12 mg, yield: 10%). LC-MS (ESI): m/z = 546 [M-H]+.

¹HNMR (500MHz, CD₃OD) *δ*: 8.17 (s, 1H), 7.76 (d, *J* = 9 Hz, 1H), 7.58-7.66 (m, 3H), 7.35-7.38 (m, 1H), 7.29-7.33 (m,1H), 7.17 (d, *J* = 8 Hz, 1H), 6.77 (dd, *J₁* = 9 Hz, *J₂* = 1 Hz, 1H), 6.69 (m, 1H), 4.38 (m, 2H), 4.35 (s, 4H), 4.10 (d, *J* = 12 Hz, 1H), 3.90 (d, *J* = 12 Hz, 1H), 2.28 (s, 3H),1.65 (s, 3H) ppm.

### Example 21

2-(3-(2-(2-Methylbiphenyl-3-yl)ethyl)-4-(trifluoromethyl)benzylamino)-3-hydroxy-2-methylpropanoic acid (compound **21**)

Synthesis route:

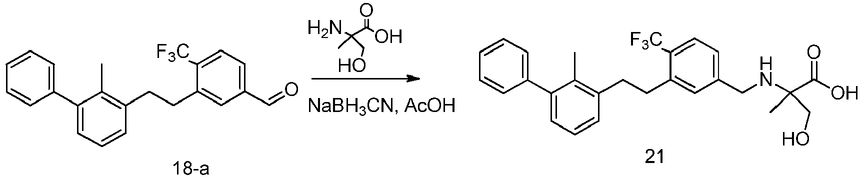

### Synthesis of compound 21

To a mixed solution of **18-a** (100 mg, 0.27 mmol) and 2-methylserine (65 mg, 0.54 mmol) in methanol (10 mL) and dichloromethane (10 mL) mL, 0.54 mmol) was added acetic acid (0.03 mL, 0.54 mmol) at room temperature, and the reaction solution was stirred at room temperature for 2 hours. Then, to the reaction solution was added sodium cyanoborohydride (68 mg, 1.08 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **21** (27 mg, yield: 21.3%). LC-MS (ESI): m/z = 470[M-H]⁺.

¹H NMR (400 MHz, DMSO-d6) *δ*:7.69-7.68 (d, *J* = 6.4 Hz, 1H), 7.62 (s, 1H), 7.49-7.43 (m, 3H), 7.38-7.35 (m, 1H), 7.31-7.29 (m, 2H), 7.25-7.20 (m, 2H),7.07-7.05 (m, 1H), 3.94-3.90 (m, 2H), 3.61-3.52 (m, 2H), 2.99-2.92 (m, 4H), 2.17 (s, 3H), 1.24 (s, 3H) ppm.

### Example 22

2-(3-(2-(2-Methylbiphenyl-3-yl)ethyl)-4-(trifluoromethyl)benzylamino) propanoic acid (compound **22**)

Synthesis route:

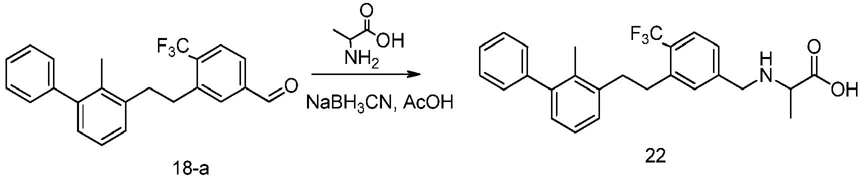

### Synthesis of compound 22

To a mixed solution of **18-a** (100 mg, 0.27 mmol) and alanine (44 mg, 0.54 mmol) in methanol (10 mL) and dichloromethane (10 mL) was added acetic acid (0.03 mL, 0.54 mmol) at room temperature, and the reaction solution was stirred at room temperature for 2 hours. Then, to the reaction solution was added sodium cyanoborohydride (68 mg, 1.08 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **21** (25 mg, yield: 21%). LC-MS (ESI): m/z = 440 [M-H]+.

¹H NMR (400 MHz, DMSO-d6) *δ*: 7.68-7.67 (d, *J* = 6.4 Hz, 1H), 7.59 (s, 1H), 7.46-7.43 (m, 3H), 7.38-7.35 (m, 1H), 7.30-7.29 (m, 2H), 7.22-7.19 (m, 2H), 7.07-7.15(m, 1H), 3.98-3.95 (d, *J* = 11.2 Hz, 1H), 3.85-3.83 (d, *J* = 11.2 Hz, 1H), 3.17-3.15 (m, 1H), 2.99-2.92(m, 4H), 2.17(s, 3H), 1.25-1.24 (d, *J* = 5.6 Hz, 3H) ppm.

### Example 23

(*E*)-2-(3-(2-(2',3'-difluoro-2-methylbiphenyl-3-yl)vinyl)-4-(trifluoromethyl)benzylamino)-3-hydroxy-2-methylpropanoic acid (compound **23**)

Synthesis route:

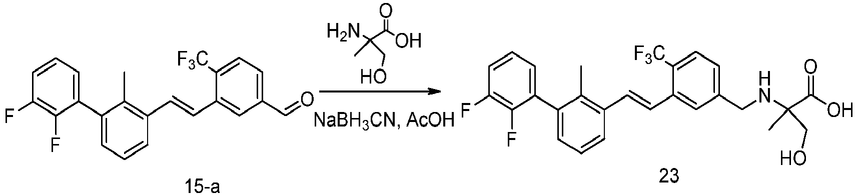

### Synthesis of compound 23

To a mixture of **15-a** (120 mg, 0.3 mmol) and 2-methylserine (71.4 mg, 0.6 mmol) in methanol (5 mL) and dichloromethane (5 mL) was added acetic acid (0.04 mL, 0.65 mmol) at room temperature, and the reaction solution was stirred at room temperature for 2 hours. Then, to the reaction solution was added sodium cyanoborohydride (56.7 mg, 0.9 mmol) and was stirred for 12 hours. The reaction solution was concentrated under reduced pressure, and the residue was purified by high performance liquid chromatography (mobile phase: water (10 mM ammonium bicarbonate), acetonitrile; gradient: 42% to 72% (the initial mobile phase was 42% water and 68% acetonitrile, and the final mobile phase was 72% water and 28% acetonitrile, wherein, % refers to volume percentage) to obtain compound **23** (26 mg, yield: 17%). LC-MS (ESI): m/z = 506 [M-H]+.

¹H NMR (400 MHz,DMSO-d6) *δ*: 8.19 (s, 1H), 7.81 (d, *J* = 6.4 Hz, 1H), 7.68-7.64 (m, 3H), 7.39-7.21 (m, 5H), 7.12-7.09 (m, 1H), 4.36 (d, *J* = 10.0 Hz, 1H), 4.30 (d, *J* = 10.0 Hz, 1H), 4.02 (d, *J* = 9.2 Hz, 1H), 3.85 (d, *J* = 10.0 Hz, 1H), 2.29 (s, 3H), 1.57 (s, 3H) ppm.

### Example 24

(*E*)-2-((7-(3-(2,3-dihydrobenzo[b][1,4]dioxin-6-yl)-2-methylstyryl)-2,3-dihydrobenzofuran-5-yl)methylamino)-3-hydroxy-2-methylpropanoic acid (compound **24**)

Synthesis route:

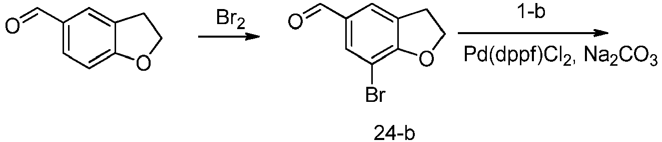

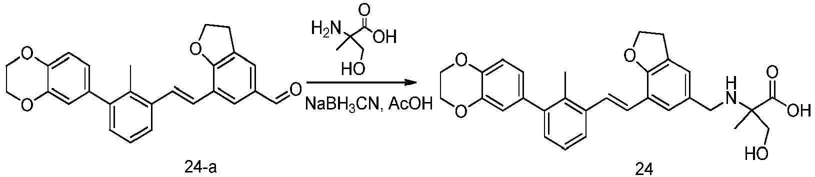

### Synthesis of compound 24-b

Benzodihydrofuran-5-carbaldehyde (2.96 g, 20 mmol) was dissolved in acetic acid (40 mL), and anhydrous sodium acetate (2.1 g, 24 mmol) was added. The reaction mixture was cooled to 10 °C, followed by addition of bromine (6.39 g, 40 mmol) dropwise, and then warmed to room temperature and stirred for 16 hours. To the mixture was added ice water (100 mL), and the pH of which was adjusted to 9 to 10 with potassium carbonate. The mixture was extracted with ethyl acetate (100 mL × 2), the organic phase was dried over anhydrous sodium sulfate, filtered and concentrated under reduced pressure, and the residue was purified by silica gel column chromatography (petroleum ether: ethyl acetate = 10: 1) to obtain **24-b** (3.8g, yield: 85%) as a yellow solid.

¹H NMR (500 MHz, CDCl₃) *δ*: 9.78 (s, 1H), 7.82 (d, *J* = 1 Hz, 1H), 7.66 (d, *J* = 1 Hz, 1H), 4.79 (t, *J* = 9 Hz, 2H), 3.38 (d, *J* = 9 Hz, 2H) ppm.

### Synthesis of compound 24-a

Compound **24-b** (158 mg, 0.69 mmol) was dissolved in dioxane (5 mL) and water (0.5 mL), and compound **1-b** (370 mg, 0.97 mmol), 1,1'-bis(diphenylphosphino)ferrocene palladium dichloride (59 mg, 0.07 mmol) and sodium carbonate (219 mg, 2.07 mmol) were added. The reaction mixture was heated to 80 °C and stirred for 16 hours under nitrogen. After the reaction mixture was cooled to room temperature, to the reaction mixture were added dichloromethane (50 mL) and water (50 mL), the organic phase was dried over anhydrous sodium sulfate and concentrated under reduced pressure, and the residue was purified by silica gel column chromatography (petroleum ether: ethyl acetate = 3: 1) to obtain **16-a** (180 mg, yield: 65%) as a yellow solid.

¹H NMR (500 MHz, CDCl₃) *δ*: 9.89 (s, 1H), 7.84 (s, 1H), 7.70 (s, 1H), 7.66 (d, *J* = 10 Hz, 1H), 7.57 (d, *J* = 8 Hz, 1H), 7.24 (m, 1H), 7.15 (d, *J* = 7 Hz, 1H), 7.04 (d, *J* = 11 Hz, 1H), 6.91(d, *J* = 8 Hz, 1H), 6.83 (d, *J* = 2 Hz, 1H), 6.77 (m, 1H), 4.80 (t, *J* = 9 Hz, 2H), 4.31 (s, 4H), 3.31 (d, *J* = 9 Hz, 2H), 2.32 (s,3H) ppm

### Synthesis of compound 24

Compound **24-a** (180 mg, 0.46 mmol) and 2-methylserine (54 mg, 0.92 mmol) were dissolved in a mixed solution of tetrahydrofuran (4 mL), ethanol (4 mL) and water (4 mL), and sodium hydroxide (75 mg, 1.84 mmol) was added and the reaction solution was stirred at 25 °C for 18 hours. Then, to the reaction solution was added sodium borohydride (70 mg, 1.84 mmol) and was stirred for half an hour. The reaction solution was concentrated under reduced pressure, and the pH of the residue was adjusted to 5 with 0.5 M hydrochloric acid, and then the mixture was extracted with ethyl acetate (50 mL × 2). The organic phase was dried over anhydrous sodium sulfate, concentrated under reduced pressure, and the residue was purified by silica gel thin-layer chromatography preparative plate (dichloromethane: methanol = 10: 1) to obtain compound **24** (12 mg, yield: 6%). LC-MS (ESI): m/z = 502 [M-H]+.

¹H NMR (500 MHz, CD₃OD) *δ*: 7.74 (d, *J* = 16 Hz, 1H), 7.56 (m, 1H), 7.48 (m, 1H), 7.29 (m, 1H), 7.21 (m,1H), 7.08 (m, 1H), 7.03 (m, *J* = 11 Hz, 1H), 6.88 (m, 1H), 6.76 (m, 1H), 4.78 (t, *J* = 9 Hz, 2H), 4.29 (s, 4H), 4.12 (m, 1H), 3.77 (m, 1H), 3.62 (m, 1H), 3.32 (t, *J* = 9 Hz, 2H), 2.29 (s, 3H), 1.49 (s, 3H) ppm.

### Example 25

(*E*)-2-(3-(3-(2,3-dihydrobenzo[b][1,4]dioxin-6-yl)-2-methylstyryl)-4-fluorobenzylamino)-3-hydroxy-2-methylpropanoic acid (compound **25**)

Synthesis route:

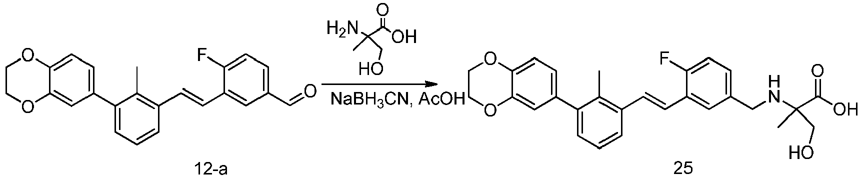

### Synthesis of compound 25

To a mixed solution of **12-a** (186 mg, 0.5 mmol) and 2-methylserine (119 mg, 1.0 mmol) in methanol (10 mL) and dichloromethane (10 mL) was added acetic acid (0.04 mL, 0.65 mmol) at room temperature, and the reaction solution was stirred at room temperature for 6 hours. Then, to the reaction solution was added sodium cyanoborohydride (94.5 mg, 1.5 mmol) and was stirred for 18 hours. The reaction solution was concentrated under reduced pressure, and the residue was purified by high performance liquid chromatography (mobile phase: water (10 mM ammonium bicarbonate), acetonitrile; gradient: 35% to 65% (the initial mobile phase was 35% water and 65% acetonitrile, and the final mobile phase was 65% water and 35% acetonitrile, wherein, % refers to volume percentage) to obtain compound **25** (29 mg, yield: 12.1%). LC-MS (ESI): m/z = 478 [M-H]+.

¹H NMR (400 MHz,CD3OD) *δ*: 7.98 (d, *J* = 4.0 Hz, 1H), 7.68 (d, *J* = 12.8 Hz, 1H), 7.59 (d, *J* = 6.4 Hz, 1H), 7.50-7.47 (m, 1H), 7.26-7.13 (m, 4H), 6.89 (d, *J*= 6.4 Hz, 1H), 6.77-6.73 (m, 2H), 4.29 (s, 4H), 4.26-4.18 (q, 2H), 3.99 (d, *J* = 10 Hz, 1H), 3.83(d, *J* = 10 Hz, 1H), 2.32 (s, 3H), 1.55 (s, 3H) ppm.

### Example 26

(*S,E*)-2-(3-(3-(5-fluoro-2,3-dihydrobenzo[b][1,4]dioxin-6-yl)-2-methylstyryl)-4-methylbenzylamino)-3-hydroxy-2-methylpropanoic acid (compound **26**)

Synthesis route:

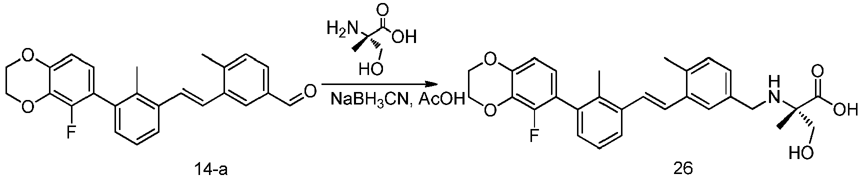

### Synthesis of compound 26

To a mixed solution of **14-a** (100 mg, 0.26 mmol) and (*S*)-2-methylserine (68 mg, 0.52 mmol) in methanol (10 mL) and dichloromethane (10 mL) was added acetic acid (0.03 mL, 0.52 mmol) at room temperature, and the reaction solution was stirred at room temperature for 2 hours. Then, to the reaction solution was added sodium cyanoborohydride (65 mg, 1.03 mmol) and was stirred for 16 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **26** (20 mg, yield: 15.6%). LC-MS (ESI): m/z = 490 [M-H]+.

¹H NMR (400 MHz, DMSO-d6) *δ*: 7.78 (s, 1H), 7.72-7.03 (d, *J* = 6.0 Hz, 1H), 7.38-7.35 (m, 1H), 7.31-7.25 (m, 3H), 7.23-7.22 (m, 1H), 7.12-7.11 (m, 1H), 6.81-6.79 (m, 1H), 6.73-6.70 (m, 1H), 4.34 (s, 4H), 4.01-3.93 (m, 2H), 3.67-3.65 (m, 1H), 3.59-3.57 (m, 1H), 2.41 (s, 3H), 2.19 (s, 3H), 1.29 (s, 3H) ppm.

### Example 27

(*R,E*)-2-(3-(2-(2-methylbiphenyl-3-yl)vinyl)-4-(trifluoromethyl)benzylamino)-3-hydroxy-2-methylpropanoic acid (compound **27**)

Synthesis route:

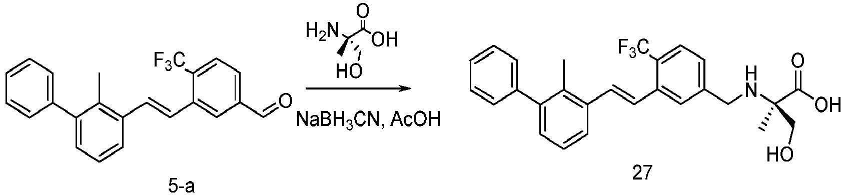

### Synthesis of compound 27

To a mixed solution of **5-a** (100 mg, 0.27 mmol) and (*R*)-2-methylserine (65 mg, 0.54 mmol) in methanol (10 mL) and dichloromethane (10 mL) was added acetic acid (32.8 mg, 0.54 mmol) at room temperature, and the reaction solution was stirred at room temperature for 1 hour. Then, to the reaction solution was added sodium cyanoborohydride (85.8 mg, 1.36 mmol) and was stirred for 16 hours. The reaction solution was concentrated under reduced pressure, and the residue was dissolved in ethyl acetate (50 mL), washed with water (20 mL) and saturated brine (20 mL). The organic phase was dried over anhydrous sodium sulfate, and concentrated under reduced pressure, and the residue was purified by silica gel thin-layer chromatography preparative plate (dichloromethane: methanol = 10: 1) to obtain compound 27 (24 mg, yield: 18.7%). LC-MS (ESI): m/z = 468 [M-H]⁺

¹H NMR (500MHz, CD₃OD) *δ*: 8.17 (s, 1H), 7.81-7.79 (d, *J* = 8.5 Hz, 1H), 7.68-7.62 (m, 2H), 7.57-7.55 (d, *J* = 8.0 Hz, 1H), 7.46-7.43 (m, 2H), 7.39-7.28 (m, 5H), 7.20-7.19 (d, *J* = 7.0Hz, 1H), 4.36-4.28 (q, 2H), 4.03-4.00 (d, *J* = 12.5Hz, 1H), 3.86-3.84 (d, *J* = 12.5Hz, 1H), 2.33 (s,3H), 1.57 (s,3H) ppm.

### Example 28

(*S,E*)-2-(3-(2-(2-methylbiphenyl-3-yl)vinyl)-4-methylbenzylamino)-3-hydroxy-2-methylpropanoic acid (compound **28**)

Synthesis route:

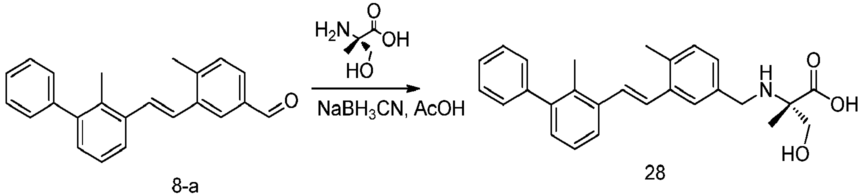

### Synthesis of compound 28

To a mixed solution of **8-a** (120 mg, 0.38 mmol) and (*S*)-2-methylserine (92 mg, 0.77 mmol) in methanol (10 mL) and dichloromethane (10 mL) was added acetic acid (0.034 mL, 0.77 mmol) at room temperature, and the reaction solution was stirred at room temperature for 2 hours. Then, to the reaction solution was added sodium cyanoborohydride (97 mg, 1.53 mmol) and was stirred for 12 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **28** (50 mg, yield: 31.7%). LC-MS (ESI): m/z = 414 [M-H]+.

¹H NMR (400 MHz, DMSO-d6) *δ*: 7.78 (s, 1H), 7.69-7.68 (d, *J* = 6.0 Hz, 1H), 7.47-7.44 (m, 2H), 7.39-7.36 (m, 2H), 7.33-7.22 (m, 6H), 7.15-7.14 (m, 1H), 4.01-3.93 (m, 2H), 3.67-3.65 (m, 1H), 3.59-3.58 (m, 1H), 2.41 (s, 3H), 2.27 (s, 3H), 1.29 (s, 3H) ppm.

### Example 29

(*S,E*)-2-(3-(2-(2-methylbiphenyl-3-yl)vinyl)-4-(trifluoromethyl)benzylamino)-3-hydroxy-2-methylpropanoic acid (compound **29**)

Synthesis route:

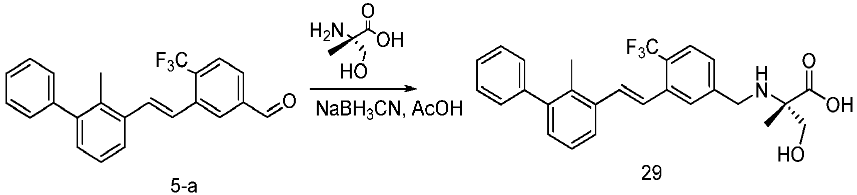

### Synthesis of compound 29

To a mixed solution of **5-a** (100 mg, 0.27 mmol) and (*S*)-2-methylserine (65 mg, 0.54 mmol) in methanol (10 mL) and dichloromethane (10 mL) was added acetic acid (32.8 mg, 0.54 mmol) at room temperature, and the reaction solution was stirred at room temperature for 1 hour. Then, to the reaction solution was added sodium cyanoborohydride (85.8 mg, 1.36 mmol) and was stirred for 16 hours. The reaction solution was concentrated under reduced pressure, and the residue was dissolved in ethyl acetate (50 mL), washed with water (20 mL) and saturated brine (20 mL), and the organic phase was dried over anhydrous sodium sulfate and concentrated under reduced pressure. The residue was purified by silica gel thin-layer chromatography preparative plate (dichloromethane: methanol = 10: 1) to obtain compound **29** (42 mg, yield: 32.8%). LC-MS (ESI): m/z = 468 [M-H]⁺

¹H NMR (500 MHz, CD₃OD) *δ*: 8.05 (s, 1H), 7.68-7.67 (d, *J* = 8.0 Hz, 1H), 7.56-7.50 (m, 2H), 7.45-7.44 (d, *J* = 7.5Hz, 1H), 7.34-7.31 (m, 2H), 7.27-7.16 (m, 5H), 7.08-7.07 (d, *J* = 7.0 Hz, 1H), 4.24-4.15 (q, 2H), 3.90-3.88 (d, *J* = 12.0 Hz, 1H), 3.74-3.71 (d, *J* = 12.0 Hz, 1H), 2.21 (s, 3H), 1.45 (s, 3H) ppm.

### Example 30

(*S,E*)-2-(3-(3-(5-fluoro-2,3-dihydrobenzo[b][1,4]dioxin-6-yl)-2-methylstyryl)-4-methylbenzylamino)-3-hydroxy-2-methylpropanoic acid (compound **30**)

Synthesis route:

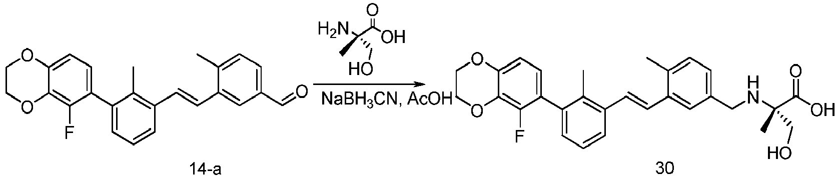

### Synthesis of compound 30

To a mixed solution of **14-a** (100 mg, 0.26 mmol) and (*S*)-2-methylserine (69 mg, 0.52 mmol) in methanol (10 mL) and dichloromethane (10 mL) was added acetic acid (0.03 mL, 0.52 mmol) at room temperature, and the reaction solution was stirred at room temperature for 2 hours. Then, to the reaction solution was added sodium cyanoborohydride (65 mg, 1.03 mmol) and was stirred for 12 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **30** (40 mg, yield: 31.3%). LC-MS (ESI): m/z = 490 [M-H]+.

¹H NMR (400 MHz, DMSO-d6) *δ*: 7.79 (s, 1H), 7.72-7.71 (d, *J* = 6.0 Hz, 1H), 7.39-7.35 (m, 1H), 7.31-7.26 (m, 3H), 7.23-7.21 (m, 1H),7.13-7.11 (m, 1H), 6.81-6.79 (m, 1H), 6.74-6.70 (m, 1H), 4.34 (s, 4H), 4.00-3.93 (m, 2H), 3.67-3.57 (m, 2H), 2.41 (s, 3H), 2.19 (s, 3H), 1.29(s, 3H) ppm

### Example 31

(*S,E*)-2-(3-(2-(2-methylbiphenyl-3-yl)vinyl)-4-methylbenzylamino)-3-hydroxy-2-methylpropanoic acid (compound **31**)

Synthesis route:

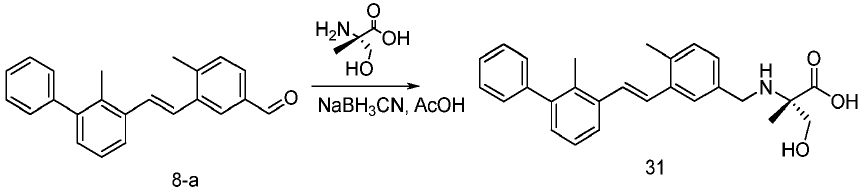

### Synthesis of compound 31

To a mixed solution of **8-a** (120 mg, 0.38 mmol) and (*S*)-2-methylserine (92 mg, 0.77 mmol) in methanol (10 mL) and dichloromethane (10 mL) was added acetic acid (0.04 mL, 0.77 mmol) at room temperature, and the reaction solution was stirred at room temperature for 2 hours. Then, to the reaction solution was added sodium cyanoborohydride (97 mg, 1.53 mmol) and was stirred for 12 hours. The reaction solution was concentrated under reduced pressure, and the residue was purified by high performance liquid chromatography (mobile phase: water (10 mM ammonium bicarbonate), acetonitrile; gradient: 40% to 70% (the initial mobile phase was 40% water and 60% acetonitrile, and the final mobile phase was 70% water and 30% acetonitrile, wherein, % refers to volume percentage) to obtain compound **31** (50 mg, yield: 31.7%). LC-MS (ESI): m/z = 414 [M-H]⁺.

¹H NMR (400 MHz, DMSO-d6) *δ*: 7.78 (s, 1H), 7.69-7.68 (d, *J* = 6.0 Hz, 1H), 7.47-7.44 (m, 2H), 7.39-7.36 (m, 2H), 7.33-7.22 (m, 6H), 7.15-7.14 (m, 1H), 4.01-3.93 (m, 2H), 3.67-3.65 (m, 1H), 3.59-3.58 (m, 1H), 2.41 (s, 3H), 2.27 (s, 3H), 1.29 (s, 3H) ppm.

### Example 32

(*E*)-2-(1-(3-(2-(2-methylbiphenyl-3-yl)vinyl)-4-(trifluoromethyl)phenyl)ethylamino)acetic acid (compound **32**)

Synthesis route:

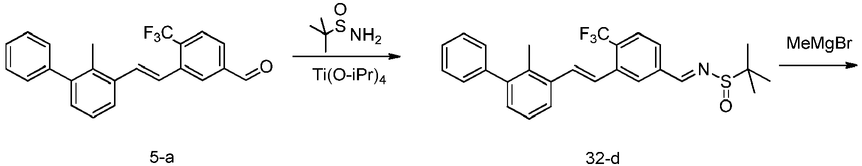

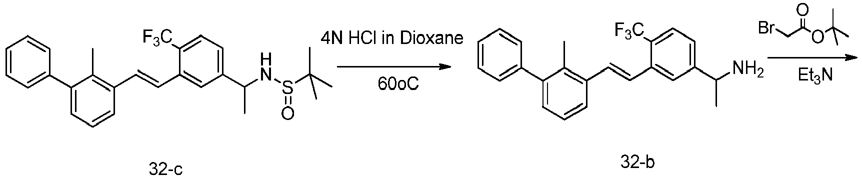

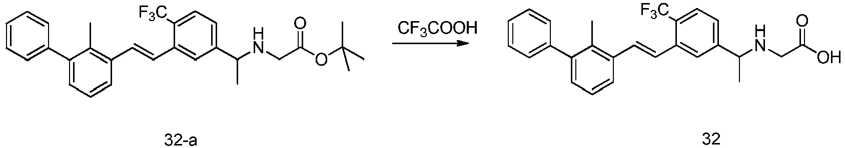

### Synthesis of compound 32-d

Compound **5-a** (500 mg, 1.37 mmol) and *t*-butylsulfinamide (248 mg, 2.05 mmol) were dissolved in tetrahydrofuran (10 mL), and titanium tetraisopropyl (773 mg, 2.74 mmol) was added. After the reaction solution was heated to 60 °C and stirred for 1 hour, the reaction solution was cooled to room temperature. To the reaction solution was added saturated sodium chloride solution (20 mL) and extracted with ethyl acetate (50 mL × 3), and the organic phase was dried over anhydrous sodium sulfate, filtered and concentrated under reduced pressure, and the residue was purified by silica gel column chromatography (petroleum ether: ethyl acetate = 5: 1) to obtain **32-d** (610 mg, yield: 95%) as a canary solid.

### Synthesis of compound 32-c

Compound **32-d** (610 mg, 1.3 mmol) was dissolved in anhydrous tetrahydrofuran (10mL), and the mixture was cooled to 0 °C, followed by addition of a solution of methylmagnesium bromide in ether (3 M, 0.9 mL, 2.7 mmol). After the completion of the addition, the reaction solution was stirred for 30 minutes. The reaction was quenched with saturated ammonium chloride solution (20 mL) and extracted with ethyl acetate (50 mL), and the organic phase was dried over anhydrous sodium sulfate, filtered and concentrated under reduced pressure, and the residue was washed with *tert-butyl* methyl ether (50 mL) to obtain **32-c** (410 mg, yield: 65%) as a white solid. No further purification was required to the product. LC-MS (ESI): m/z = 485 [M+H]⁺.

### Synthesis of compound 32-b

Compound **32-c** (410 mg, 0.85 mmol) was dissolved in methanol (5 mL), and a 4 N solution of hydrogen chloride in dioxane (5 mL) was added, and the reaction solution was heated to 60 °C and stirred for 2 hours. After the reaction solution was cooled to room temperature, the reaction solution was concentrated under reduced pressure, and the residue was washed with petroleum ether (50 mL) to obtain **32-b** (320 mg, yield: 91%) as a white solid. No further purification was required to the product. LC-MS (ESI): m/z = 382 [M+H]⁺.

### Synthesis of compound 32-a

Compound **32-b** (180 mg, 0.43 mmol) was dissolved in acetonitrile (5 mL), and triethylamine (1 mL) and *tert*-butyl bromoacetate (92 mg, 0.48 mmol) were added, and the reaction solution was heated to 80 °C and stirred for 3 hours. After the reaction solution was cooled to room temperature, the reaction solution was concentrated under reduced pressure, and the residue was purified by silica gel column chromatography (petroleum ether: ethyl acetate = 3: 1) to obtain **32-a** (60 mg, yield: 28%) as a yellow solid.

### Synthesis of compound 32

Compound **32-a** (60 mg, 0.12 mmol) was dissolved in dichloromethane (3 mL), and trifluoroacetic acid (3 mL) was added. After the reaction solution was stirred at room temperature for 3 hours, the reaction solution was concentrated under reduced pressure, and the pH of the residue was adjusted to 5 to 6 with saturated aqueous sodium hydrogen carbonate solution. The mixture was extracted with ethyl acetate (50 mL), and the organic phase was dried over anhydrous sodium sulfate, filtered and concentrated under reduced pressure, and the residue was purified by silica gel thin layer chromatography preparative plate (dichloromethane: methanol = 10: 1) to obtain 32 (25 mg, yield: 47%) as a white solid. LC-MS (ESI): m/z = 440 [M+H]⁺.

¹H NMR (500 MHz, CD₃OD) *δ*: 8.08 (s, 1H), 7.83 (d, *J* = 8 Hz, 1H), 7.64 (d, *J* = 15 Hz, 1H), 7.57 (m, 2H), 7.45 (m, 2H), 7.29-7.38 (m, 5H), 7.21 (d, *J* = 8 Hz, 1H), 4.60 (m, 1H), 3.46 (d, *J* = 16 Hz, 1H), 3.36 (d, *J* = 16 Hz, 1H), 2.34 (s, 3H), 1.73 (d, *J* = 7 Hz, 1H) ppm.

### Example 33

(*S,E*)-2-(3-(2-(2-fluorobiphenyl-3-yl)vinyl)-4-(trifluoromethyl)benzylamino)-3-hydroxy-2-methylpropanoic acid (compound **33**)

Synthesis route:

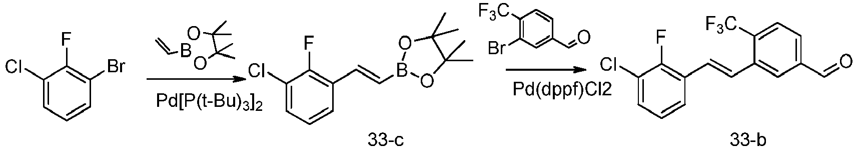

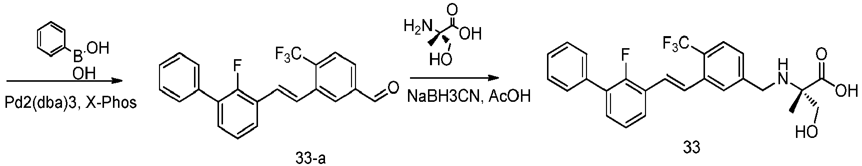

### Synthesis of compound 33-c

To a solution of 1-bromo-3-chloro-2-fluorobenzene (5.0 g, 23.87 mmol) and pinacol vinylboronate (4.47 g, 28.65 mmol) in toluene (100 mL) were added bis(tri-*tert*-butylphosphine)palladium (853.9 mg, 1.67 mmol) and triethylamine (19.32 g, 190.9 mmol) at room temperature, and the reaction solution was heated to 80 °C and stirred overnight under nitrogen. After the completion of the reaction, the reaction solution was diluted with ethyl acetate (50 mL), washed with water (50 mL) and saturated brine (50 mL). The obtained organic phase was dried over anhydrous sodium sulfate and concentrated under reduced pressure. The residue was purified by silica gel column chromatography (petroleum ether: ethyl acetate = 10: 1) to obtain compound **33-c** (3.45 g, yield: 51.1%).

¹H NMR (500 MHz, CDCl₃) *δ*: 7.56-7.52 (d, *J* = 18.5 Hz, 1H), 7.47-7.44 (m, 1H), 7.34-7.30 (m, 1H), 7.07-7.04 (t, 1H), 6.27-6.23 (d, *J* = 18.5 Hz, 1H), 1.32 (s, 12H) ppm.

### Synthesis of compound 33-b

To a solution of 3-bromo-4-trifluoromethylbenzaldehyde (2.57 g, 10.18 mmol) and **33-c** (3.45 g, 12.21 mmol) in 1,4-dioxane (40 mL) and water (2 mL) were added [1,1'-bis(diphenylphosphino)ferrocene]palladium dichloride (880 mg, 1.64 mmol) and sodium carbonate (2.69 g, 25.44 mmol) at room temperature, and the reaction solution was heated to 80 °C and stirred overnight under nitrogen. After the completion of the reaction, the reaction solution was diluted with ethyl acetate (50 mL), washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **33-b** (1.62 g, yield: 48.5%).

¹H NMR (500 MHz, CDCl₃) *δ*: 10.15 (s, 1H), 8.28 (s, 1H), 7.91-7.86 (m, 2H), 7.58-7.55 (m, 1H), 7.53-7.50 (m, 1H), 7.39-7.35 (m, 1H), 7.34-7.31 (d, *J* = 16.5 Hz, 1H), 7.15-7.12 (m, 1H) ppm.

### Synthesis of compound 33-a

To a solution of phenylboronic acid (721.8 mg, 5.92 mmol) and **33-b** (300 mg, 1.108 mmol) in toluene (50 mL) were added tris(dibenzylideneacetone) dipalladium (226.2 mg, 0.24 mmol), 2-dicyclohexylphosphino-2',4',6'-triisopropylbiphenyl (470 mg, 0.98 mmol) and potassium phosphate (3.14g, 14.79 mmol) at room temperature, and the reaction solution was heated to 90 °C and stirred overnight under nitrogen. After the completion of the reaction, the reaction solution was diluted with ethyl acetate (50 mL), and washed with water (50 mL) and saturated brine (50 mL) sequentially. The obtained organic phase was dried over anhydrous sodium sulfate, concentrated under reduced pressure, and the residue was purified by silica gel column chromatography (petroleum ether: ethyl acetate = 40: 1) to obtain compound **33-a** (1.56 g, yield: 85.2%).

¹H NMR (500 MHz, CDCl₃) *δ*: 10.14 (s, 1H), 8.31 (s, 1H), 7.89-7.85 (m, 2H), 7.64-7.56 (m, 4H), 7.49-7.39 (m, 5H), 7.28-7.24 (m, 1H) ppm.

### Synthesis of compound 33

To a mixed solution of **33-a** (100 mg, 0.27 mmol) and (*S*)-2-methylserine (64.3 mg, 0.54 mmol) in methanol (10 mL) and dichloromethane (10 mL) was added acetic acid (32.4 mg, 0.54 mmol) at room temperature, and the reaction solution was stirred at room temperature for 1 hour. Then, to the reaction solution was added sodium cyanoborohydride (84.8 mg, 1.35 mmol) and was stirred for 16 hours. After the completion of the reaction, the organic solvent was concentrated by rotary evaporation to dryness, and the residue was dissolved with ethyl acetate (50 mL), followed by washing with water (50 mL) and saturated brine (50 mL) sequentially. The obtained organic phase was dried over anhydrous sodium sulfate and concentrated under reduced pressure, and the residue was purified by silica gel thin layer chromatography preparative plate (dichloromethane: methanol = 10: 1) to obtain compound **33** (30 mg, yield: 23.6%). LC-MS (ESI): m/z = 472 [M-H]+.

¹H NMR (400 MHz, CD₃OD) *δ*: 8.17 (s, 1H), 7.79-7.77 (d, *J* = 8.0 Hz, 1H), 7.67-7.59 (m, 3H), 7.56-7.50 (m, 3H), 7.48-7.37 (m, 4H), 7.32-7.28 (m, 1H), 4.35-4.24 (m,2H), 4.01-3.98 (d, *J* = 12.4 Hz, 1H), 3.84-3.81 (d, *J* = 12.0 Hz,1H), 1.55 (s, 3H) ppm.

### Example 34

(*R,E*)-2-(3-(2-(2-fluorobiphenyl-3-yl)vinyl)-4-(trifluoromethyl)benzylamino)-3-hydroxy-2-methylpropanoic acid (compound **34**)

Synthesis route:

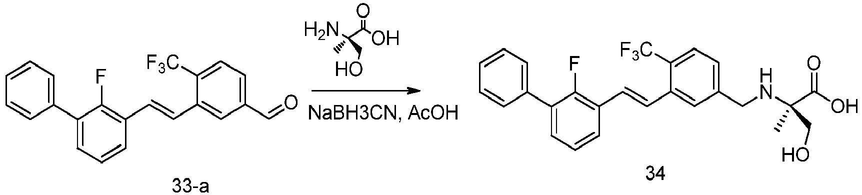

### Synthesis of compound 34

To a mixed solution of **33-a** (100 mg, 0.27 mmol) and (*R*)-2-methylserine (64.3 mg, 0.54 mmol) in methanol (10 mL) and dichloromethane (10 mL) was added acetic acid (32.4 mg, 0.54 mmol) at room temperature, and the reaction solution was stirred at room temperature for 1 hour. Then, to the reaction solution was added sodium cyanoborohydride (84.8 mg, 1.35 mmol) and was stirred for 16 hours. After the completion of the reaction, the organic solvent was concentrated by rotary evaporation to dryness, and the residue was dissolved with ethyl acetate (50 mL), followed by washing with water (50 mL) and saturated brine (50 mL) sequentially. The obtained organic phase was dried over anhydrous sodium sulfate, concentrated under reduced pressure, and the residue was purified by silica gel thin layer chromatography preparative plate (dichloromethane: methanol = 10: 1) to obtain compound **34** (24 mg, yield: 18.9%). LC-MS (ESI): m/z = 472 [M-H]+.

¹H NMR (400 MHz, CD₃OD) *δ*: 8.17 (s, 1H), 7.79-7.77 (d, *J* = 8.0 Hz,1H), 7.67-7.59 (m, 3H), 7.56-7.50 (m, 3H), 7.48-7.37 (m, 4H), 7.32-7.28 (m, 1H), 4.35-4.24 (m, 2H), 4.01-3.98 (d, *J* = 12.4 Hz, 1H), 3.84-3.81 (d, *J* = 12.0 Hz,1H), 1.55 (s, 3H) ppm.

### Example 35

(*S,E*)-2-(3-(3-(benzo[d]oxazol-6-yl)-2-methylstyryl)-4-(trifluoromethyl)benzylamino)-3-hydroxy-2-methylpropanoic acid (compound **35**)

Synthesis route:

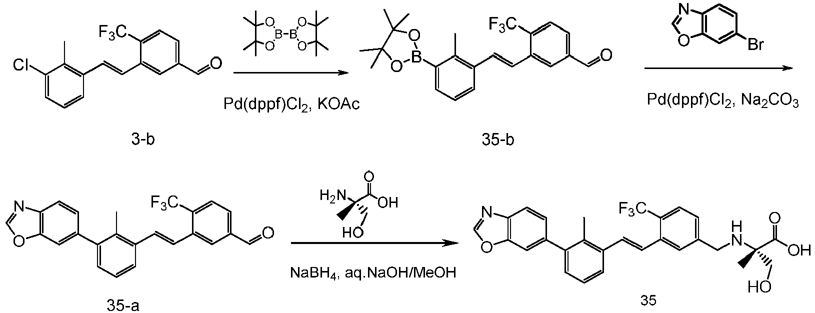

### Synthesis of compound 35-b

To a 100 mL reaction flask were added **3-b** (3.24 g, 10 mmol), bis(pinacolato)diboron (3.05 g, 12 mmol), tris(dibenzylideneacetone)dipalladium (458 mg, 0.5 mmol), 2-dicyclohexylphosphino-2',4',6'-triisopropylbiphenyl (952 mg, 2.0 mmol), potassium acetate (3.00 g, 112 mmol) and 80 mL of toluene. The mixture was stirred at 90 °C for 16 hours under nitrogen. The reaction solution was cooled to room temperature, filtered, and the filtrate was concentrated to dryness on a rotary evaporator. The obtained residue was purified by silica gel column chromatography (petroleum ether: ethyl acetate = 25: 1) to obtain compound **35-b** (3.06 g, yield: 82%). ¹H NMR (500 MHz, CD₃Cl) *δ*: 10.15 (s, 1H), 8.28 (s, 1H), 7.85 (s, 2H), 7.73-7.76 (m, 1H), 7.62 (d, *J* = 7.5 Hz, 1H), 7.50 (d, *J* = 18 Hz, 1H), 7.26-7.28 (m, 1H), 7.23 (d, *J* = 7.5 Hz, 1H), 2.65 (s, 3H), 1.37 (s, 12H) ppm.

### Synthesis of compound 35-a

To a mixture of 6-bromobenzo[d]oxazole (600 mg, 3.0 mmol) and **35-b** (1.00 g, 2.4 mmol) in 1,4-dioxane (20 mL) were added water (3 mL), [1,1'-bis (diphenylphosphino)ferrocene]palladium dichloride (180 mg, 0.24 mmol) and sodium carbonate (636 mg, 6.0 mmol), and the reaction solution was heated to 80 °C and stirred for 16 hours under nitrogen. The reaction solution was concentrated under reduced pressure, and the residue was purified by silica gel column chromatography (petroleum ether: ethyl acetate = 25: 1) to obtain compound **35-a** (700 mg, yield: 72%).

### Synthesis of compound 35

To a 50 mL single-necked bottle were added 3 mL of a solution of (S)-2-methylserine (119 mg, 1 mmol) in water and 2 mL of 1 M aqueous sodium hydroxide solution. After the completion of the addition, to the solution was added 3 mL of solution of **35-a** (194 mg, 0.5 mmol) in tetrahydrofuran, followed by addition of 5 mL of methanol to make the mixture homogeneous. After the reaction solution was stirred at room temperature for 16 hours, the reaction solution was cooled in an ice water bath and sodium borohydride (38 mg, 1 mmol) was added. After completion of the addition, the reaction solution continued to be stirred for 1 hour in ice water bath. The solvent was removed by rotary evaporation under reduced pressure, and the residue was diluted with water and the pH of which was adjusted to 5 to 6 with citric acid. The mixture was filtered, and the solid was collected and dried to obtain a crude product. To the crude product was added 10 mL of ethyl acetate, heated to reflux for several minutes, cooled to room temperature and filtered, and the solid was collected and dried to obtain compound **35** (44 mg, 17%) as a white solid product. LC-MS (ESI): m/z = 511 (M+H)⁺.

¹H NMR (500 MHz, CD₃OD) *δ*: 8.54 (s, 1H), 8.19 (s, 1H), 7.83 (d, *J* = 8.0 Hz, 1H), 7.81 (d, *J* = 8.0 Hz, 1H), 7.69 (d, *J* = 18.0 Hz, 1H), 7.61-7.63 (m, 3H), 7.33-7.41 (m, 3H), 7.27 (d, *J* = 7.5 Hz, 1H), 4.36 (d, *J* = 12.5 Hz, 1H), 4.30 (d, *J* = 12.5 Hz, 1H), 4.02 (d, *J* = 11.5 Hz, 1H), 3.84 (d, *J* = 11.5 Hz, 1H), 2.36 (s, 3H), 1.57 (s, 3H) ppm.

### Example 36

(*S,E*)-2-(3-(3-imidazo[1,2-a]pyridin-6-yl)-2-methylstyryl)-4-(trifluoromethyl)benzylamino)-3-hydroxy-2-methylpropanoic acid (compound **36**)

Synthesis route:

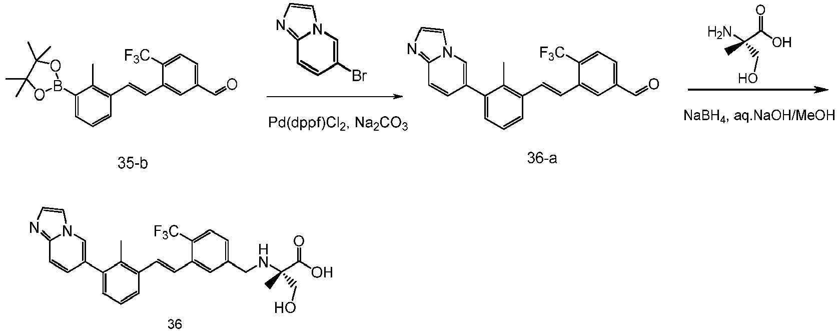

### Synthesis of compound 36-a

To a solution of 6-bromoimidazo[1,2-a]pyridine (197 mg, 1.0 mmol) and **35-b** (416 mg, 1.0 mmol) in 1,4-dioxane (20 mL ) were added water (3 mL), [1,1'-bis(diphenylphosphino)ferrocene]palladium dichloride (73 mg, 0.1 mmol) and sodium carbonate (318 mg, 3.0 mmol) at room temperature. The reaction solution was heated to 80 °C and stirred for 16 hours under nitrogen. The reaction solution was concentrated under reduced pressure, and the residue was purified by silica gel column chromatography (petroleum ether: ethyl acetate = 3: 1) to obtain compound **36-a** (280 mg, yield: 69%).

### Synthesis of compound 36

To 30 mL of a suspension of (*S*)-2-methylserine (238 mg, 2.0 mmol) and **36-a** (203 mg, 0.5 mmol) in methanol was added 6 mL of a solution of sodium hydroxide (80 mg, 2.0 mmol) in water under stirring. The mixture became a clear homogeneous system and was stirred at room temperature for 3 hours, and then cooled to 0 °C. To the mixture was added sodium borohydride (38 mg, 1.0 mmol) at 0 °C, and after the completion of the addition, the reaction solution was warmed to room temperature naturally and continued to be stirred for 2 hours. The methanol was removed by rotary evaporation under reduced pressure, and the residue was diluted with 10 mL of water and the pH of which was adjusted to 7 with citric acid. 10 mL of ethyl acetate was added and the mixture was stirred for 10 minutes. The mixture was filtered, and the solid was collected and dried to obtain the product **36** (68 mg, 26%). LC-MS (ESI): m/z = 510 (M+H)⁺.

¹H NMR (500 MHz, CD₃OD) *δ*: 8.45 (s, 1H), 8.17 (s, 1H), 7.91 (m, 1H), 7.79 (m, 1H), 7.62-7.68 (m, 5H), 7.30-7.38 (m, 4H), 4.29 (d, *J* = 10 Hz, 1H), 4.24 (d, *J* = 10 Hz, 1H), 3.97 (d, *J* = 11 Hz, 1H), 3.83 (d, *J* = 11 Hz, 1H), 2.40 (s, 3H), 1.54 (s, 3H) ppm.

### Example 37

(*S,E*)-2-(3-(3-imidazo[1,2-a]pyrazin-6-yl)-2-methylstyryl)-4-(trifluoromethyl)benzylamino)-3-hydroxyl-2-methylpropanoic acid (compound **37**)

Synthesis route:

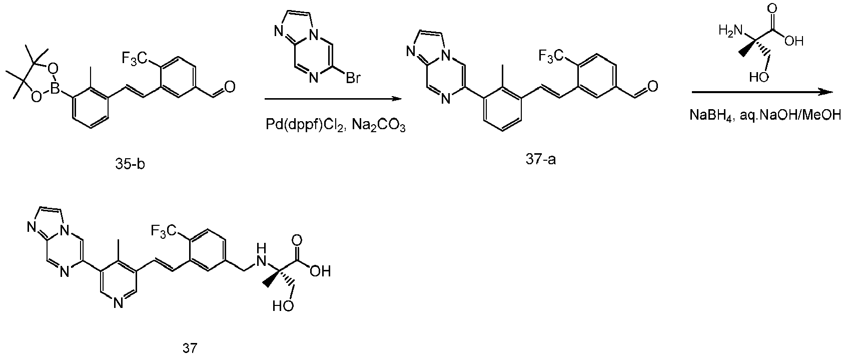

### Synthesis of compound 37-a

To a solution of 6-bromoimidazo[1,2-a]pyridine (198 mg, 1.0 mmol) and **35-b** (416 mg, 1.0 mmol) in 1,4-dioxane (20 mL) were added water (3 mL), [1,1'-bis(diphenylphosphino)ferrocene]palladium dichloride (73 mg, 0.1 mmol) and sodium carbonate (270 mg, 2.5 mmol) at room temperature. The reaction mixture was heated to 80 °C and stirred for 16 hours under nitrogen. The reaction mixture was concentrated under reduced pressure, and the residue was purified by silica gel column chromatography (petroleum ether: ethyl acetate = 6: 1) to obtain compound **37-a** (95 mg, yield: 23%). LC-MS (ESI): m/z = 408 (M+H)⁺.

### Synthesis of compound 37

To 20 mL of a suspension of (*S*)-2-methylserine (119 mg, 1.0 mmol) and 37-a (81 mg, 0.2 mmol) in methanol was added 5 mL of a solution of sodium hydroxide (40 mg, 1.0 mmol) in water under stirring. The mixture became a clear homogeneous system and was stirred at room temperature for 3 hours, and then cooled to 0 °C. To the mixture was added sodium borohydride (19 mg, 0.5 mmol) at 0 °C, and after the completion of the addition, the reaction solution was warmed to room temperature naturally and continued to be stirred for 2 hours. The methanol was removed by rotary evaporation under reduced pressure, and the residue was diluted with 10 mL of water and the pH of which was adjusted to 7 with citric acid. 10 mL of ethyl acetate was added and the mixture was stirred for 10 minutes. The mixture was filtered, and the solid was collected and dried to obtain the product **37** (33 mg, yield: 32%). LC-MS (ESI): m/z = 511 (M+H)⁺.

¹H NMR (500 MHz, CD₃OD) *δ*: 9.10 (s, 1H), 8.64 (s, 1H), 8.20 (s, 1H), 8.15 (s, 1H), 7.91 (s, 1H), 7.80 (d, *J* = 8 Hz, 1H), 7.64-7.71 (m, 3H), 7.34-7.43 (m, 3H), 4.37 (d, *J* = 12 Hz, 1H), 4.31 (d, *J* = 12 Hz, 1H), 4.02 (d, *J* = 12 Hz, 1H), 3.86(d, *J* = 12 Hz, 1H), 2.43 (s, 3H), 1.58 (s, 3H) ppm.

### Example 38

(*S,E*)-2-(3-(3-(benzo[d]isoxazol-6-yl)-2-methylstyryl)-4-(trifluoromethyl)benzylamino)-3-hydroxy-2-methylpropanoic acid (compound **38**)

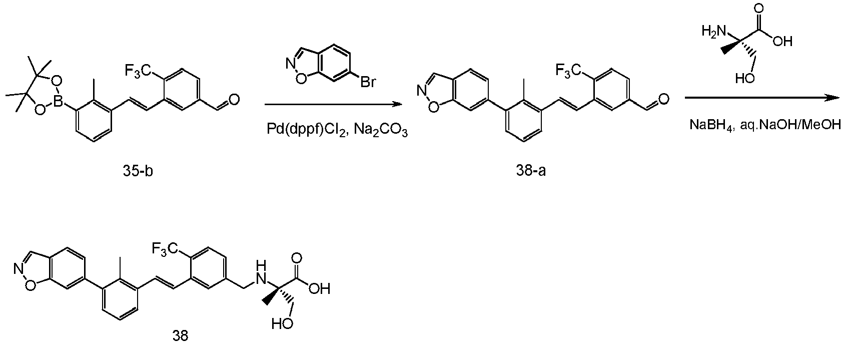

### Synthesis of compound 38-a

To a solution of 6-bromobenzo[d]isoxazole (198 mg, 1.0 mmol) and **35-b** (416 mg, 1.0 mmol) in 1,4-dioxane (20 mL) were added water (3 mL), [1,1'-bis(diphenylphosphino)ferrocene]palladium dichloride (73 mg, 0.1 mmol) and sodium carbonate (270 mg, 2.5 mmol) at room temperature. The reaction mixture was heated to 80 °C and stirred for 16 hours under nitrogen. The reaction mixture was concentrated under reduced pressure, and the residue was purified by silica gel column chromatography (petroleum ether: ethyl acetate = 6: 1) to obtain compound **37-a** (86 mg, yield: 21%). LC-MS (ESI): m/z = 408 (M+H)⁺.

### Synthesis of compound 38

To 20 mL of a suspension of (S)-2-methylserine (48 mg, 0.4 mmol) and **38-a** (82 mg, 0.2 mmol) in methanol was added 4 mL of a solution of sodium hydroxide (20 mg, 0.5 mmol) in water under stirring. The mixture became a clear homogeneous system and was stirred at room temperature for 3 hours, and then cooled to 0 °C. To the mixture was added sodium borohydride (23 mg, 0.6 mmol) at 0 °C, and after the completion of the addition, the reaction solution was warmed to room temperature naturally and continued to be stirred for 2 hours. The methanol was removed by rotary evaporation under reduced pressure, and the residue was diluted with 10 mL of water and the pH of which was adjusted to 5 to 6 with citric acid. 10 mL of ethyl acetate was added and the mixture was stirred for 10 minutes. The mixture was filtered, and the solid was collected and dried to obtain the product **38** (12 mg, yield: 12%). LC-MS (ESI): m/z = 511 (M+H)⁺.

¹H NMR (500 MHz, CD₃OD) *δ*: 8.17 (s, 1H), 7.80 (d, *J* = 8 Hz, 1H), 7.59-7.87 (m, 5H), 7.31-7.37 (m, 2H), 7.19 (d, *J* = 7.5 Hz, 1H), 6.89-6.91 (m, 2H), 4.33 (d, *J* = 12 Hz, 1H), 4.27 (d, *J* = 12 Hz, 1H), 4.00 (d, *J* = 12 Hz, 1H), 3.84 (d, *J* = 12 Hz, 1H), 2.34 (s, 3H), 1.56 (s, 3H) ppm.

### Example 39

(*S,E*)-3-hydroxy-2-methyl-2-(3-(2-methyl-3-(pyridin-2-yl)styryl)-4-(trifluoromethyl)benzylamino)propanoic acid (compound **39**)

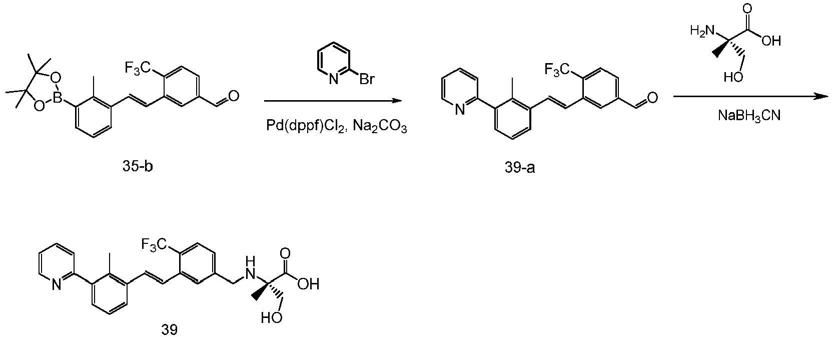

### Synthesis of compound 39-a

2-Bromopyridine (158 mg, 1.00 mmol) and **35-b** (500.0 mg, 1.20 mmol) were dissolved in a mixture of 1,4-dioxane and water (20 mL, the volume ratio of 1,4-dioxane to water was 20: 1), and [1,1'-bis(diphenylphosphino)ferrocene] palladium dichloride (86.5 mg, 0.10 mmol) and sodium carbonate (814.6 mg, 2.50 mmol) were added. The reaction solution was heated to 80 °C and stirred overnight under nitrogen. After the completion of the reaction, the reaction solution was diluted with ethyl acetate, washed with water three times and saturated brine once. The obtained organic phase was dried over anhydrous sodium sulfate, and concentrated by rotary evaporation to dryness to obtain a crude compound. The crude compound was purified by preparative plate (PE/EA = 5: 1) to obtain the target compound **39-a** (90 mg, yield: 24%).

### Synthesis of compound 39

To a mixed solution of **39-a** (105 mg, 0.29 mmol) and (*S*)-2-methylserine (68 mg, 0.57 mmol) in methanol and dichloromethane (10 mL, the volume ratio of methanol to dichloromethane was 1: 1) was added acetic acid (34 mg, 0.57 mmol), and the reaction solution was stirred at room temperature for 1 hour. Then, to the reaction solution was added sodium cyanoborohydride (90 mg, 1.43 mmol) and was stirred overnight. After the completion of the reaction, the solvent was concentrated by rotary evaporation to dryness to obtaina crude compound. The crude compound was purified by preparative plate (DCM: MeOH = 15: 1) to obtain the target compound **39** (34 mg, yield: 25%). LC-MS (ESI): m/z = 471.0 [M+H]⁺.

¹H NMR (500 MHz, CD₃OD) *δ*: 8.64-8.63 (d, *J* = 5.0 Hz, 1H), 8.19 (s, 1H), 7.99-7.96 (m, 1H), 7.82-7.80 (d, *J* = 7.5 Hz, 1H), 7.70-7.64 (m, 3H), 7.54 (d, *J* = 7.0 Hz, 1H), 7.48-7.46 (m, 1H), 7.40-7.32 (m, 3H), 4.37(d, *J* = 12 Hz, 1H), 4.29(d, *J* = 12 Hz, 1H), 4.02 (d, *J* = 12 Hz, 1H), 3.85(d, *J* = 12 Hz, 1H), 2.36 (s, 3H), 1.57 (s, 3H) ppm.

### Example 40

(*S,E*)-3-hydroxy-2-methyl-2-(3-(2-methyl-3-(pyridin-3-yl)styryl)-4-(trifluoromethyl)benzylamino)propanoic acid (conpound **40**)

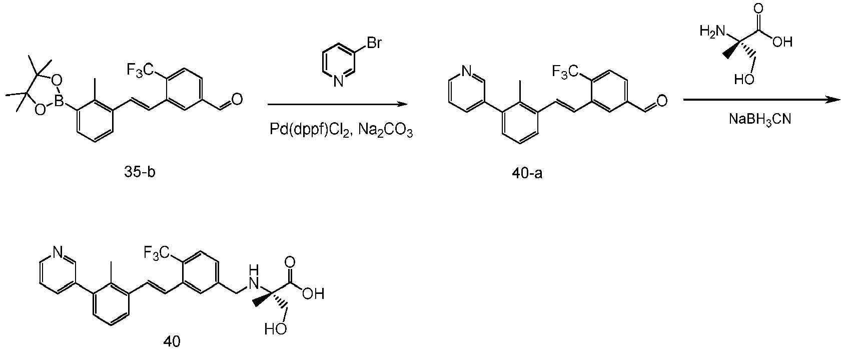

### Synthesis of compound 40-a

To a mixed solution of 3-bromopyridine (63.2 mg, 0.40 mmol) and **35-b** (200.0 mg, 0.48 mmol) in 1,4-dioxane and water (20 mL, the volume ratio of 1,4-dioxane to water was 20: 1) were added [1,1'-bis(diphenylphosphino)ferrocene] palladium dichloride (34.6 mg, 0.04 mmol) and sodium carbonate (106 mg, 1.0 mmol) at normal temperature. The reaction solution was heated to 80 °C and stirred overnight under nitrogen. After the completion of the reaction, the reaction solution was diluted with ethyl acetate, and washed with water three times and saturated brine once. The obtained organic phase was dried over anhydrous sodium sulfate, and concentrated by rotary evaporation to dryness to obtain a crude compound. The crude compound was purified by preparative plate (PE/EA = 10: 1 to 5: 1) to obtain the target compound **40-a** (57 mg, yield: 39%). LC-MS (ESI): m/z = 368.0 [M+H]⁺.

### Synthesis of compound 40

To a mixed solution of **40-a** (57 mg, 0.155 mmol) and (*S*)-2-methylserine (37 mg, 0.31 mmol) in methanol and dichloromethane (10 mL, the volume ratio of methanol to dichloromethane was 1: 1) was added acetic acid (19 mg, 0.31 mmol), and the reaction solution was stirred at room temperature for 1 hour. Then, to the reaction solution was added sodium cyanoborohydride (49 mg, 0.78 mmol) and was stirred overnight. After the completion of the reaction, the solvent was concentrated by rotary evaporation to dryness to obtain the crude compound. The crude compound was purified by preparative plate (DCM: MeOH = 15: 1) to obtain the target compound **40** (19 mg, yield: 26%). LC-MS (ESI): m/z = 471.0 [M-H]+.

¹H NMR (500 MHz, CD₃OD) *δ*: 8.58 (dd, *J* = 2.0 Hz, *J* = 5.5 Hz, 1H), 8.54 (s, 1H), 8.19 (s, 1H), 7.86 (d, *J* = 8.0 Hz, 1H), 7.81(d, *J* = 7.5 Hz, 1H ), 7.69-7.64 (m, 3H), 7.58-7.55 (m, 1H), 7.39-7.36 (m, 2H), 7.25 (d, *J* = 12 Hz, 1H), 4.37 (d, *J* = 12 Hz, 1H), 4.29 (d, *J* = 12 Hz, 1H), 4.03 (d, *J* = 12 Hz, 1H), 3.85 (d, *J* = 12 Hz, 1H), 2.36 (s, 3H), 1.57 (s, 3H) ppm.

### Example 41

(*S,E*)-3-hydroxy-2-methyl-2-(3-(2-methyl-3-(pyrazin-2-yl)styryl)-4-(trifluoromethyl)benzylamino)propanoic acid (compound **41**)

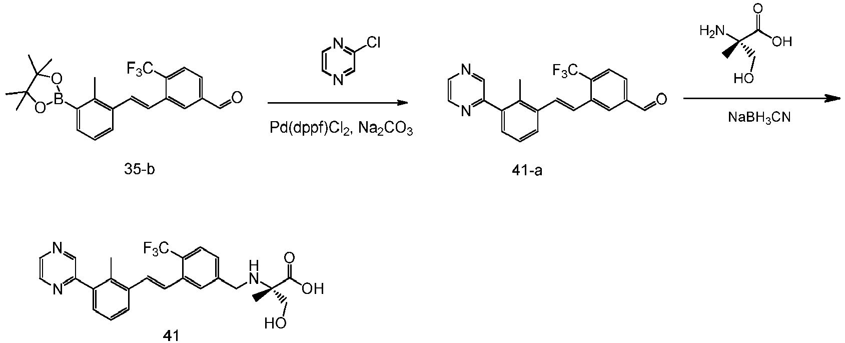

### Synthesis of compound 41-a

To a solution of 2-chloropyrazine (180 mg, 1.57 mmol) and **35-b** (985 mg, 2.37 mmol) in 1,4-dioxane (5 mL) were added water (2 mL), anhydrous sodium carbonate (500 mg, 4.71 mmol) and 1,1'-bisdiphenylphosphinoferrocene palladium dichloride (87 mg, 0.11 mmol), and the reaction solution was heated to 80 °C overnight under nitrogen. The reaction solution was concentrated under reduced pressure, and the residue was purified by silica gel column chromatography (petroleum ether: ethyl acetate = 3: 1) to obtain **41-a** (75 mg, yield: 13%) as a canary solid. LC-MS (ESI): m/z = 369 [M+H]⁺.

### Synthesis of compound 41

To a mixed solution of **41-a** (75 mg, 0.21 mmol) in dichloromethane (5 mL) and methanol (5 mL) were added (*S*)-2-methylserine (49 mg, 0.41 mmol) and acetic acid (1 drop). After the mixture was stirred at room temperature for 3 hours, sodium cyanoborohydride (20 mg, 0.32 mmol) was added. The reaction solution was stirred overnight at room temperature, concentrated to dryness, and purified by silica gel thin layer chromatography plate (dichloromethane: methanol = 10: 1) to obtain **41** (46 mg, yield: 40%) as an off-white solid. LC-MS (ESI): m/z = 572 [M+H]⁺.

¹H-NMR (500MHz, MeOD) *δ*: 8.78 (d, *J* = 1 Hz, 1H), 8.73 (d, *J* = 3 Hz, 1H), 8.64 (d, *J* = 3 Hz, 1H), 8.19 (s, 1H), 7.65-7.82 (m, 4H), 7.37-7.43 (m, 3H), 4.33 (m, 2H), 4.01 (d, *J* = 12 Hz, 1H), 3.85 (d, *J* = 12 Hz, 1H), 2.42 (s, 3H), 1.58 (s, 3H) ppm.

### Example 42

(*S,E*)-3-hydroxy-2-methyl-2-(3-(2-methyl-3-(2-methyl-2H-indazol-6-yl)-styryl)-4-(trifluoromethyl)benzylamino)propanoic acid (compound **42**)

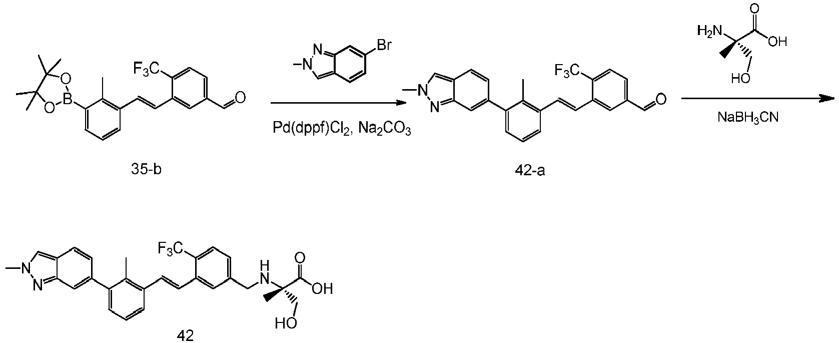

### Synthesis of compound 42-a

6-Bromo-2-methyl-2*H*-indazole (210 mg, 1.0 mmol) and **35-b** (560 mg, 1.35 mmol) were dissolved in 1,4-dioxane (5 mL), and water (2 mL), anhydrous sodium carbonate (191 mg, 3.0 mmol) and 1,1'-bisdiphenylphosphinoferrocene palladium dichloride (100 mg, 0.1 mmol) were added, and the reaction solution was heated to 80 °C and reacted overnight under nitrogen. The reaction solution was concentrated under reduced pressure, and the residue was purified by silica gel column chromatography (petroleum ether: ethyl acetate = 10: 1) to obtain **42-a** (150 mg, yield: 36%) as a canary solid.

### Synthesis of compound 42

To a mixed solution of **42-a** (150 mg, 0.36 mmol) dissolved in dichloromethane (5 mL) and methanol (5 mL) were added (*S*)-2-methylserine (85 mg, 0.72 mmol) and acetic acid (1 drop). After the mixture was stirred at room temperature for 3 hours, sodium cyanoborohydride (67 mg, 1.08 mmol) was added. The reaction solution was stirred overnight at room temperature, concentrated to dryness, and purified by silica gel thin layer chromatography plate (dichloromethane: methanol = 10: 1) to obtain **42** (56 mg, yield: 30%) as an off-white solid. LC-MS (ESI): m/z = 524 [M+H]⁺.

¹H-NMR (500 MHz, MeOD) δ: 8.26 (s, 1H), 8.18 (s, 1H), 7.58-7.81 (m, 5H), 7.38 (s, 1H), 7.26-7.35 (m, 3H), 7.06 (d, *J* = 9 Hz, 1H), 4.28 (m, 5H), 3.99 (d, *J* = 12 Hz, 1H), 3.83 (d, *J* = 12 Hz, 1H), 2.37 (s, 3H), 1.54 (s, 3H) ppm.

### Example 43

(*S,E*)-3-hydroxy-2-methyl-2-(3-(2-methyl-3-(2-methylbenzo[d]oxazol-6-yl)-styryl)-4-(trifluoromethyl)benzylamino)-3-hydroxy-2-methylpropanoic acid (compound **43**)

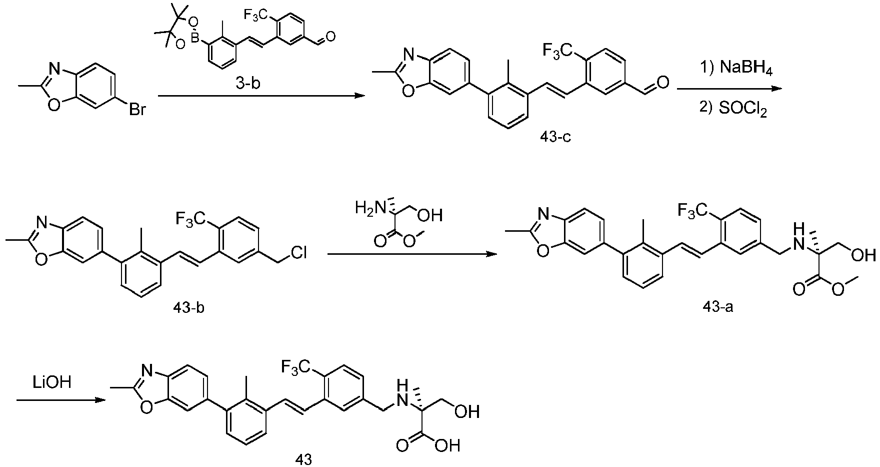

### Synthesis of compound 43-c

To a solution of 6-bromo-2-methyl-benzo[d]-oxazole (400 mg, 1.89 mmol) and 3-b (1104 mg, 2.65 mmol) dissolved in 1,4-dioxane (15 mL) were added water (3 mL), anhydrous sodium carbonate (600 mg, 5.66 mmol) and 1,1'-bisdiphenylphosphinoferrocene palladium dichloride (164 mg, 0.21 mmol), and the reaction solution was heated to 80 °C overnight under nitrogen. The reaction solution was concentrated under reduced pressure and purified by silica gel column chromatography (petroleum ether: ethyl acetate = 5: 1) to obtain **43-c** (510 mg, yield: 64%) as a canary solid.

### Synthesis of compound 43-b

**43-c** (510 mg, 1.21 mmol) was dissolved in ethanol (10 mL), and sodium borohydride (92 mg, 2.42 mmol) was added. After the reaction solution was stirred at room temperature for 2 hours, the reaction solution was concentrated to dryness, and the residue was separated with ethyl acetate (50 mL) and water (50 mL). The organic phase was separated and dried, filtered, concentrated to dryness and redissolved in dichloromethane (20 mL). DMF (1 drop) and sulfoxide chloride (3 mL) were added and the mixture was stirred at room temperature for 2 hours. The residue was concentrated to dryness to obtain **43-b,** which was directly used in the next reaction.

### Synthesis of compound 43-a

**43-b** (360 mg, 0.82 mmol) and L-2-methylserine methyl ester hydrochloride (127 mg, 0.82 mmol) were dissolved in acetonitrile (10 mL), and potassium carbonate (339 mg, 2.46 mmol) and sodium iodide ( 125 mg, 0.82 mmol) were added. The mixture was heated to 85 °C for 8 hours and concentrated to dryness, and the residue was separated with ethyl acetate (50 mL) and water (50 mL). The organic phase was separated, dried over anhydrous sodium sulfate, filtered, concentrated to dryness and purified by a column (petroleum ether: ethyl acetate = 1: 1) to obtain **43-a** (150 mg, yield: 34%) as a canary oil by column chromatography. LC-MS (ESI): m/z = 539 [M+H]⁺.

### Synthesis of compound 43

To a mixed solution of **43-a** (150 mg, 0.28 mmol) in methanol (2 mL) and water (2 mL) was added lithium hydroxide (18 mg, 0.42 mmol). The reaction solution was stirred overnight at room temperature and concentrated to dryness, and the residue was diluted with water. After acidification, the pH was 3 to 4. The mixture was filtered, washed with water, and dried to obtain **43** (67 mg, yield: 46%) as a canary solid. LC-MS (ESI): m/z = 525 [M+H]⁺.

¹H-NMR (500MHz, MeOD) *δ*: 8.18 (s, 1H), 7.53-7.81 (m, 6H), 7.25-7.38 (m, 4H), 4.32 (m, 2H), 4.00 (m, 1H), 3.84 (d, *J* = 12 Hz, 1H), 2.69 (s, 3H), 2.35 (s, 3H), 1.53 (s, 3H) ppm.

### Example 44

(*S,E*)-3-hydroxy-2-methyl-2-(3-(2-(2',3',4',5',6'-pentdeuterated-2-methylbiphenyl-3-yl)vinyl)-4-(trifluoromethyl)benzylamino)-propionic acid (compound **44**)

Synthesis route:

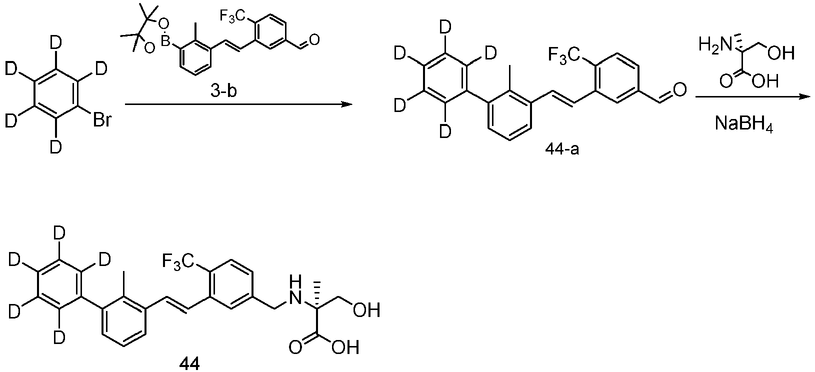

### Synthesis of compound 44-a

To a mixed solution of pentadeuterated bromobenzene (162 mg, 1.0 mmol) 3-b (416 mg, 1.0 mmol) in ethylene glycol dimethyl ether(15 mL) were added [1,1'-bis (diphenylphosphino)ferrocene]palladium chloride (73 mg,0.1 mmol), potassium phosphate (424 mg,2.0 mmol) and potassium fluoride (116 mg, 2.0 mmol). The reaction solution was heated to 80°C in nitrogen, and stirred for 6 hours. Then the reaction solution was concentrated under reduced pressure, and the residue was purified by silica gel column chromatography (petroleum ether : ethyl acetate=100 : 1) to obtain compound **44-a** (190 mg, yield : 50 %).

### Synthesis of compound 44

3 mL solution of (*S*)-2-methylserine (119 mg, 1 mmol) in water and 2 mL 1M sodium hydroxide solution were added to a 50 mL flask. Then, 4 mL solution of 44-a (185 mg, 0.5 mmol) in tetrahydrofuran was added to the mixture and 10 mL methanol was added to adjust the mixture to be homogeneous. After the mixture was stirred at room temperature for 16 hours, sodium borohydride (38 mg, 1 mmol) was added to the mixture under icewater bath. Then, the mixture was stirred for 1 hour under icewater bath. After that, the solvent was removed by rotary evaporation under reduced pressure, and the residue was diluted with water and the pH was adjusted to 5-6 with citric acid. The residue was filtered, the solid was collected and dried to get the crude product. 10 mL ethyl acetate was added to the crude product, and the mixture was refluxed for minutes, the mixture was cooled to room temperature and filtered, the solid was collected and dried to obtain white solid product **44** (50 mg, yield : 20 %). LC-MS (ESI): m/z = 475(M+H)⁺.

¹H NMR (500 MHz, CD₃OD) *δ*: 8.16 (s, 1H), 7.77 (d, *J*=8.0Hz, 1H), 7.64 (d, *J*=18.0Hz, 1H), 7.61(d, *J*=8.0Hz, 1H), 7.54 (d, *J*=7.5 Hz, 1H), 7.31-7.35 (m, 1H) , 7.28(t, *J*=8.0Hz, 1H), 7.17(d, *J*=8.0Hz, 1H), J=4.33 (d, *J*=13Hz, 1H), 4.27 (d, *J*=13Hz, 1H), 3.99 (d, *J*=12Hz, 1H), 3.82 (d, *J*=12Hz, 1H), 2.32(s, 3H), 1.55(s, 3H) ppm.

### Effect Example 1

Homogenouse Time-Resolved Fluorescence (HTRF) binding assay was used to determine the binding activity of the compound of the present disclosure to PD-1/PD-L1.

The purchased kit (CisBio, #64CUS000C-1) contained the reagents required for experiments such as PD-1, PD-L1, anti-tagl-Eu, Anti-tag2-XL665, Dilute Buffer and Detection Buffer.

### Experimental procedure

1. The compound was formulated to 10 concentrations with a three-fold concentration gradient with 100% DMSO.

2. The solution of the compound in DMSO was added to Dilute Buffer, and mixed evenly and then transfered to a 96-well plate.

3. PD-L1 was diluted with Dilute Buffer and added to the 96-well plate above.

4. PD-1 was diluted with Dilute Buffer, then added to the 96-well plate above and incubated at room temperature for 30 minutes.

5. One portion of anti-tag1-Eu and one portion of anti-tag2-XL665 were added to Detection Buffer, mixed evenly and transfered to the 96-well plate above.

6. The mixed solution in the 96-well plate were incubated at room temperature for 1 to 24 hours.

7. HTRF values were read by Envision.

### Experimental results

The biological activity of the compound of the present disclosure was determined by the above assay, and the measured results were as followed (Table 1).

**Table 1: IC₅₀ values of some compounds of the present disclosure binding to PD-1/PD-L1**

| Compound | IC₅₀ (µM) | Conpound | IC₅₀ (µM) |
|---|---|---|---|
| **1** | 0.014 | **2** | 3.1 |
| **3** | 0.046 | **5** | 0.023 |
| **6** | 0.32 | **7** | 1.4 |
| **8** | 0.042 | **9** | 0.057 |
| **12** | 0.024 | **13** | 0.013 |
| **14** | 0.019 | **15** | 0.014 |
| **16** | 0.013 | **17** | 0.020 |
| **18** | 0.046 | **19** | 0.061 |
| **20** | 0.018 | **21** | 0.085 |
| **22** | 0.044 | **23** | 0.026 |
| **24** | 0.430 | **25** | 0.066 |
| **26** | 0.020 | **27** | 0.018 |
| **28** | 0.029 | **29** | 0.019 |
| **30** | 0.027 | **31** | 0.041 |
| **32** | 0.024 | **33** | 0.230 |
| **34** | 0.180 | **35** | 1.1 |
| **36** | >10 | **37** | 1.3 |
| **38** | >10 | **39** | 2.2 |
| **40** | 0.580 | **41** | 0.540 |
| **42** | 0.880 | **43** | 0.540 |

### Effect example 2: Pharmacokinetic experiment in mice

Glipizide (molecular formula was C₂₁H₂₇N₅O₄S, molecular weight was 445.5 g/mol, Analytical Reagent) purchased from Sigma-Aldrich (U.S.A.), was used as the internal standard for analysis. Methanol, acetonitrile and formic acid (HPLC grade) were purchased from Sigma-Aldrich (U.S.A.), and pure water was purchased from Hangzhou Wahaha Group Co., Ltd. (Hangzhou, China). Other chemical reagents were all analytical reagents.

CD1 male mice, six per group, 6-7 weeks old, 29-31 g, were purchased from LC Laboratory Animal Co. LTD. Before the experiment, the animals should be kept for at least 3 days to adapt to the environment. Throughout the experiment, the animals were required to fast overnight, and allowed to drink water freely during the period, and the surviving animals resumed feeding 4 hours after administration.

### Experimental procedure

1. The compound was formulated into a solution with a concentration of 0.4 mg/mL with 10% DMSO, 10% Solutol HS 15 and 80% Saline.

2. The above prepared solution was administered to 3 mice by tail vein injection (compound dosage was 2 mg/kg), and was administered to another 3 mice by oral gavage (compound dosage was 10 mg/kg) simultaneously.

3. Approximately 30 µL of blood was collected from each of the above 6 mice at 5 minutes, 15 minutes, 30 minutes, 1 hour, 2 hours, 4 hours, 8 hours and 24 hours after administration into the EDTA-K2 anticoagulation tube (set on wet ice), and 20 µL of blood was immediately diluted with 60 µL of water, and then put in the refrigerator at -70 °C for long-term storage until the sample was analyzed.

4. 20 µL of the above solution was transfered into a 96-well deep well sample plate, and 150 µL of glipizide internal standard acetonitrile solution (the concentration was 100 ng/mL) was added, vortexed for 10 min, and centrifuged at 58 rpm for 10 min. 1 µL of the supernatant was taken for LCMSMS analysis.

5. Based on the drug concentration-time data, WinNonlin 6.4 software (USA) was used to calculate the pharmacokinetic parameters of the compound according to the non-compartment model.

### Experimental results

The results of the pharmacokinetics of the compounds of the present disclosure in mice were as followed (Table 2):

**Table 2 Oral bioavailability of the pharmacokinetics of some compounds of the present disclosure in mice**

| Compound | Oral bioavailability |
|---|---|
| **13** | 16.1% |
| **27** | 52.3% |
| **29** | 38.2% |
| 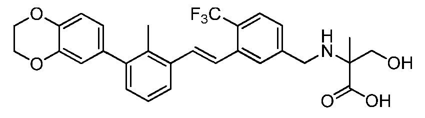 F3C O H N OH O O OH | 5.98% |
| 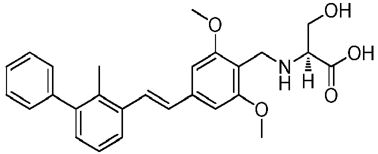 | 3.08% |
| 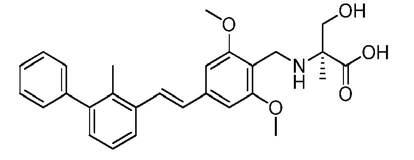 | 3.89% |
| 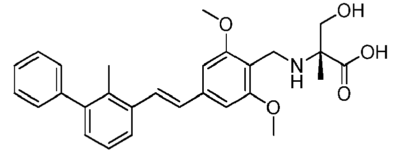 | 4.42% |

### Effect example 3:

*In vivo* pharmacodynamics study of PD-L1 antibody Durvalumab and compound 29 used singly or in combination in hPD-1 knock-in mouse colon cancer MC-38 - hPD-L1 model

### 1. Experimental materials

**Antibody:** PD-L1 antibody Imfinzi (Durvalumab), 120 mg/2.4 mL (50 mg/mL), Lot: 041E17C. Manufacturer: AstraZeneca, purchased from Hong Kong Mingchuang Pharmacy Co., Ltd., stored at 2-8°C.

**Experimental animals:** Human PD-1 transgenic mice, strain: C57BL/6-hPD-1 mice, 6-8 weeks, weighing 18-21 grams, female, 120 mice, purchased from GemPharmatech Co., Ltd..

**Preparation materials:** Cremophor RH40 (CAS: 61788-85-0, Lot: 29761847G0, supplier: Shanghai Xietai Chem Co., Ltd.); SBE-β-CD (CAS: 128446-35-5, Lot: R1804474, supplier: Shanghai Shaoyuan Co., Ltd.); DMEM (CAS: 11995-065, Lot: 2025378, supplier: Gibco); Penicillin-streptomycin (CAS: 15140-122, Lot: 1953101, supplier: Hyclone); Fetal Bovine Serum ( CAS: 10099-141, Lot: 1966174C, supplier: Gibco); hygromycinB (CAS: 1c0687010, Lot: HY069-L12, supplier: Invitrogen).

### 2. Establishment of tumor model

Human PD-L1 gene was knocked into MC-38 cells(MC-38-hPD-Ll cells), MC-38 cells was adherent cultured *in vitro* in DMEM medium with 10% heat-inactivated fetal bovine serum and hygromycinB (final concentration 100 µL/mL) at 37°C under 5%CO₂. Passage cultivation was conducted three times a week. The cells were collected and counted at the exponential growth phase. 100 µL 1×106 MC-38-hPD-Ll cells suspension was inoculated into the right dorsal subcutaneous of C57BL / 6-hPD-1 mice. 6 days after inoculation, 60 tumor-bearing mice with transplanted tumor volume ranging from 31.49 mm³⁻110.26 mm³ were selected, the mice were randomly divided into 6 groups, with 10 in each group, according to the experimental plan, and administration was started on the same day.

### 3. Preparation of test article

**Preparation of vehicle:** 800mL sterile water was measured and added to volumertic flask. Magnetic stirring was carried out to produce vortex on the liquid surface. An aqueous solution of 10% (w/v) Cremophor RH40 + 20% (w/v) SBE-*β-*CD was obtained by slow addition of 100g Cremophor RH40, followed by 200g SBE-β-CD, and water to fix the solution volume to 1000mL with fully stirring.

**Preparation of Compound 29 suspension:** 150.92mg compound 29 was weighed and 12.5mL150.92 mg 10% (w/v) Cremophor RH40 + 20% (w/v) SBE-β-CD (hereinafter referred to as vehicle) aqueous solution was added. A suspension was with a concentration of 12.0 mg/mL was obtained by fully mixing with vortexing for 2 minutes and ultrasonic treatment for 30 minutes. 5.0 mL of the suspension of compound 29 with a concentration of 12.0 mg/mL was pipetted, and 5.0 mL of vehicle was added to the suspension. The suspension with a concentration of 6.0 mg/mL was obtained by fully mixing with vortexing for 1 minute and ultrasonic treatment for 5 minutes. 2.0 mL of the suspension of compound 29 with a concentration of 12.0 mg/mL was pipetted, and 6.0 mL of vehicle was added to the suspension. The suspension with a concentration of 3.0 mg/mL was obtained by fully mixing with vortexing for 1 minute and ultrasonic treatment for 5 minutes. The suspension of compound 29 was prepared once a day.

**Preparation of PD-L1 antibody:** 0.12 mL of Durvalumab stock solution (concentration: 50 mg/mL) was pipetted and divided into 6 portions in 5mL sterile centrifuge tube, with 6.0 mg in each portion, then the portions were stored in a 4°C refrigerator for late use. Before administration, 0.12 mL of stock solution (concentration: 50 mg/mL) was pipetted, 2.88 mL of 0.9% sodium chloride solution was added to the stock solution. 3 mL of the solution of Durvalumab with a final concentration 2 mg/mL was obtained by shaking slightly and fully vortexing.

### 4. Experimental procedure

1) The mice in the vehicle control group were weighed and recorded in an electronic balance according to the number. The mice in the vehicle control group were adminitered by oral gavage with vehicle according to the weight twice a day with a capacity of O.lmL/lOg. The time for administration was about 20s for each mouse, and about 5 minutes in total for each group of 10 mice.
2) The mice in the compound 29(30 mg/kg) group were weighed and recorded in an electronic balance according to the number. The mice in this group were adminitered by oral gavage with the prepared Compound 29 suspension according to the weight twice a day with a dosage of 30mg/kg and a capacity of O.lmL/lOg. The time for administration was about 20s for each mouse, and about 5 minutes in total for each group of 10 mice.
3) The mice in the compound 29 (60 mg/kg) group were weighed and recorded in an electronic balance according to the number. The mice in this group were adminitered by oral gavage with the prepared Compound 29 suspension according to the weight twice a day with a dosage of 60mg/kg and a capacity of O.lmL/lOg. The time for administration was about 20s for each mouse, and about 5 minutes in total for each group of 10 mice.
4) The mice in the compound 29 (120 mg/kg) group were weighed and recorded in an electronic balance according to the number. The mice in this group were adminitered by oral gavage with the prepared Compound 29 suspension according to the weight twice a day with a dosage of 120mg/kg and a capacity of O.lmL/lOg. The time for administration was about 20s for each mouse, and about 5 minutes in total for each group of 10 mice.
5) The mice in the combined administration group (compound 29: 60 mg/kg; Durvalumab: 20 mg/kg) were weighed and recorded in an electronic balance according to the number. The mice were adminitered by oral gavage with the prepared Compound 29 suspension according to the weight twice a day with a dosage of 60 mg/kg and a capacity of O.lmL/lOg. The time for administration was about 20s for each mouse, and about 5 minutes in total for each group of 10 mice.
6) The mice in Durvalumab (20 mg/kg) group were weighed and recorded in an electronic balance according to the number. The mice were adminitered by intraperitoneal injection with the prepared Durvalumab according to the weight twice a day with a dosage of 20mg/kg and a capacity of 0.1mL/10g, the intraperitoneal injection time was about 20s for each mouse, total about 5 minutes for a group of 10 mice.
7) The mice in the combined administration group (compound 29: 60 mg/kg; Durvalumab: 20 mg/kg) mice were weighed and recorded in an electronic balance according to the number. The mice were adminitered by intraperitoneal injection with the prepared Durvalumab according to the weight twice a day with a dosage of 20mg/kg and a capacity of O.lmL/lOg. The time for administration was about 20s for each mouse, and about 5 minutes in total for a group of 10 mice.

### 5. Data collection and experimental endpoints

1) The tumor was measured by digital vernier caliper and the tumor volume was calculated three times a week. Euthanasia was imposed if the size of the tumor volume excessed 2000 mm³, or when the animal suffered from serious illness, pain, or was unable to eat and drink freely. The experiment ended if the avarge size of tumor of the control group reached 2000 mm³.
2) The animal was weighed by electronic balance every day. Euthanasia was imposed when the animal was obviously wasting and had weight loss of more than 20%.
3) The experiment ended 19 days after administration of compound 29.

### 6. Data analysis and results

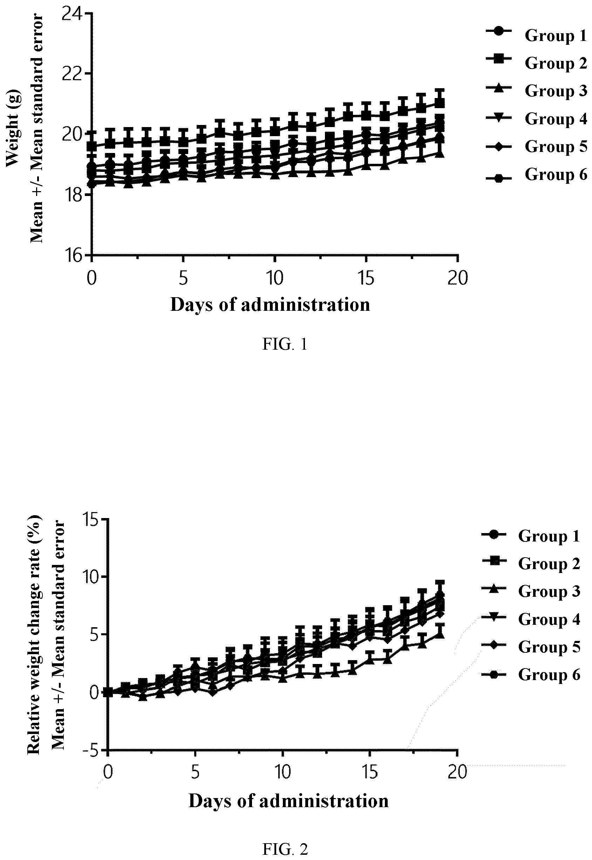

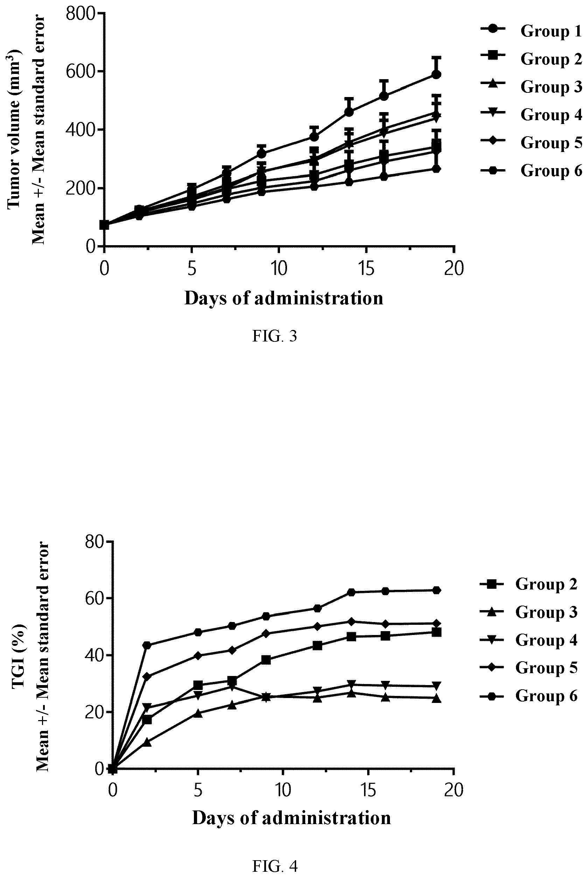

Fig. 1 is a curve graphically depicting the weight change of mice in each group, plotted by the average weight of mice in each group measured daily during the experiment. Fig.2 is a curve graphically depicting the weight change rate of mice in each group, plotted by the average weight change rate of mice in each group measured daily during the experiment. Fig.3 is a curve graphically depicting the tumor volume change of mice in each group, plotted by the average tumor volume of mice in each group measured during the experiment. Fig.4 is a curve graphically depicting the tumor inhibition rate change of mice in each group, plotted by the average tumor inhibition rat of mice in each group measured during the experiment. The calculation equation of tumor inhibition rate is TGI(%)=(1-(tumor volume of administration goup on administration day- tumor volume of administration goup on the first day)/(tumor volume of blank goup on administration day- tumor volume of blank goup on the first day))^{∗}100%

Figures were drawn by GraphPad Prism 5.0, tumor volme change of mice was analyzed by Two-way ANOVA, and was compared with vehicle control group according to Bonferroni posttests method, P<0.05 which showed significant difference. Experimental results were showed in table 3 as followed.

**Table3: In vivo Pharmacodynamic results of single or combined medication in hPD-1 knock-in mouse colon cancer MC-38 - hPD-Ll model**

| | **Test Article** | **Tumor Volume (mm³)^{a}** | **TGI (%)** | ***P* Value^{b}** |
|---|---|---|---|---|
| Group 1 | Vehicle Control | 588.68 ± 58.56 /D19 | -- | -- |
| Group2 | Durvalumab, 20mg/kg, IP, BIW | 341.19 ± 56.59 /D19 | 48.17 /D19 | <0.0001 |
| Group3 | Compound 29, 30mg/kg, PO, BID | 459.82 ± 58.24 /D19 | 24.95 /D19 | <0.0001 |
| Group4 | Compound 29, 60mg/kg, PO, BID | 439.04 ± 51.23 /D19 | 29.06 /D19 | <0.0001 |
| Group5 | Compound 29, 120mg/kg, PO, BID | 325.23 ± 38.59 /D19 | 51.21 /D19 | <0.0001 |
| Group6 | Compound 29, 60mg/kg, PO, BID + Durvalumab (anti-hPD-Ll), 20mg/kg, IP, BIW | 265.55 ± 64.11 /D19 | 62.82 /D19 | <0.0001 |

Note: a. Mean ± SEM.

b. P value calculated by tumor volume (compared with vehicle control group).

Experimental results: Until the 19^{th} day of administration, compared with vehicle control group, the tumor inhibition rate of group 2 (PD-L1 antibody Durvalumab) is 48.17%, the max tumor inhibition rate of Compound 29 is 51.21%, and the tumor inhibition rate of combined administration group is 62.82%. Consequently, it is concluded that the combined administration group has a superior inhibitory effect on colon cancer (MC-38-hPD-L1) cells compared to that of single component compound 29 or PD-L1 antibody, which improves the tumor inhibition rate of mice and has a statistical significance.

Although the embodiments of the present disclosure are described above, those skilled in the art should understand that, these are only examples, and various changes or modifications can be made to these emboidments without departing from the principle and essence of the present disclosure. Therefore, the protection scope of the present disclosure is restricted by the appended claims.

## Claims

1. A pharmaceutical composition comprising:
a PD-L1 antibody, and
a small-molecule PD-1/PD-L1 inhibitor or a pharmaceutically acceptable salt thereof.

2. The pharmaceutical composition of claim 1, wherein the PD-L1 antibody is one or more selected from Atezolizumab, Durvalumab, Avelumab, Cemiplimab, KN035, CS1001, MBS2311, BGB-A333, KL-A167, SHR-1316 and STI-A1014.

3. The pharmaceutical composition of claim 2, wherein the PD-L1 antibody is Durvalumab.

4. The pharmaceutical composition of any one of claims 1-3, wherein the small-molecule PD-1/PD-L1 inhibitor is an aromatic vinyl or aromatic ethyl derivative, a pharmaceutically acceptable salt, a deuterated compound, a metabolite, a metabolic precursor or a prodrug thereof;
and/or, the small-molecule PD-1/PD-L1 inhibitor has a molecular weight of less than 1500 daltons;
and/or, the small-molecule PD-1/PD-L1 inhibitor has an IC₅₀ value of less than 100 nM in the PD-1/PD-L1 binding experiments;
and/or, the small-molecule PD-1/PD-L1 inhibitor binds to PD-L1.

5. The pharmaceutical composition of any one of claims 1-4, wherein the the small-molecule PD-1/PD-L1 inhibitor is an aromatic vinyl or aromatic ethyl derivative represented by general formula (I), a pharmaceutically acceptable salt, a deuterated compound, a metabolite, a metabolic precursor or a prodrug thereof: 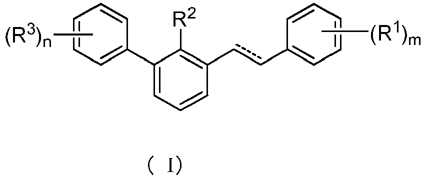 wherein, 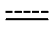 is a single bond or a double bond;
each of R¹ is identical or different, and is independently deuterium, halogen, substituted or unsubstituted hydroxyl, substituted or unsubstituted amino, substituted or unsubstituted alkyl, or substituted or unsubstituted alkoxy;
or two adjacent R¹ together with the two carbon atoms to which they are attached form a 5- to 7- membered carbocyclic ring or heterocarbocyclic ring; in the heterocarbocyclic ring, the heteroatom(s) is(are) oxygen and/or nitrogen, and the number of the heteroatom(s) is 1 to 4;
R² is substituted or unsubstituted alkyl or halogen;
each of R³ is identical or different, and is independently deuterium, halogen, substituted or unsubstituted alkylthio, substituted or unsubstituted hydroxyl, substituted or unsubstituted amino, substituted or unsubstituted alkyl, substituted or unsubstituted alkoxy, 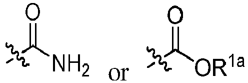 , wherein, R^{1a} is C₁-C₄ alkyl, or two adjacent R³ together with the two carbon atoms to which they are attached form a 5-to 7- membered carbocyclic ring or heterocarbocyclic ring; in the heterocarbocyclic ring, the heteroatom(s) is(are) oxygen and/or nitrogen, and the number of the heteroatom(s) is 1 to 4; when two R³ are adjacent, and the two adjacent R³ together with the two carbon atoms to which they are attached form a 5- to 7- membered carbocyclic ring or heterocarbocyclic ring, then the carbocyclic ring or heterocarbocyclic ring is further substituted by one or more than one C₁₋₄ alkyl;
the substituent(s) in the substituted alkyl in each of R¹, R² and each of R³, the substituent(s) in the substituted alkoxy in each of R¹ and each of R³, and the substituent(s) in the substituted alkylthio in each of R³ are one or more selected from halogen, C₁₋₄ alkyl, hydroxyl, 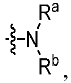 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 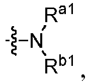 C₁-C₄ alkoxy, C₁-C₄ carboxyl, C₁-C₄ ester group and C₁-C₄ amide group; R^{a1} and R^{b1} are independently hydrogen or C₁-C₄ alkyl;
in each of R¹ or R³, the substituent(s) in the substituted hydroxyl or the substituted amino is(are) one or more selected from C₁₋₄ alkyl, C₁₋₄ alkoxy, C₁₋₄ carboxyl, C₁₋₄ ester group and C₁₋₄ amide group;
m is 1, 2, 3, 4 or 5;
n is 0, 1, 2, 3, 4 or 5; when 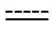 is a double bond and m is 2, then two R¹ are located on ortho and meta positions of the phenyl, and the two R¹ are identical or different;
when 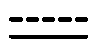 is a double bond and m is 3, then two of R¹ are adjacent, and the two adjacent R¹ together with the two carbon atoms to which they are attached form a 5-to 7- membered heterocarbocyclic ring;
or in the aromatic vinyl or aromatic ethyl derivative represented by general formula (I), 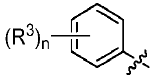 is replaced by a substituted or unsubstituted heteroaromatic ring, in the heteroaromatic ring, the heteroatom(s) is(are) selected from nitrogen, oxygen and sulfur, and the number of the heteroatom(s) is 1 to 4; the substituent(s) in the substituted heteroaromatic ring is(are) one or more selected from halogen, C₁₋₄ alkyl, hydroxyl, 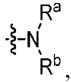 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 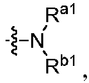 C₁-C₄ alkoxy, C₁-C₄ carboxyl, C₁-C₄ ester group and C₁-C₄ amide group; R^{a1} and R^{b1} are independently hydrogen or C₁-C₄ alkyl;
the aromatic vinyl or aromatic ethyl derivative represented by general formula (I) excludes the compound:

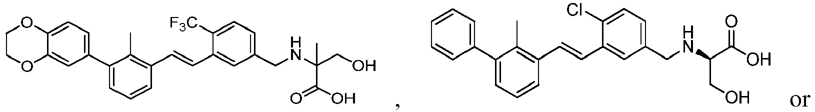

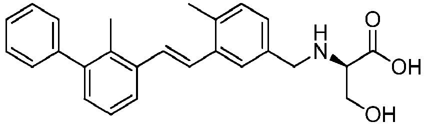

6. The pharmaceutical composition of claim 5, wherein,
when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the heteroaromatic ring are halogen, then the halogen is F, Cl, Br or I;
and/or, when the substituent(s) in the substituted alkyl in each of R¹, R² and each of R³, the substituent(s) in the substituted alkoxy, the substituted hydroxyl or the substituted amino in each of R¹ and each of R³, the substituent(s) in the substituted alkylthio in each of R³, the substituent(s) in the substituted heteroaromatic ring, and the substituent(s) in the 5- to 7- membered heterocarbocyclic ring are C₁-C₄ alkyl, then the C₁-C₄ alkyl is methyl, ethyl, n-propyl, isopropyl, n-butyl, isobutyl or *tert-butyl;*
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 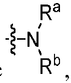 and R^{a} or R^{b} is halogen, then the halogen is F, Cl, Br or I;
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 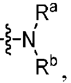 and R^{a} or R^{b} is C₁-C₄ alkyl, then the C₁-C₄ alkyl is methyl, ethyl, n-propyl, isopropyl, n-butyl, isobutyl or *tert-butyl;*
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 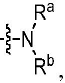 R^{a} or R^{b} is 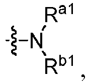 and R^{a1} or R^{b1} is C₁-C₄ alkyl, then the C₁-C₄ alkyl is methyl, ethyl, n-propyl, isopropyl, n-butyl, isobutyl or *tert-butyl;*
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 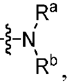 R^{a} or R^{b}is C₁-C₄ alkoxy, then the C₁₋₄ alkoxy is methoxy, ethoxy, n-propoxy, isopropoxy, n-butoxy, isobutoxy or *tert*-butoxy;
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 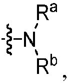 R^{a} or R^{b} is C₁-C₄ carboxyl, then the C₁₋₄ carboxyl is 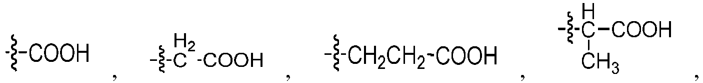 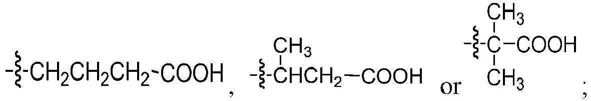
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 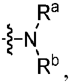 and R^{a} or R^{b} is C₁-C₄ ester group, then the C₁-C₄ ester group is 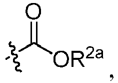 and R^{2a} is methyl, ethyl, *n*-propyl, isopropyl, n-butyl, isobutyl or *tert-butyl;*
and/or, when the substituent(s) in the substituted alkyl in each of R¹, R² and each of R³, the substituent(s) in the substituted alkoxy in each of R¹ and each of R³, the substituent(s) in the substituted alkylthio in each of R³, and the substituent(s) in the substituted heteroaromatic ring are 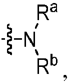 and R^{a} or R^{b} is C₁-C₄ amide group, then the C₁-C₄ amide group is 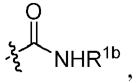 and R^{1b} is hydrogen, methyl, ethyl, n-propyl, isopropyl, n-butyl, isobutyl or *tert-butyl;*
and/or, when the substituent(s) in the substituted alkyl in each of R¹, R² and each of R³, the substituent(s) in the substituted alkoxy, the substituted hydroxyl or the substituted amino in each of R¹ and each of R³, the substituent(s) in the substituted alkylthio in each of R³, and the substituent(s) in the substituted heteroaromatic ring are C₁-C₄ alkoxy, then the C₁₋₄ alkoxy is methoxy, ethoxy, n-propoxy, isopropoxy, n-butoxy, isobutoxy or *tert*-butoxy;
and/or, when the substituent(s) in the substituted alkyl in each of R¹, R² and each of R³, the substituent(s) in the substituted alkoxy, the substituted hydroxyl or the substituted amino in each of R¹ and each of R³, the substituent(s) in the substituted alkylthio in each of R³, and the substituent(s) in the substituted heteroaromatic ring are C₁-C₄ carboxyl, then the C₁₋₄ carboxyl is 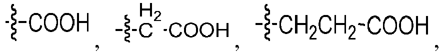 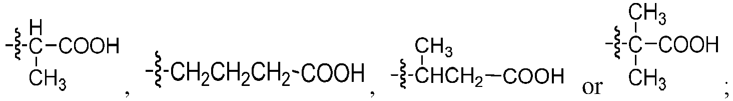
and/or, when the substituent(s) in the substituted alkyl in each of R¹, R² and each of R³, the substituent(s) in the substituted alkoxy, the substituted hydroxyl or the substituted amino in each of R¹ and each of R³, the substituent(s) in the substituted alkylthio in each of R³, and the substituent(s) in the substituted heteroaromatic ring are C₁-C₄ ester group, then the C₁-C₄ ester group is 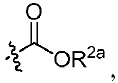 and R^{2a} is methyl, ethyl, n-propyl, isopropyl, n-butyl, isobutyl or *tert-*butyl*;*
and/or, when the substituent(s) in the substituted alkyl in each of R¹, R² and each of R³, the substituent(s) in the substituted alkoxy, the substituted hydroxyl or the substituted amino in each of R¹ and each of R³, the substituent(s) in the substituted alkylthio in each of R³, and the substituent(s) in the substituted heteroaromatic ring are C₁-C₄ amide group, then the C₁-C₄ amide group is 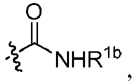 and R^{1b} is hydrogen, methyl, ethyl, n-propyl, isopropyl, n-butyl, isobutyl or *tert*-butyl.

7. The pharmaceutical composition of claim 5, wherein,
in each of R¹, R² and each of R³, the halogen is F, Cl, Br or I;
and/or, in each of R¹, R² and each of R³, the substituted or unsubstituted alkyl is substituted or unsubstituted C₁-C₄ alkyl; the substituted or unsubstituted C₁-C₄ alkyl is preferably substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, or, substituted or unsubstituted *tert-*butyl*;*
and/or, in each of R¹ and each of R³, the substituted or unsubstituted alkoxy is substituted or unsubstituted C₁-C₄ alkoxy; the substituted or unsubstituted C₁-C₄ alkoxy is preferably substituted or unsubstituted methoxy, substituted or unsubstituted ethoxy, substituted or unsubstituted n-propoxy, substituted or unsubstituted isopropoxy, substituted or unsubstituted *n*-butoxy, substituted or unsubstituted isobutoxy, or, substituted or unsubstituted *tert*-butoxy;
and/or, in each of R³, the substituted or unsubstituted alkylthio is -S-R^{s}, wherein, R^{s} is substituted or unsubstituted C₁-C₄ alkyl; the substituted or unsubstituted C₁-C₄ alkyl is preferably substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, or, substituted or unsubstituted *tert*-butyl;
and/or, in each of R³, in 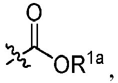 R^{1a} is methyl, ethyl, *n*-propyl, isopropyl, n-butyl, isobutyl or *tert*-butyl;
and/or, when two adjacent R³ together with the two carbon atoms to which they are attached form a 5- to 7- membered heterocarbocyclic ring, then the 5- to 7-membered heterocarbocyclic ring is azetidine ring, piperazine ring, piperidine ring, pyrrole ring, morpholine ring, thiomorpholine ring, 1,4-dioxane, pyran ring, dihydroimidazole ring, dihydroisoxazole ring, dihydroisothiazole ring, dihydrooxadiazole ring, dihydrooxazole ring, dihydropyrazine ring, dihydropyrazole ring, dihydropyridine ring, dihydropyrimidine ring, dihydropyrrole ring, dihydroquinoline ring, dihydrotetrazole ring, dihydrothiadiazole ring, dihydrothiazole ring, dihydrotriazole ring, dihydroazetidine ring, imidazole ring, pyrazole ring, pyrrole ring, furan ring, thiophene ring, isothiazole ring, oxazole ring, oxadiazole ring, isoxazole ring, pyrazine ring, pyridazine ring, pyridine ring, pyrimidine ring, tetrazole ring, thiadiazole ring, thiazole ring, thiophene ring or triazole ring;
and/or, when 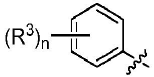 is replaced by a substituted or unsubstituted heteroaromatic ring, then the heteroaromatic ring is C₁-C₁₀ heteroaromatic ring, the heteroatom(s) in the heteroaromatic ring is(are) preferably selected from nitrogen and oxygen, and the number of the heteroatom(s) is 1 to 3; the heteroaromatic ring is more preferably acridine ring, carbazole ring, cinnoline ring, carboline ring, quinoxaline ring, imidazole ring, pyrazole ring, pyrrole ring, indole ring, indoline ring, benzotriazole ring, benzimidazole ring, furan ring, thiophen ring, isothiazole ring, benzothiophene ring, dihydrobenzothiophene ring, benzofuran ring, isobenzofuran ring, benzoxazole ring, benzofuraxan ring, benzopyrazole ring, quinoline ring, isoindoline ring, isoquinoline ring, oxazole ring, oxadiazole ring, isoxazole ring, indole ring, pyrazine ring, pyridopyridine ring, tetrazolopyridine ring, imidazopyridine ring, imidazopyrazine ring, pyridazine ring, pyridine ring, naphthopyrimidine ring, pyrimidine ring, tetrazole ring, thiadiazole ring, thiazole ring, thiophene ring, triazole ring, quinazoline ring, tetrahydroquinoline ring, dihydrobenzimidazole ring, dihydrobenzofuran ring, dihydrobenzoxazole ring or dihydroquinoline ring.

8. The pharmaceutical composition of any one of claims 5-7, wherein,
each of R¹ is independently halogen, or, substituted or unsubstituted alkyl; or two adjacent R¹ together with the two carbon atoms to which they are attached form a 5-to 7- membered heterocarbocyclic ring; or each of R¹ is independently halogen, substituted or unsubstituted alkyl, or substituted or unsubstituted alkoxy;
and/or, R² is alkyl or halogen;
and/or, each of R³ is independently deuterium, halogen, alkylthio or alkoxy; or two adjacent R³ together with the two carbon atoms to which they are attached form a 5- to 7- membered heterocarbocyclic ring; when two R³ are adjacent, and the two adjacent R³ together with the two carbon atoms to which they are attached form the 5-to 7- membered carbocyclic ring or heterocarbocyclic ring, then the carbocyclic ring or heterocarbocyclic ring can be further substituted by one or more than one C₁-C₄ alkyl;
and/or, n is 0, 1, 2, 3, 4 or 5;
and/or, m is 2 or 3; preferably, m is 2;
and/or, 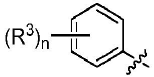 can be replaced by a substituted or unsubstituted heteroaromatic ring.

9. The pharmaceutical composition of claim 8, wherein,
when R¹ is substituted alkyl, the substituent(s) in the substituted alkyl is(are) one or more selected from halogen and 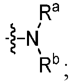 R^{a} and R^{b} are independently hydrogen, or, substituted or unsubstituted alkyl; in R^{a} or R^{b}, the substituent(s) in the substituted alkyl is(are) one or more selected from hydroxyl and C₁-C₄ carboxyl;
and/or, in R¹, the substituent(s) in the substituted alkyl, the substituent(s) in the substituted alkoxy, the substituent(s) in the substituted hydroxyl or the substituent(s) in the substituted amino are substituted by one or more than one halogen, preferably substituted by one or more than one F;
and/or, when n is 1, then R³ is halogen, alkylthio or alkoxy; and R³ is located on ortho, meta or para position of the phenyl;
and/or, when n is 2, then two R³ are located on ortho and meta positions of the phenyl, and the two R³ are identical or different; or two R³ are adjacent, and the two adjacent R³ together with the two carbon atoms to which they are attached form a 5-to 7- membered heterocarbocyclic ring; when two R³ are adjacent, and the two adjacent R³ together with the two carbon atoms to which they are attached form a 5-to 7- membered carbocyclic ring or heterocarbocyclic ring, then the carbocyclic ring or heterocarbocyclic ring can be further substituted by one or more than one C₁-C₄ alkyl; the 5- to 7- membered heterocarbocyclic ring is preferably 2,3-dihydro-1,4-dioxane, oxazole ring, isoxazole ring or pyrazole ring;
and/or, when n is 3, then two of R³ are adjacent, and the two adjacent R³ together with the two carbon atoms to which they are attached form a 5- to 7- membered heterocarbocyclic ring; when two R³ are adjacent, and the two adjacent R³ together with the two carbon atoms to which they are attached form a 5- to 7- membered carbocyclic ring or heterocarbocyclic ring, then the carbocyclic ring or heterocarbocyclic ring can be further substituted by one or more than one C₁-C₄ alkyl; the 5- to 7- membered heterocarbocyclic ring is preferably 2,3-dihydro-1,4-dioxane, oxazole ring, isoxazole ring or pyrazole ring;
and/or, when n is 4 or 5, R³ is deuterium;
and/or, when m is 2, then two R¹ are located on ortho and meta positions of the phenyl, respectively, and the two R¹ are identical or different; R¹ located on ortho position of the phenyl is alkyl or alkyl substituted by halogen; R¹ located on meta position of the phenyl is alkyl substituted by 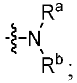
and/or, when m is 2, then two R¹ are located on ortho and meta positions of the phenyl, respectively, and the two R¹ are identical or different; R¹ located on ortho position of the phenyl is F, substituted or unsubstituted alkyl, or substituted or unsubstituted alkoxy;
and/or, when m is 3, then two of R¹ are adjacent, and the two adjacent R¹ together with the two carbon atoms to which they are attached form a 5- to 7- membered heterocarbocyclic ring, and the third R¹ is alkyl substituted by 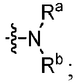
and/or, when 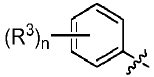 is replaced by a substituted or unsubstituted heteroaromatic ring, then the heteroatom(s) in the heteroaromatic ring is(are) selected from nitrogen and oxygen, and the number of the heteroatom(s) is 1 to 3; further preferably, when the heteroaromatic ring is a monocyclic ring, then the heteroatom(s) is(are) nitrogen, and the number of the heteroatom(s) is 1 or 2; when the heteroaromatic ring is a dicyclic heteroaromatic ring and the heteroatom(s) is(are) nitrogen, then the number of the heteroatom(s) is 3; when the heteroaromatic ring is a dicyclic heteroaromatic ring, the heteroatom(s) is(are) selected from nitrogen and oxygen, and the number of the heteroatoms is 2, then the heteroatoms are not adjacent.

10. The pharmaceutical composition of claim 9, wherein,
R¹ located on ortho position of the phenyl is C₁₋₄ alkyl or C₁₋₄ alkyl substituted by one or more than one halogen, such as trifluoromethyl;
and/or, the alkyl substituted by 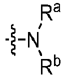 is C₁-C₄ alkyl substituted by 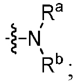 the C₁-C₄ alkyl substituted by 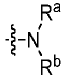 is preferably 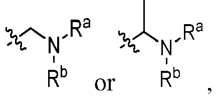 wherein, one of R^{a} and R^{b} is H, and the other is alkyl substituted by hydroxyl and/or carboxyl; the C₁-C4 alkyl substituted by 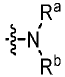 is more preferably 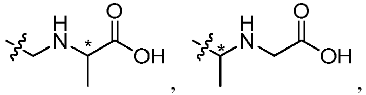 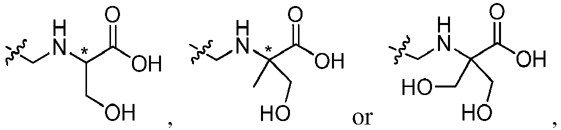 wherein, the carbon labelled by ^{∗} is an S-configuration chiral carbon, an *R*-configuration chiral carbon or an achiral carbon; 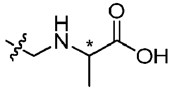 is preferably 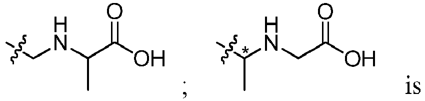 preferably 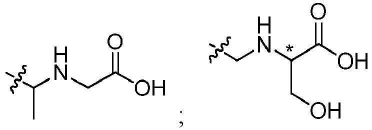 is preferably 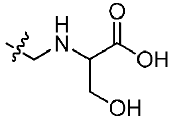 or 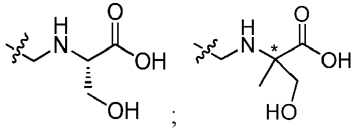 is preferably 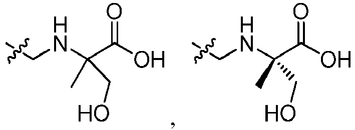 or

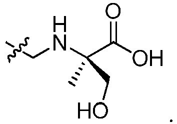

11. The pharmaceutical composition of any one of claims 5-10, wherein,
each of R¹ is independently H, or, substituted or unsubstituted alkyl; or two adjacent R¹ together with the two carbon atoms to which they are attached form a 5-to 7- membered heterocarbocyclic ring; the substituent(s) in the substituted alkyl is(are) one or more selected from halogen or 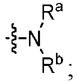 R^{a} and R^{b} are independently hydrogen, or, substituted or unsubstituted alkyl; in R^{a} or R^{b}, the substituent(s) in the substituted alkyl is(are) one or more selected from hydroxyl or C₁-C₄ carboxyl;
R² is alkyl or halogen;
each of R³ is independently H, halogen, alkylthio or alkoxy; or two adjacent R³ together with the two carbon atoms to which they are attached form a 5- to 7-membered heterocarbocyclic ring;
n is 0, 1, 2, 3, 4 or 5; when n is 1, then R³ is halogen, alkylthio or alkoxy; and R³ is located on ortho, meta or para position of the phenyl; when n is 2, then two R³are located on ortho and meta positions of the phenyl, wherein, the two R³ are identical or different; or two R³ are adjacent, and the two adjacent R³ together with the two carbon atoms to which they are attached form a 5- to 7- membered heterocarbocyclic ring; when n is 3, then one of R³ is halogen, the other two R³ are adjacent, and the two adjacent R³ together with the two carbon atoms to which they are attached form a 5-to 7- membered heterocarbocyclic ring; when two R³ are adjacent, and the two adjacent R³ together with the two carbon atoms to which they are attached form a 5-to 7- membered carbocyclic ring or heterocarbocyclic ring, then the carbocyclic ring or heterocarbocyclic ring is further substituted by one or more than one C₁-C₄ alkyl; when n is 4 or 5, R³ is deuterium;
m is 2 or 3; when m is 2, then two R¹ are located on ortho and meta position of the phenyl, respectively, R¹ located on ortho position of the phenyl is alkyl or alkyl substituted by halogen; R¹ located on meta position of the phenyl is alkyl substituted by 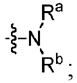 when m is 3, then two of R³ are adjacent, and the two adjacent R³ together with the two carbon atoms to which they are attached form a 5- to 7- membered heterocarbocyclic ring, and the third R³ is alkyl substituted by 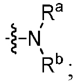
and 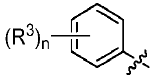 can be replaced by a substituted or unsubstituted heteroaromatic ring, in the heteroaromatic ring, the heteroatom(s) is(are) selected from nitrogen and oxygen, and the number of the heteroatom(s) is 1 to 3; further preferably, when the heteroaromatic ring is a monocyclic ring, then the heteroatom(s) is(are) nitrogen, and the number of the heteroatom(s) is 1 or 2; when the heteroaromatic ring is a dicyclic heteroaromatic ring and the heteroatom(s) is(are) nitrogen, then the number of the heteroatom(s) is 3; when the heteroaromatic ring is a dicyclic heteroaromatic ring, the heteroatom(s) is(are) selected from nitrogen and oxygen, and the number of the heteroatoms is 2, then the heteroatoms are not adjacent.

12. The pharmaceutical composition of any one of claims 5-11, wherein, 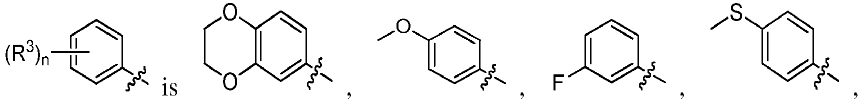 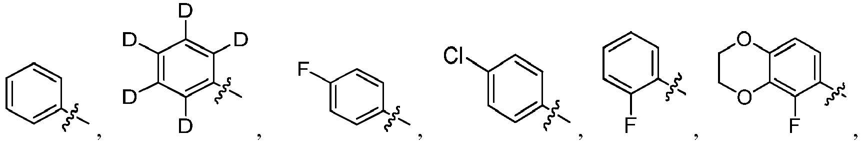 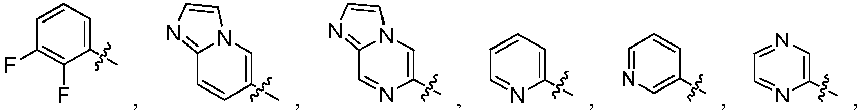 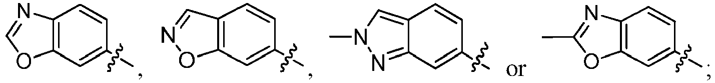 and/or,

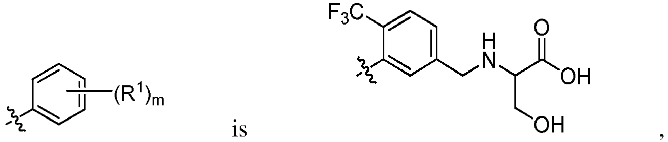

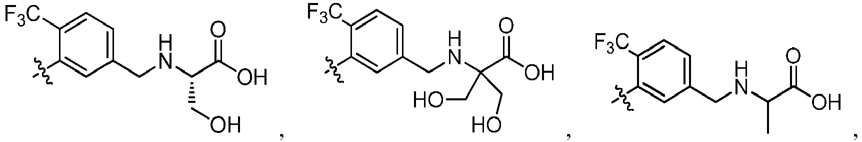

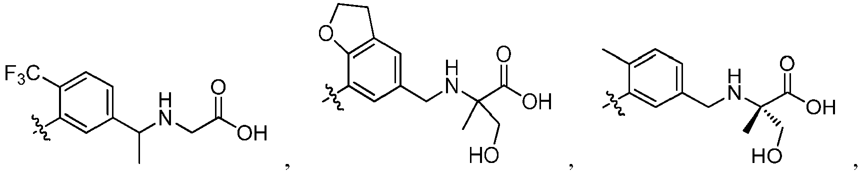

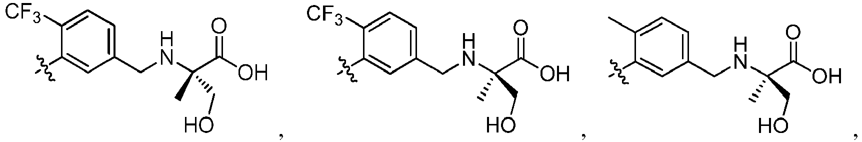

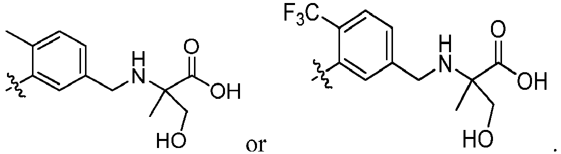

13. The pharmaceutical composition of claim 5, wherein,
each of R¹ is independently halogen, substituted or unsubstituted alkyl, or substituted or unsubstituted alkoxy;
R² is substituted or unsubstituted alkyl or halogen;
each of R³ is independently deuterium, halogen, substituted or unsubstituted alkylthio, substituted or unsubstituted hydroxyl, substituted or unsubstituted amino, substituted or unsubstituted alkyl, substituted or unsubstituted alkoxy, 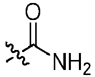 or 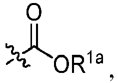 wherein, R^{1a} is C₁-C₄ alkyl;
in each of R¹, the substituent(s) in the substituted alkyl, the substituent(s) in the substituted alkoxy, the substituent(s) in the substituted hydroxyl or the substituent(s) in the substituted amino are substituted by one or more than one halogen, preferably substituted by one or more than one F;
the substituent(s) in the substituted alkyl in each of R² and R³, the substituent(s) in the substituted alkoxy and the substituent(s) in the substituted alkythio in each of R³ are one or more selected from halogen, C₁₋₄ alkyl, hydroxyl, 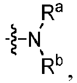 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 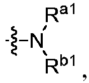 C₁-C₄ alkoxy, C₁-C₄ carboxyl, C₁-C₄ ester group and C₁-C₄ amide group; R^{a1} and R^{b1} are independently hydrogen or C₁-C₄ alkyl;
in each of R³, the substituent(s) in the substituted hydroxyl or the substituted amino is(are) one or more selected from C₁₋₄ alkyl, C₁₋₄ alkoxy, C₁₋₄ carboxyl, C₁₋₄ ester group and C₁₋₄ amide group;
m is 2 or 3;
n is 0, 1, 2, 3, 4 or 5;
when m is 2, then two R¹ are located on ortho and meta positions of the phenyl, and the two R¹ are identical or different; the R¹ located on ortho positions of the phenyl is F, substituted or unsubstituted alkyl, or, substituted or unsubstituted alkoxy;
when 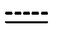 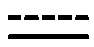 is a double bond and m is 3, then two of R¹ are adjacent, and the two adjacent R¹ together with the two carbon atoms to which they are attached form a 5-to 7- membered heterocarbocyclic ring;
or in the aromatic vinyl or aromatic ethyl derivative represented by general formula (I), 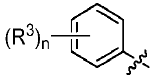 is replaced by a substituted or unsubstituted heteroaromatic ring, in the heteroaromatic ring, the heteroatom(s) is(are) selected from nitrogen, oxygen and sulfur, and the number of the heteroatom(s) is 1 to 4; the substituent(s) in the substituted heteroaromatic ring is(are) one or more selected from halogen, C₁₋₄ alkyl, hydroxyl, 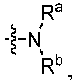 C₁₋₄ alkoxy, C₁₋₄ carboxyl, C₁₋₄ ester group and C₁₋₄ amide group; when there are more substituents than one, then the substituents are identical or different; R^{a} and R^{b} are independently hydrogen, or, substituted or unsubstituted alkyl; in R^{a} or R^{b}, the substituent(s) in the substituted alkyl is(are) one or more selected from halogen, C₁-C₄ alkyl, hydroxyl, 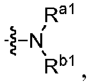 C₁-C₄ alkoxy, C₁-C₄ carboxyl, C₁-C₄ ester group and C₁-C₄ amide group; R^{a1} and R^{b1} are independently hydrogen or C₁-C₄ alkyl.

14. The pharmaceutical composition of any one of claims 5-13, wherein, the aromatic vinyl or aromatic ethyl derivative represented by general formula (I) is:

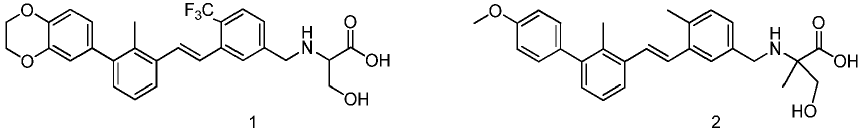

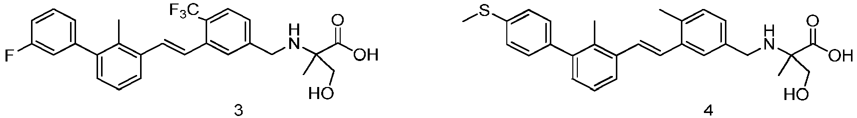

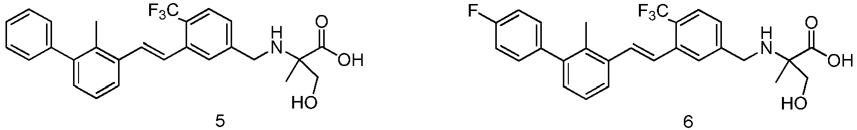

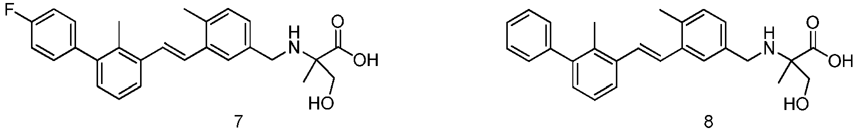

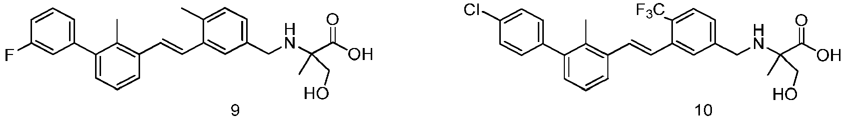

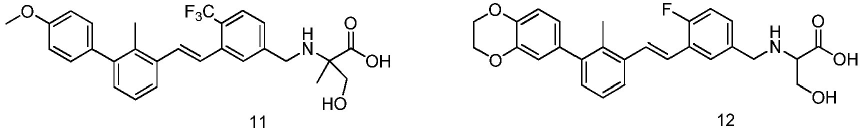

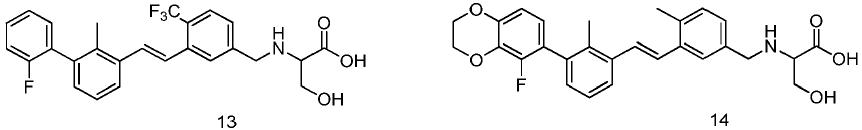

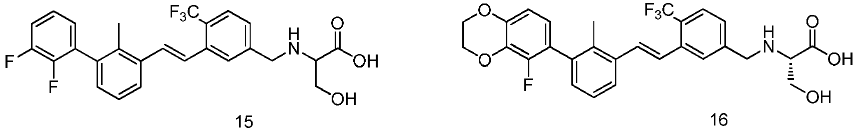

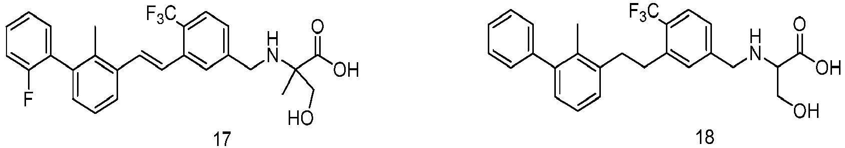

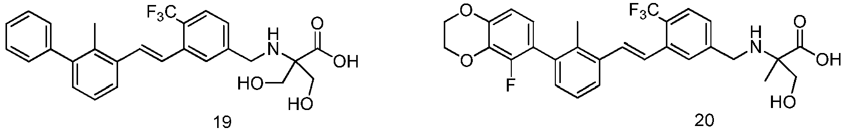

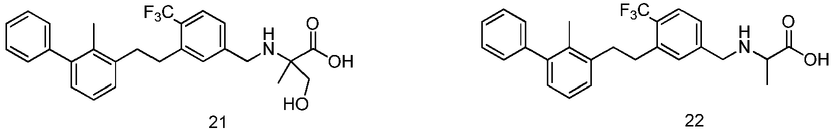

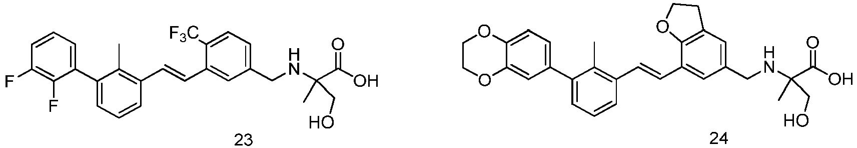

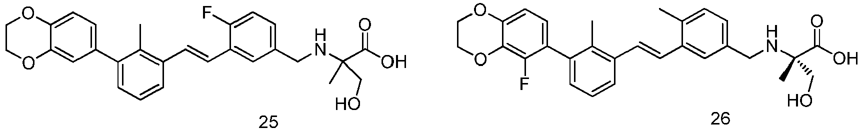

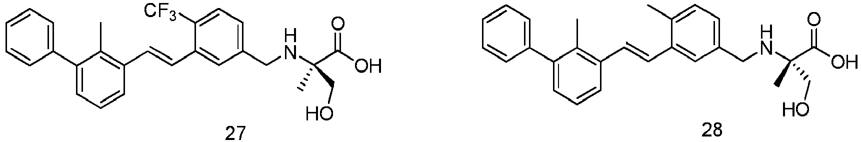

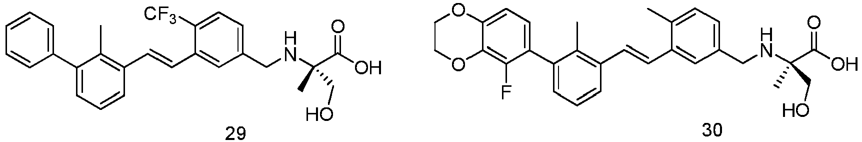

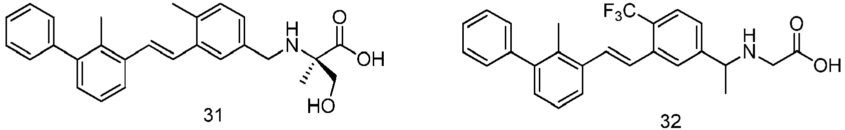

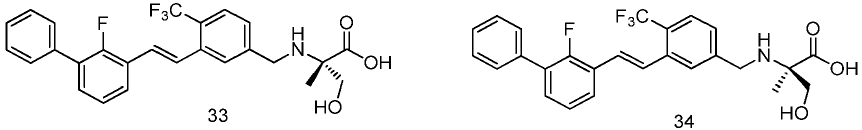

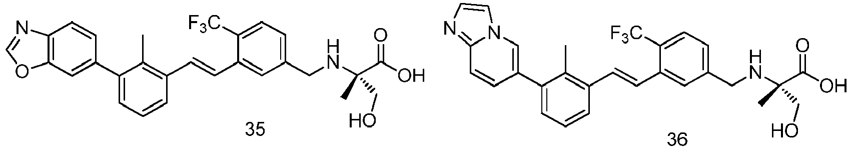

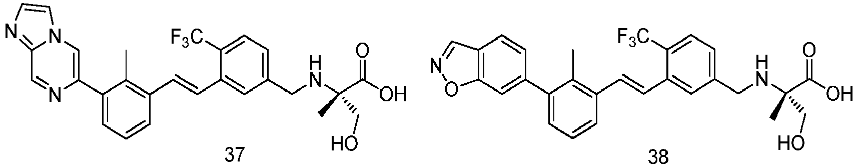

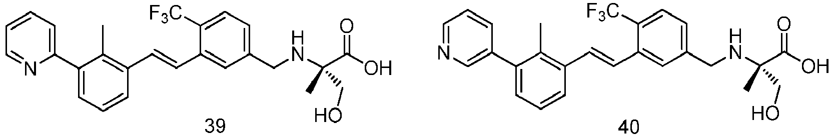

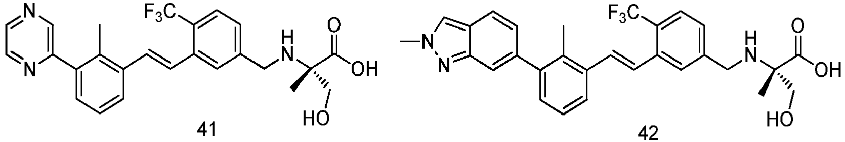

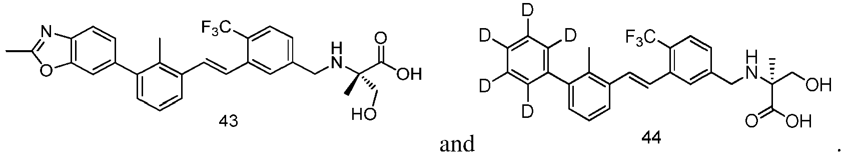

15. The pharmaceutical composition of any one of claims 5-14, wherein, the aromatic vinyl or aromatic ethyl derivative represented by general formula (I) is an aromatic vinyl or aromatic ethyl derivative represented by general formula (II): 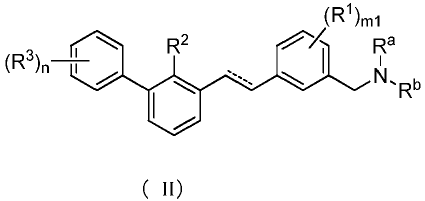 wherein, the definitions of R¹, R², R³, n, R^{a} and R^{b} are as defined in claim 5, and m1 is 0, 1 or 2.

16. The pharmaceutical composition of any one of claims 5-15, wherein, the aromatic vinyl or aromatic ethyl derivative represented by general formula (I) is Compound 29

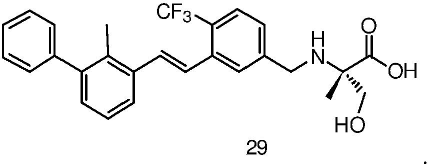

17. The pharmaceutical composition of claim 16, wherein, Compound 29 is in oral dosage form;
and/or, PD-L1 is Durvalumab, which is in injection dosage form.

18. The pharmaceutical composition of any one of claims 1-4, wherein, the small-molecule PD-1/PD-L1 inhibitor is any one of the following compounds or a pharmaceutically acceptable salt thereof:

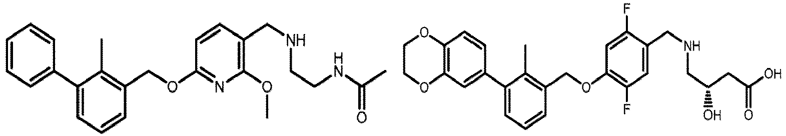

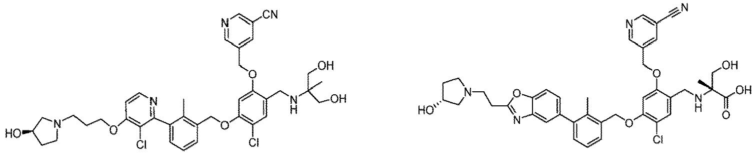

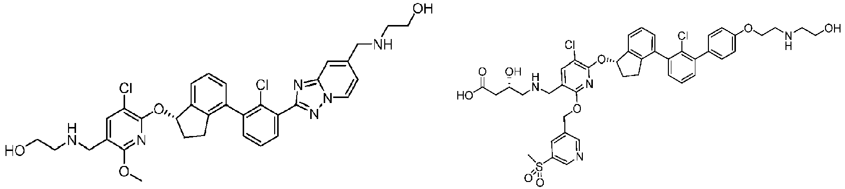

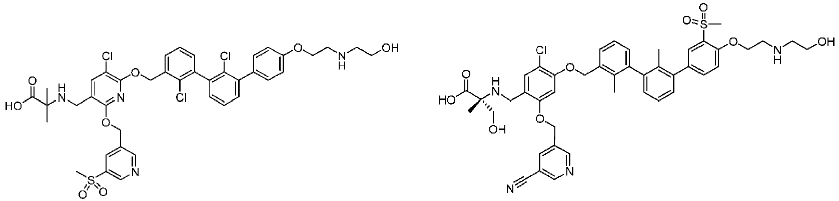

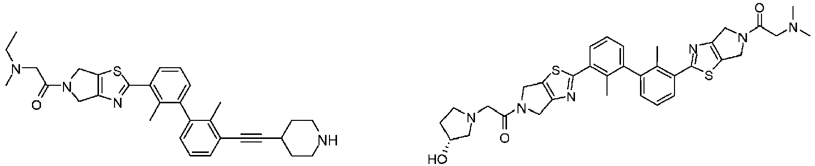

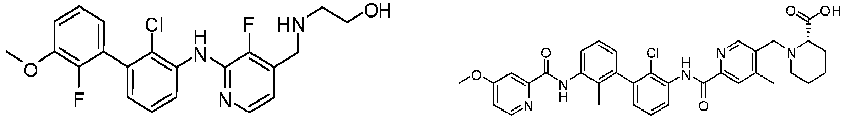

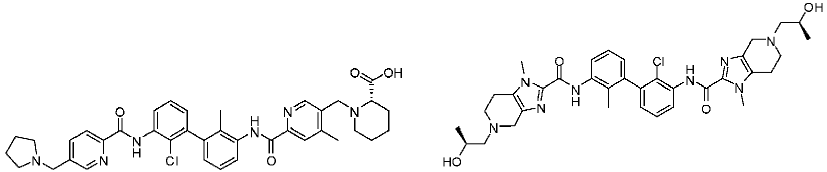

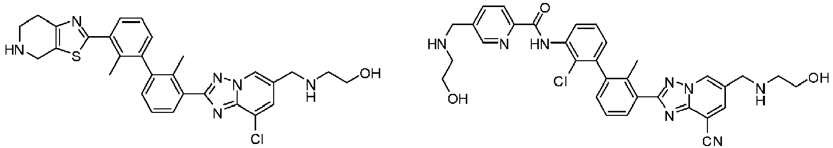

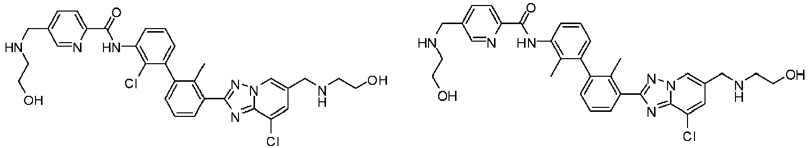

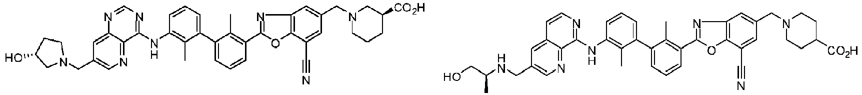

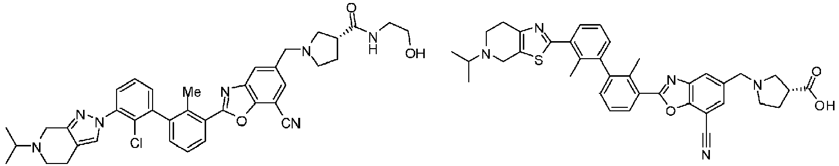

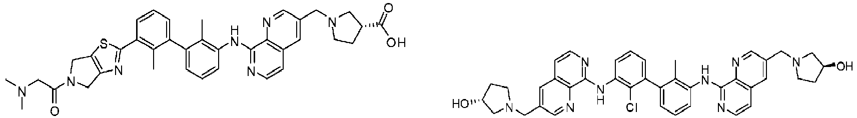

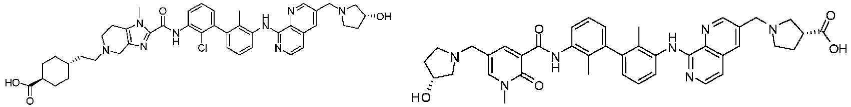

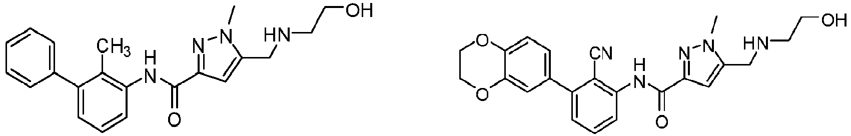

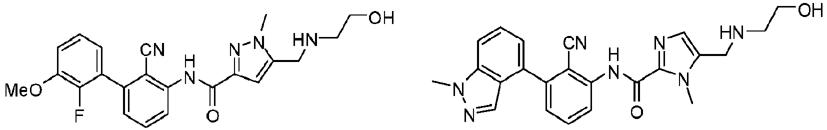

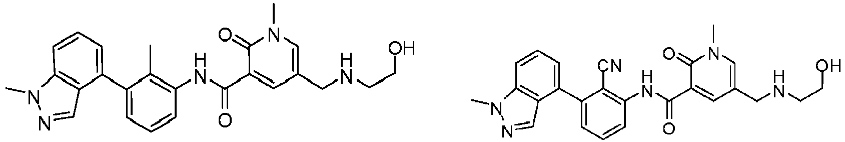

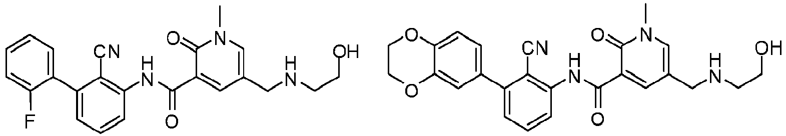

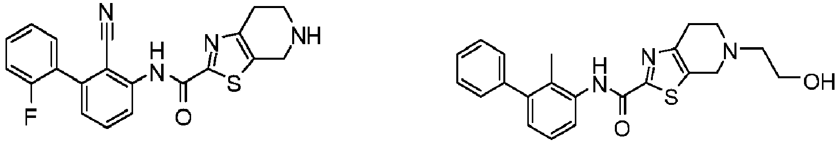

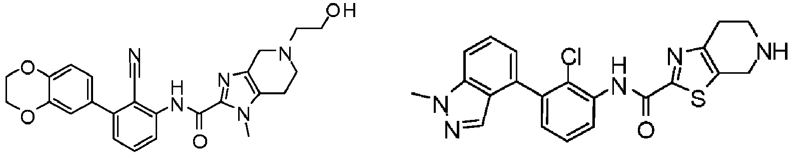

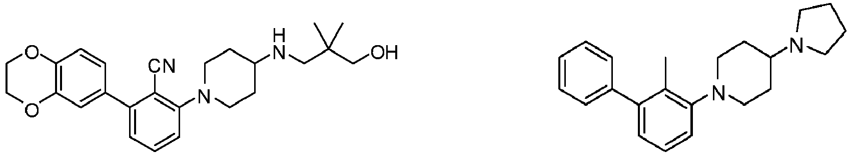

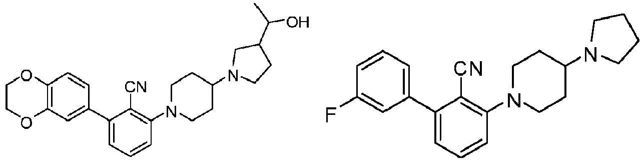

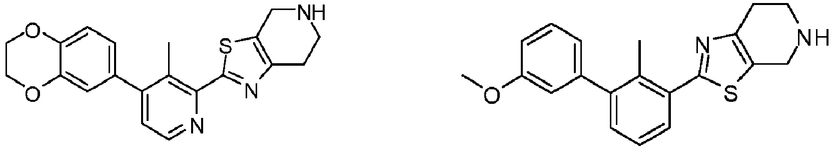

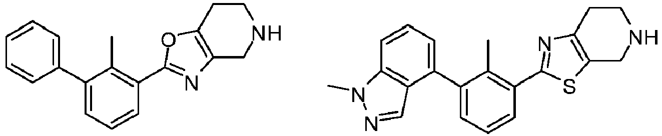

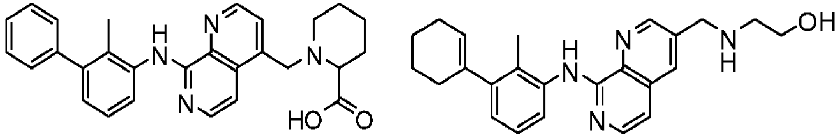

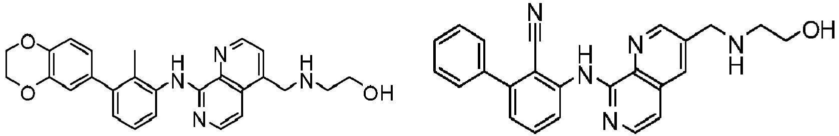

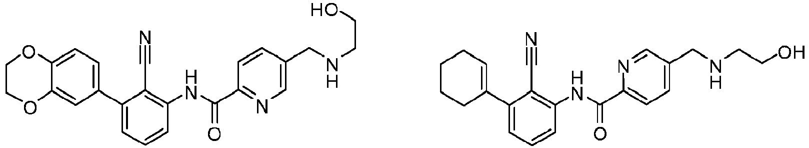

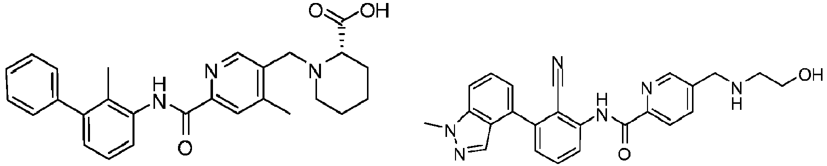

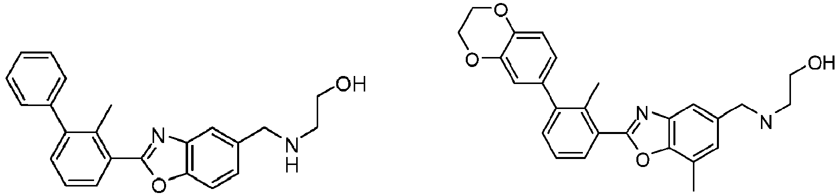

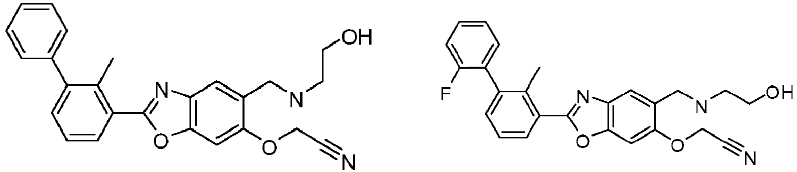

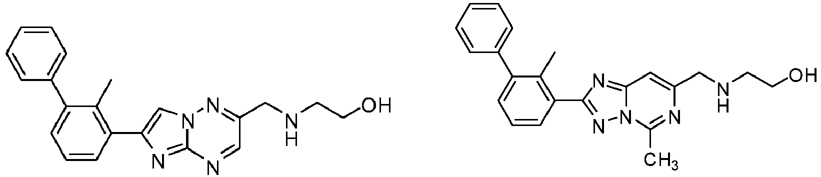

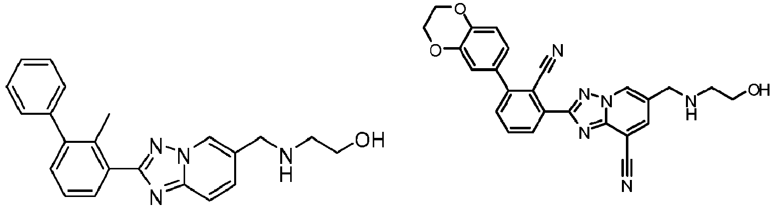

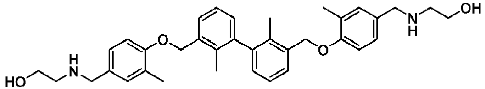

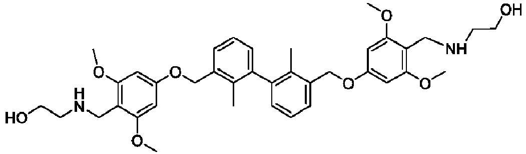

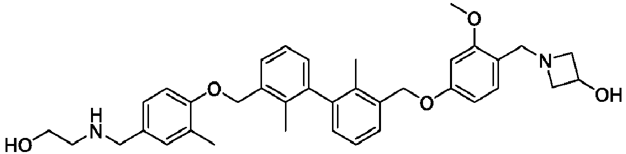

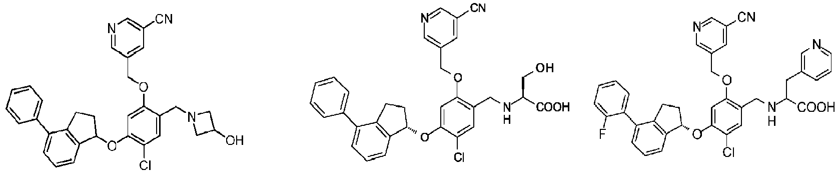

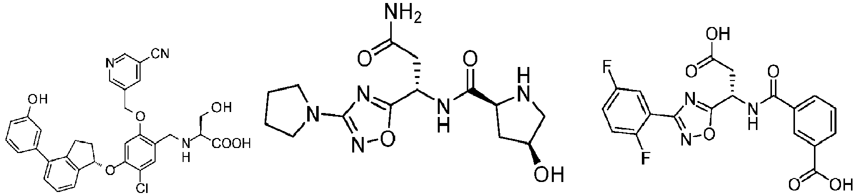

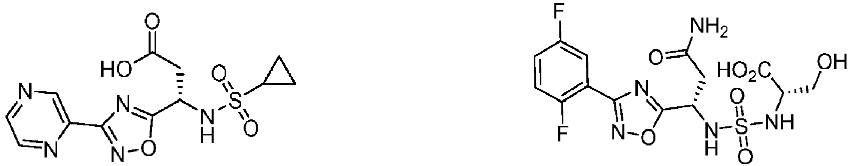

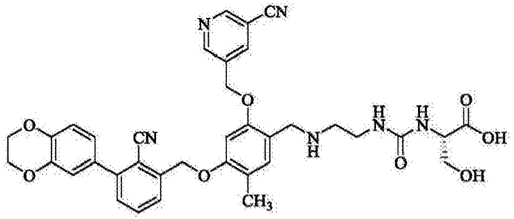

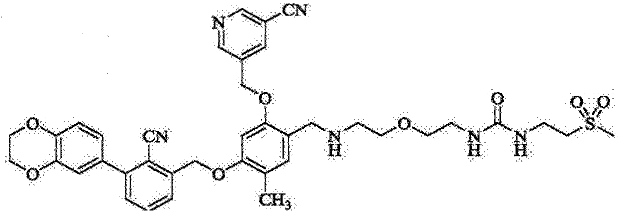

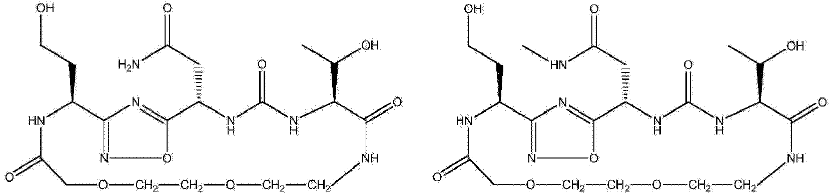

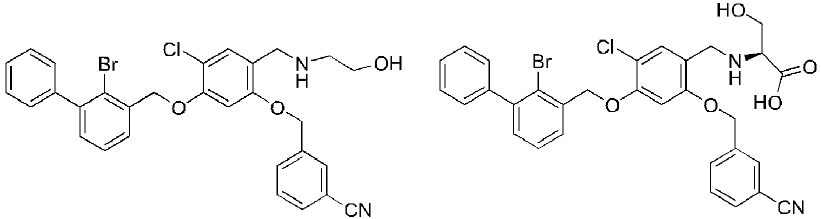

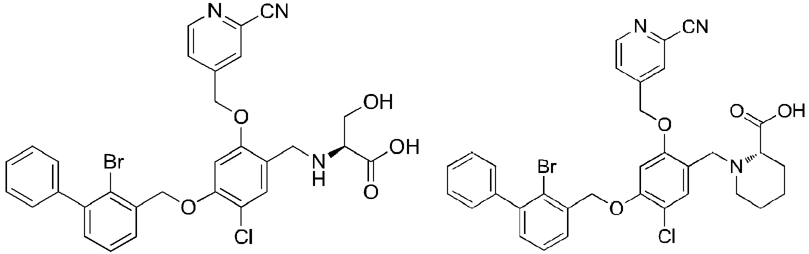

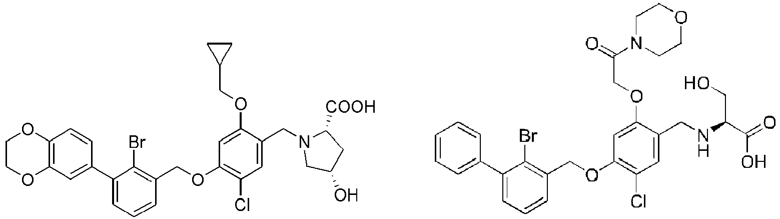

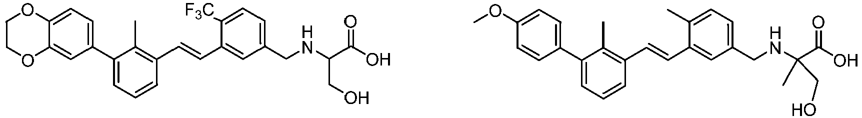

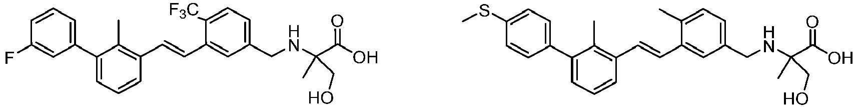

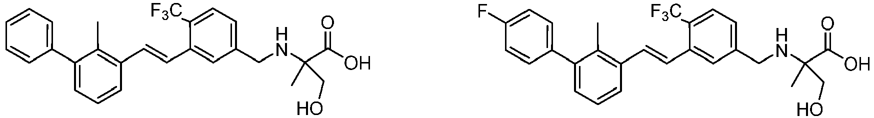

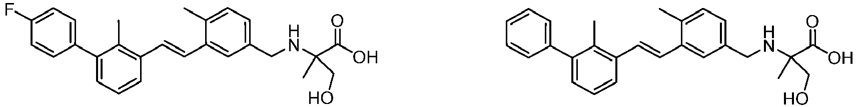

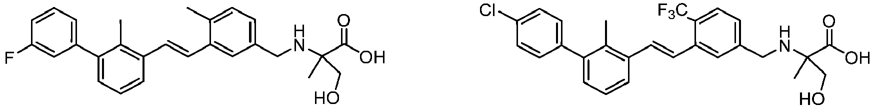

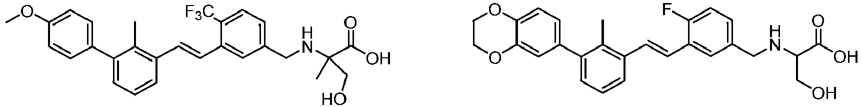

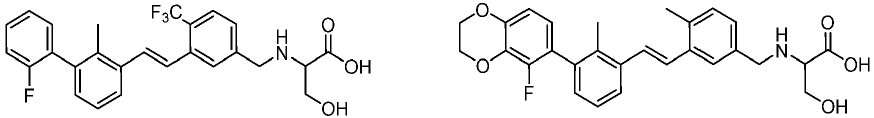

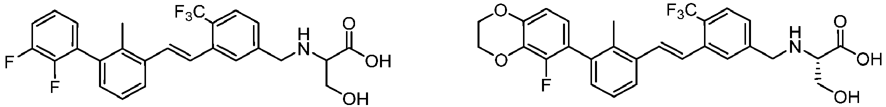

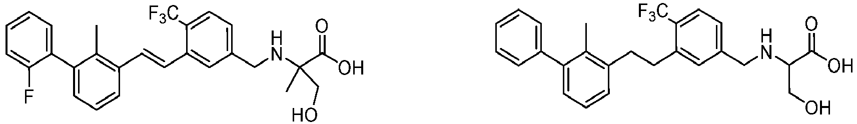

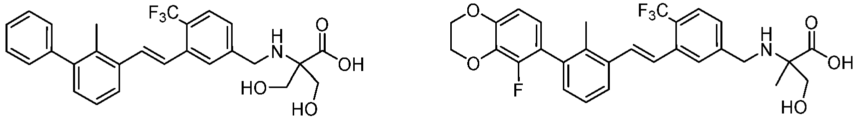

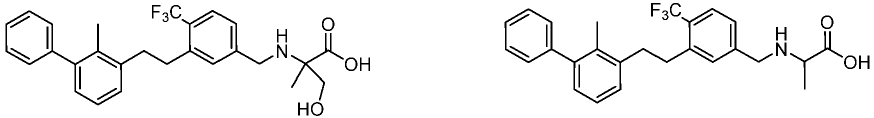

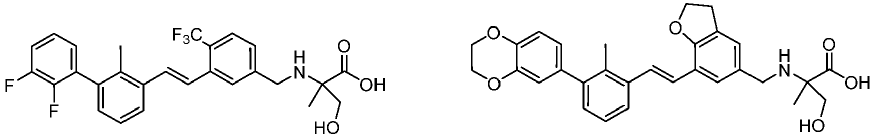

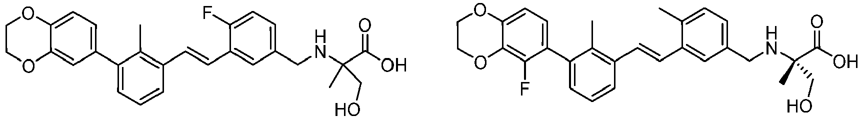

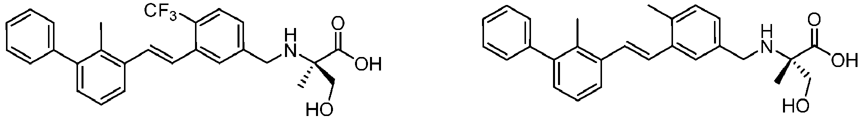

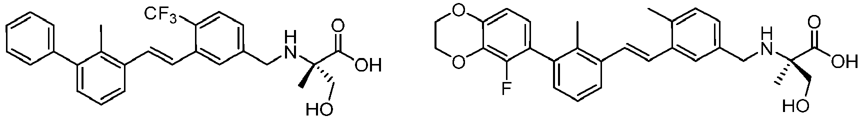

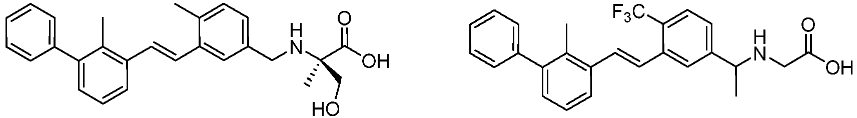

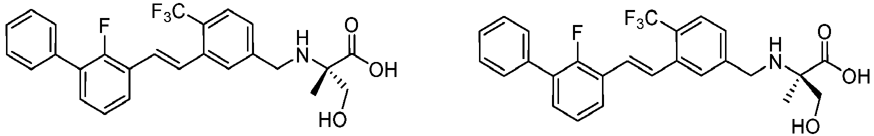

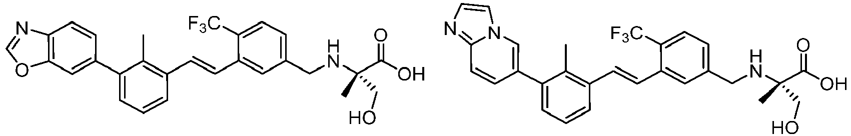

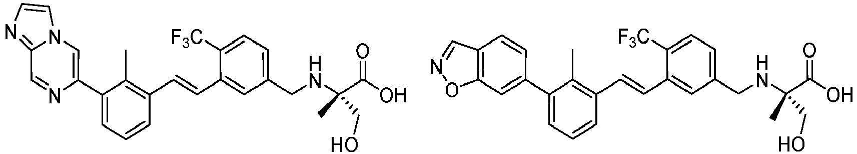

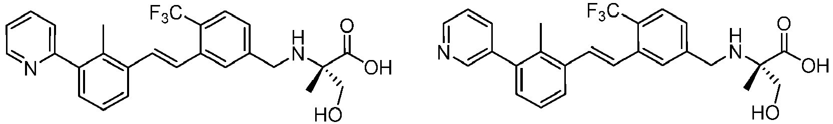

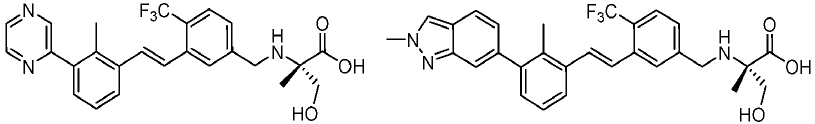

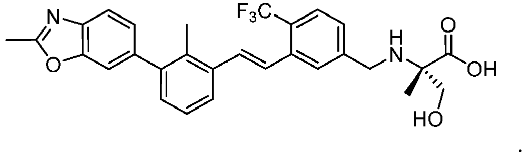

19. The pharmaceutical composition of any one of claims 1-18, wherein, the pharmaceutical composition comprises a pharmaceutically acceptable carrier;
and/or, the PD-L1 antibody and small-molecule PD-1/PD-L1 inhibitor exist in a single pharmaceutical composition or in separate pharmaceutical compositions.

20. A use of the pharmaceutical composition of any one of claims 1-19 in manufacturing a medicament for treating cancer.

21. The use of claim 20, wherein, the cancer is lung cancer, stomach cancer, colorectal cancer, cervical cancer, ovarian cancer, prostate cancer, breast cancer, pancreatic cancer, liver cancer, bladder cancer, kidney cancer, bone cancer, skin cancer, melanoma, glioma, neuroblastoma, leukemia or lymphoma, such as colorectal cancer, such as colon cancer;
and/or, the PD-L1 antibody and small-molecule PD-1/PD-L1 inhibitor are administered simultaneously or administered separately;
and/or, the PD-L1 antibody is administered by injection;
and/or, the small-molecule PD-1/PD-L1 inhibitor is administered by oral administration.
